# EUROPEAN PATENT APPLICATION

(11) **EP 4 768 078 A2**
(43) Date of publication of application: **01.07.2026**
(21) Application number: 26163118.8
(22) Date of filing: 18.03.2021
(51) Int. Cl.: A61P 1/04

(54) **IMPLANTABLE CELL CHAMBER DEVICE AND USES THEREOF**

(30) Priority: 18.03.2020 US 202062991422 P
(62) Divisional of application: 21771253.8
(71) Applicant: Takeda Pharmaceutical Company Limited, Osaka-shi, Osaka 540-8645 (JP)
(72) Inventor: ROBERTSON, Robbie James, Somerville, Massachusetts, 02145 (US); LING, Vincent, Walpole, Massachusetts, 02081 (US)
(74) Representative: Hoffmann Eitle

(57) **Abstract**

The present invention relates to an implantable cell chamber device capable of retaining cells but permitting diffusion of biomolecules. Also disclosed are methods for delivering a therapeutic biomolecule to a subject in need thereof on a continuous or semi-continuous basis, by administering to the subject a cell chamber device comprising cells that secrete the therapeutic biomolecule.

## Description

### RELATED APPLICATIONS

This application claims priority to U.S. Provisional Application No. 62/991,422, filed March 18, 2020. The entire contents of the foregoing priority application are incorporated by reference herein.

### SEQUENCE LISTING

The instant application contains a Sequence Listing which has been submitted electronically in ASCII format and is hereby incorporated by reference in its entirety. Said ASCII copy, created on March 11, 2021, is named T103022_1130WO_0465_1_SL.txt and is 66,553 bytes in size.

### FIELD OF THE INVENTION

The present invention relates to an implantable cell chamber device and related uses for delivery of therapeutic biomolecules to a subject in need thereof.

### BACKGROUND

Advances in biotechnology have made it possible to produce a variety of therapeutic biomolecules, such as proteins, for pharmaceutical applications using recombinant DNA techniques. Biomolecules are typically delivered by subcutaneous injection or intravenous infusion at regularly spaced intervals. The bioavailability of the molecule between administration of each bolus dose is a function of the rate of absorption, distribution, and elimination, with bioavailability typically peaking shortly after administration and subsequently tapering off. The pharmacokinetic (PK) profile of each biomolecule generally determines the spacing between each dose, with the goal of maintaining a drug concentration in the plasma that falls within a therapeutic window between toxicity and minimal efficacy. The need for frequent administrations can be costly and inconvenient for patients. In addition, the PK profile and dosing frequency of each biomolecule makes the drug concentration in each patient fluctuate between very high to very low and back, depending on the amount of the biomolecule present in each dose and the time interval between doses.

Because biomolecules can be larger and more complex than traditional inorganic drugs (i.e., possessing multiple functional groups in addition to complex three-dimensional structures), the formulation of such biomolecules can pose special problems. For example, for a protein to remain biologically active, a formulation must preserve the conformational integrity of at least a core sequence of the protein's amino acids, while at the same time protecting the protein's multiple functional groups from degradation. Proteins may suffer from a lack of stability during storage, and monoclonal and polyclonal antibodies in particular may be relatively unstable (See e.g., Wang, et al., J. Pharm Sci. 96:1-26 (2007)). Consequences of chemical or physical instability of therapeutic protein include a lowering of the effective administered dose, decreased safety of the therapy due to, for example irritation or immunological reactivity, and more frequent manufacturing due to short shelf life. A large number of formulation options are available, and not one approach or system is suitable for all proteins.

Accordingly, there is a need to discover suitable means to deliver therapeutic biomolecules to patients, which give rise to steady, therapeutically effective blood levels of biomolecules over an extended period of time, in a stable and convenient form.

### SUMMARY OF THE INVENTION

Provided herein is a cell chamber device capable of retaining cells, while allowing exchange of biomolecules between the interior and the exterior of the device. The device can be used to culture cells that perform functions that include, for example, secretion of a therapeutic biomolecule. Upon implantation of the cell chamber device into a subject, the device can provide a stable level of biomolecule delivery to the subject over an extended period of time. In addition or alternatively, cells in the chamber can perform physiologic functions, such as removing toxins or performing metabolic operations, due to the exchange of components between tissue surrounding or neighboring the device, and cells growing within it. The chamber comprises a layered scaffold composed of materials that induce little to no fibrotic reaction in mammalian hosts, induce little to no host immune reaction, retain cells on the inside of the chamber without cell leakage into the surrounding host tissue, prevent host cells from infiltrating into the chamber, and/or enable delivery of a therapeutic dosage of biomolecules into the host, e.g., into the bloodstream.

Accordingly, in one aspect, provided herein is a device comprising a multilayer scaffold surrounding a cell chamber, wherein the multilayer scaffold comprises an outer layer and an inner layer in contact with the cell chamber, and wherein the outer layer and the inner layer each comprise a nanofibrous polymer. In some embodiments, the nanofibrous polymer can include, for example, a nanofibrous polyester.

In some embodiments, the outer layer comprises nanofibrous polyethylene terephthalate, or nanofibrous polybutylene terephthalate. In some embodiments, the outer layer comprises a mixture of nanofibrous polyethylene terephthalate and polybutylene terephthalate. In certain embodiments, the outer layer comprises electrospun polyethylene terephthalate, electrospun polybutylene terephthalate, or a mixture of electrospun polyethylene terephthalate and polybutylene terephthalate. Other suitable polymers are described herein, including, for example, nanofibrous polytrimethylene terephthalate, or nanofibrous polyurethane.

In some embodiments, the inner layer comprises nanofibrous polyurethane. In certain embodiments, the inner layer comprises electrospun polyurethane.

In some embodiments, the inner layer comprises nanofibrous polyethylene terephthalate, nanofibrous polybutylene terephthalate, or a mixture of nanofibrous polyethylene terephthalate and polybutylene terephthalate.

In some embodiments, the inner layer comprises electrospun polyethylene terephthalate, electrospun polybutylene terephthalate, or a mixture of electrospun polyethylene terephthalate and polybutylene terephthalate.

In some embodiments, the outer layer and/or the inner layer comprises one or more charged surface modifications.

In some embodiments, the outer layer and/or the inner layer have a net positive charge. In certain embodiments, the scaffold has been treated with ethylenediamine.

In some embodiments, the outer layer and/or the inner layer have a net negative charge. In certain embodiments, the scaffold has been treated with sodium hydroxide.

In some embodiments, the outer layer and/or the inner layer comprises an anti-inflammatory agent. In some embodiments, the anti-inflammatory agent is a calcineurin inhibitor. In one embodiment, the anti-inflammatory agent is tacrolimus. In some embodiments, the anti-inflammatory agent is pyridone. In one embodiment, the anti-inflammatory agent is pirfenidone. In one embodiment the anti-inflammatory agent is a phosphodiesterase inhibitor. In one embodiment, the anti-inflammatory agent is roflumilast.

In some embodiments, the outer layer and/or the inner layer comprise nanopores. In certain embodiments, the nanopores are sized to permit the passage of biomolecules. In certain embodiments, the biomolecules are 250 kilodaltons (kDa) or less.

In some embodiments, the nanopores are sized to permit the passage of an antibody, or antigen-binding portion thereof. In some embodiments, the nanopores are sized to prevent the passage of cells. In some embodiments, the nanopores are sized to prevent cells on one side of the multilayer scaffold from contacting cells on the other side of the multilayer scaffold.

In some embodiments, the nanopores have a diameter of 1 µm or less. In some embodiments, the nanopores have a diameter of 0.5 µm or less.

In some embodiments, the multilayer scaffold further comprises a nanoporous membrane positioned between the inner layer and the outer layer. In some embodiments, the nanoporous membrane is solid, i.e., non-nanofibrous, polymer layer. In other embodiments, the nanoporous membrane can comprise an additional layer of nanofibrous polymer. In some embodiments, the nanoporous membrane comprises polybutylene terephthalate. In some embodiments, the nanoporous membrane comprises a solid, i.e., non-nanofibrous, layer of polybutylene terephthalate. In certain embodiments, the nanoporous membrane comprises nanofibrous polybutylene terephthalate. In some embodiments, the nanoporous membrane comprises membrane nanopores sized to permit the passage of biomolecules. In certain embodiments, the biomolecules are 250 kDa or less.

In some embodiments, the membrane pores are sized to permit the passage of an antibody, or antigen-binding portion thereof. In some embodiments, the membrane pores are sized to prevent the passage of cells. In some embodiments, the membrane pores are sized to prevent cells on one side of the multilayer scaffold from contacting cells on the other side of the multilayer scaffold.

In another aspect, provided herein is a device comprising a multilayer scaffold surrounding a cell chamber, wherein the multilayer scaffold comprises (i) an outer layer comprising nanofibrous polyethylene terephthalate and polybutylene terephthalate; (ii) a non-nanofibrous membrane positioned between the inner layer and the outer layer, wherein the membrane comprises submicron and/or nanopores sized to prevent the passage of cells across the membrane; and (iii) an inner layer comprising nanofibrous polyurethane.

In a further aspect, provided herein is a device comprising a multilayer scaffold surrounding a cell chamber, wherein the multilayer scaffold comprises (i) an outer layer comprising nanofibrous polyethylene terephthalate and polybutylene terephthalate; (ii) a non-nanofibrous membrane positioned between the inner layer and the outer layer, wherein the membrane comprises nanopores sized to prevent the passage of cells across the membrane; and (iii) an inner layer comprising nanofibrous polyethylene terephthalate and polybutylene terephthalate.

In some embodiments, the membrane nanopores have a diameter of 1 µm or less. In some embodiments, the membrane nanopores have a diameter of 0.5 µm or less. In some embodiments, the membrane pores have a diameter of about 0.2-0.6 µm. In some embodiments, the membrane pores have a diameter of about 0.4 µm.

In some embodiments, the device further comprises a loading port to permit the loading of cells into the cell chamber.

In some embodiments, the device comprises a total thickness of 250 µm or less. In some embodiments, the device comprises a total thickness of 150 µm or less.

In some embodiments, the cell chamber is sized to accommodate up to 1x10⁹ cells. In some embodiments, the cell chamber is sized to accommodate up to 1x10⁷ cells.

In another aspect, provided herein is a device comprising a multilayer scaffold surrounding a cell chamber, wherein the multilayer scaffold comprises an outer layer comprising nanofibrous polyethylene terephthalate and polybutylene terephthalate, and an inner layer comprising nanofibrous polyurethane, wherein the outer layer and the inner layer comprise nanopores having a diameter of 1 µm or less.

In some embodiments, the device comprises a polymeric film with a pore size less than or equal to 1 µm positioned between the outer layer and the inner layer of the scaffold.

In some embodiments, the device comprises a polymeric film with a pore size less than or equal to 1 µm, wherein one side of the film is coated with a first electrospun polymer, and the other side of the film is coated with a second electrospun polymer. In some embodiments, the first electrospun polymer and the second electrospun polymer comprise the same polymer. In other embodiments, the first electrospun polymer and the second electrospun polymer comprise different polymers. In some embodiments, the first electrospun polymer and/or the second electrospun polymer comprises nPET, nPBT, or a combination thereof. In some embodiments, the first electrospun polymer and/or the second electrospun polymer comprises nPU. In some embodiments, the first electrospun polymer comprises nPET-PBT, and the second electrospun polymer comprises nPU.

In some embodiments, the polymeric film comprises a poly(ethylene terephthalate). embodiments, the polymeric film comprises pores having a diameter of about 0.2 µm - 1 µm. In various embodiments, the polymeric film comprises pores having a diameter of about 0.2 µm, 0.3 µm, 0.4 µm, 0.5 µm, 0.6 µm, 0.7 µm, 0.8 µm, 0.9 µm or 1.0 µm. In some embodiments, the polymeric film comprises pores having a diameter of about 0.4 µm.

In some embodiments, the cell chamber comprises cells. In some embodiments, the cells adhere to the inner layer of the scaffold. In some embodiments, the cells comprise retinal pigment epithelial cells. In certain embodiments, the cells comprise ARPE-19 cells. In some embodiments, the cells comprise hepatocytes. In some embodiments, the cells comprise islets.

In some embodiments, the device comprises about 1x10⁵ cells to about 1x10⁸ cells.

In some embodiments, the device comprises about 1x10⁶ cells to about 1x10⁷ cells.

In some embodiments, the cells secrete a recombinant peptide or protein. In certain embodiments, the cells secrete a protein selected from the group consisting of a growth factor, a hormone, a cytokine, a prostaglandin, an enzyme or a combination thereof.

In some embodiments, the cells secrete a peptide therapeutic (e.g., a peptide therapeutic for gastrointestinal use, such as for the treatment of short bowel syndrome).

In some embodiments, the cells secrete an enzyme. In one embodiment, the enzyme is idursulfase. In one embodiment, the enzyme is arylsulfatase A. In one embodiment, the enzyme is laronidase.

In some embodiments, the cells secrete an antibody, or antigen-binding portion thereof. In certain embodiments, the cells secrete a chimeric antibody, or antigen-binding portion thereof. In some embodiments, the cells secrete a humanized antibody, or antigen-binding portion thereof. In some embodiments, the cells secrete a human antibody, or antigen-binding portion thereof. In some embodiments, the cells secrete a monoclonal antibody. In some embodiments, the cells secrete an antibody fragment selected from the group consisting of a Fab, a F(ab')₂, an scFv, a tandem scFv, a diabody, a minibody, and a single domain antibody.

In some embodiments, the cells secrete an antibody, or antigen-binding portion thereof, that specifically binds an antigen selected from the group consisting of α4β7 integrin, integrin β7, TNFα, IL-12, IL-23, or CD20. In some embodiments, the cells secrete an antibody selected from vedolizumab, abrilumab, adalimumab, etrolizumab, certolizumab, golimumab, ustekinumab, infliximab, rituximab, and natalizumab.

In some embodiments, the cells secrete an antibody, or antigen-binding portion thereof, that specifically binds α4β7 integrin. In certain embodiments, the cells secrete vedolizumab, or an antigen-binding portion thereof.

In some embodiments, the cell chamber comprises a population of cells having a three-dimensional architecture. In one embodiment, the population of cells having a three-dimensional architecture is a tissue explant. In some embodiments, the tissue is liver tissue, kidney tissue, or pancreatic tissue. In other embodiments, the population of cells having a three-dimensional architecture are organized as spheroids. In some embodiments, the spheroids comprise hepatocytes, liver cells, or pancreatic cells. In certain embodiments, the spheroids are organized around a sinusoid or a duct.

In another aspect, provided herein is a method of delivering a biomolecule to a subject, comprising administering to the subject a device as described herein, wherein the cell chamber of the device comprises cells secreting the biomolecule.

In another aspect, provided herein is a method of delivering a recombinant peptide or a recombinant protein to a subject, comprising administering to the subject a device of the present disclosure.

In another aspect, provided herein is a method of delivering an antibody, or antigen-binding portion thereof, to a subject, comprising administering to the subject a device of the present disclosure.

In some embodiments, the device is administered to the subject by implantation at a site selected from under the skin (subcutaneous implantation), on the omentum, in the liver, in the brain, or in the spinal canal.

In some embodiments, the device is implanted in the subject for at least 30 days. In some embodiments, the device is implanted in the subject for at least 90 days. In some embodiments, the device is implanted in the subject for at least 120 days.

In some embodiments, the device is implanted in the subject for at least 1 year.

In some embodiments, the plasma concentration of the biomolecule, recombinant peptide, or recombinant protein is at least 5 µg/mL in the subject for a period of at least 60 days following implantation.

In some embodiments, the plasma concentration of the antibody, or antigen-binding portion thereof, is at least 5 µg/mL in the subject for a period of at least 60 days following implantation.

In another aspect, provided herein is a method of treating a subject having Crohn's Disease or Ulcerative Colitis, comprising administering to the subject a device of the present disclosure.

In another aspect, provided herein is a method of treating a subject having primary sclerosing cholangitis, comprising administering to the subject a device of the present disclosure.

In another aspect, provided herein is a method of treating a subject having eosinophilic esophagitis, comprising administering to the subject a device of the present disclosure.

In another aspect, provided herein is a method of treating a subject having autoimmune hepatitis, comprising administering to the subject a device of the present disclosure.

In another aspect, provided herein is a method of treating a subject having short bowel syndrome, comprising administering to the subject a device of the present disclosure.

In another aspect, provided herein is a method of treating a subject having mucopolysaccharidosis type I (MPS I), comprising administering to the subject a device of the present disclosure (e.g., a device where the cells secrete an enzyme, such as laronidase). In certain embodiments, the device comprises cells that secrete an enzyme comprising the amino acid sequence of SEQ ID NO :50, or an enzyme having at least 90%, 92%, 94%, 95%, 96%, 98%, or 99% to SEQ ID NO 50.

In another aspect, provided herein is a method of treating a subject having mucopolysaccharidosis type II (MPS II), comprising administering to the subject a device of the present disclosure (e.g., a device where the cells secrete an enzyme, such as idursulfase). In certain embodiments, the device comprises cells that secrete an enzyme comprising the amino acid sequence of SEQ ID NO :51, or an enzyme having at least 90%, 92%, 94%, 95%, 96%, 98%, or 99% to SEQ ID NO :51.

In another aspect, provided herein is a method of treating a subject having metachromatic leukodystrophy (MLD), comprising administering to the subject a device of the present disclosure (e.g., a device where the cells secrete an enzyme, such as arylsulfatase A). In certain embodiments, the device comprises cells that secrete an enzyme comprising the amino acid sequence of SEQ ID NO :52, or an enzyme having at least 90%, 92%, 94%, 95%, 96%, 98%, or 99% to SEQ ID NO :52.

In another aspect, provided herein is a device comprising a single-layer scaffold surrounding a cell chamber. Such a cell chamber device can be used, in some embodiments, to culture cells growing in three-dimensional culture, or as tissue explants. In some embodiments, the cell chamber can comprise cells having a three-dimensional architecture. In some embodiments, the single-layer scaffold can comprise a nanofibrous polymer as described herein. In some embodiments, the nanofibrous polymer is a polyester, e.g., nanofibrous polyethylene terephthalate or nanofibrous polybutylene terephthalate. In some embodiments, the single-layer scaffold comprises a mixture of nanofibrous polyethylene terephthalate and polybutylene terephthalate. In some embodiments, the single-layer scaffold comprises nanofibrous polyurethane.

In some embodiments, the cell chamber comprises cells having a three-dimensional architecture. In one embodiment, the cells having a three-dimensional architecture are a tissue explant. In some embodiments, the tissue is liver tissue, kidney tissue, or pancreatic tissue. In other embodiments, the cells having a three-dimensional architecture are organized as spheroids. In some embodiments, the spheroids can comprise hepatocytes, liver cells, or pancreatic cells. In certain embodiments, the spheroids are organized around a sinusoid or a duct. In some embodiments, the cells are hepatocytes. In other embodiments, the cells are islet cells.

### BRIEF DESCRIPTION OF THE DRAWINGS

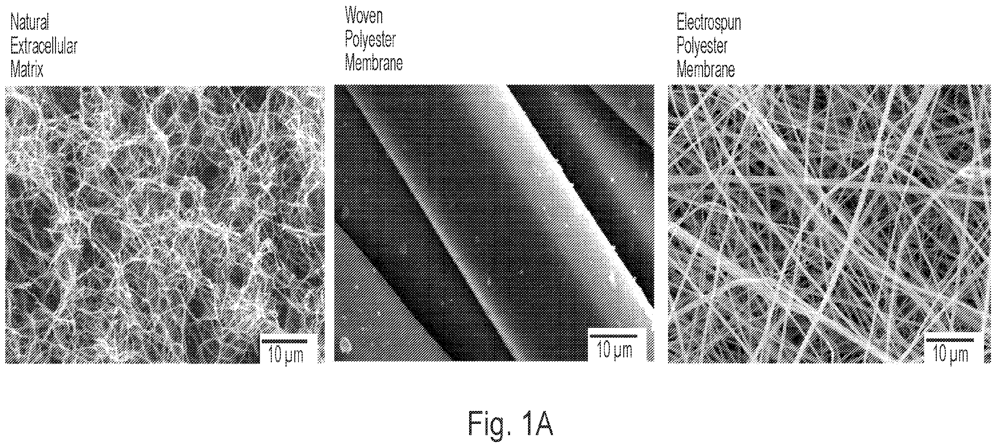

**Fig. 1A** shows scanning electron micrographs of a natural extracellular matrix, an implantable woven polyester membrane, and an electrospun polyester membrane.

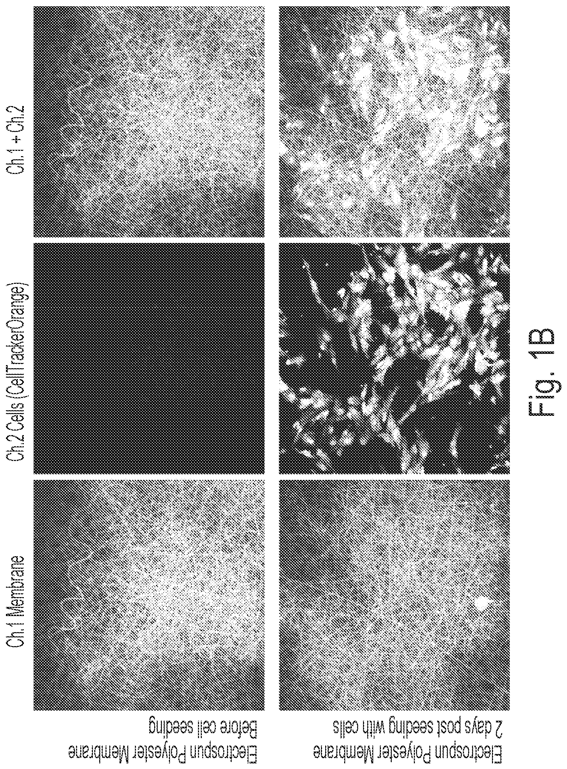

**Fig. 1B** shows confocal images of an electrospun nanofibrous polyurethane (nPU) polymers (as part of a nPET-PBT/PU scaffold) two days after seeding the nPU layer with human ARPE-19 cells.

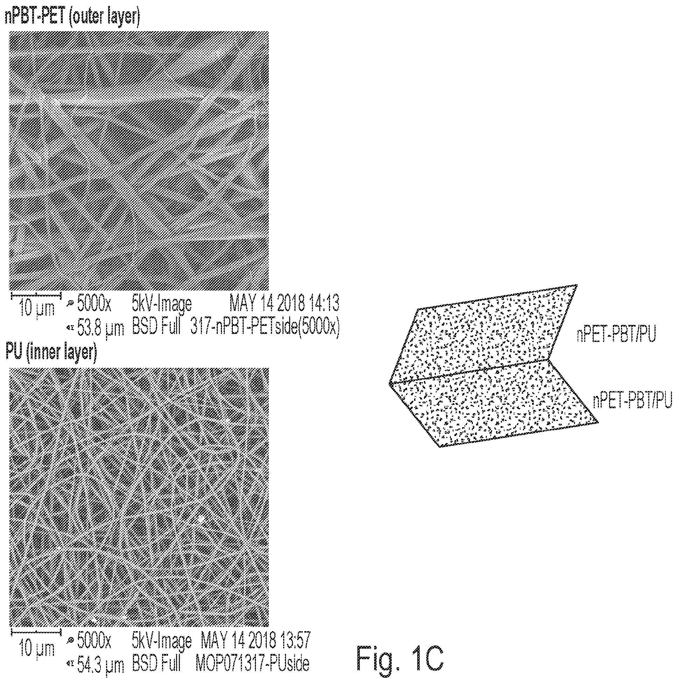

**Fig. 1C** depicts an exemplary cell chamber device. The cell chamber comprises a bilaminate scaffold surrounding a cell chamber. The bilaminate scaffold contains an outer external facing layer comprising electrospun nanofibrous polyethylene terephthalate (PET) and polybutylene terephthalate (PBT) (nPET-PBT) (top left), and an inner internal-facing layer comprising electrospun nanofibrous polyurethane (nPU) polymers (bottom left). In exemplary embodiments, two scaffold sheets, or one folded sheet, can be fabricated to surround a cell chamber (top right).

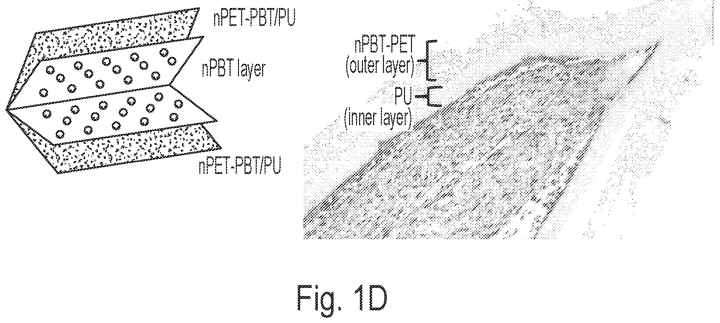

**Fig. 1D** depicts one embodiment of a multi-scaffold device. This device contains a first bilaminate scaffold that comprises an outer, external-facing layer of electrospun nPET-PBT, and an inner, internal-facing layer of electrospun nPU. In addition, the device contains a second scaffold that comprises a porous layer of electrospun nanofibrous PBT. ARPE-19 cells seeded in the cell chamber can be visualized by Hematoxylin & Eosin (H&E) staining (bottom right).

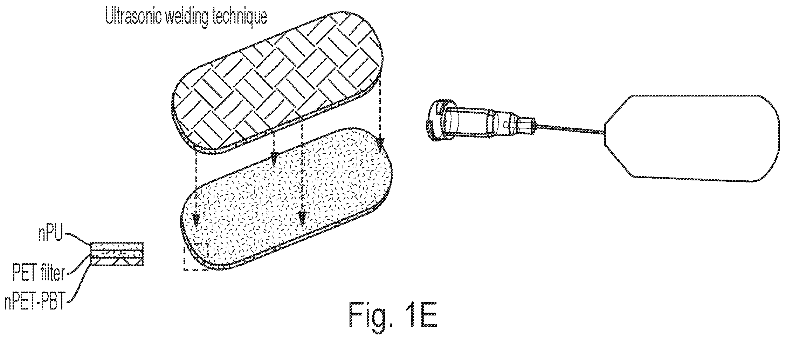

**Fig. 1E** depicts one embodiment of a multi-layer scaffold device comprising an outer, external-facing layer of electrospun nPET-PBT, a central poly(ethylene terephthalate) film with a pre-selected pore size, and an inner, internal-facing layer of electrospun nPU. An illustration of the cell chamber device is shown in the left panel and a schematic of the cell chamber device is shown in the right panel.

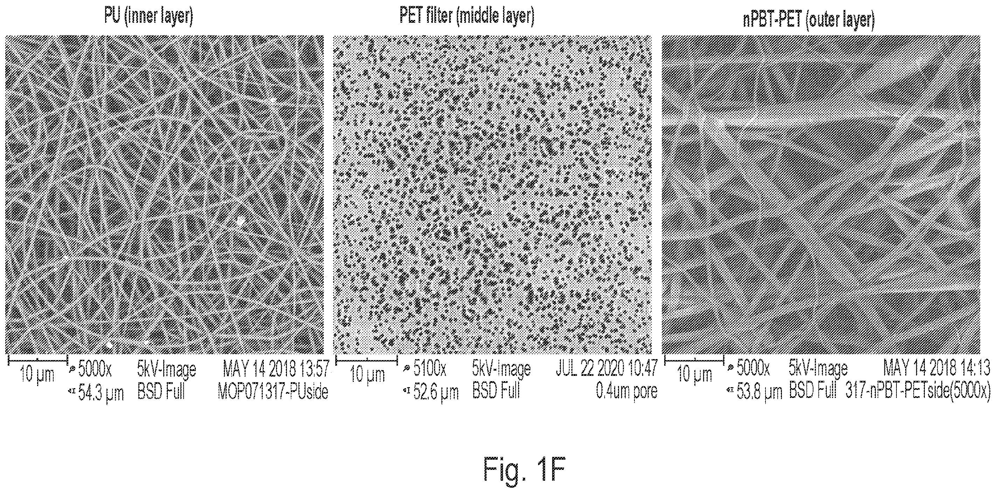

**Fig. 1F** depicts a scanning electron micrograph of each layer in the cell chamber device depicted in Fig. 1E.

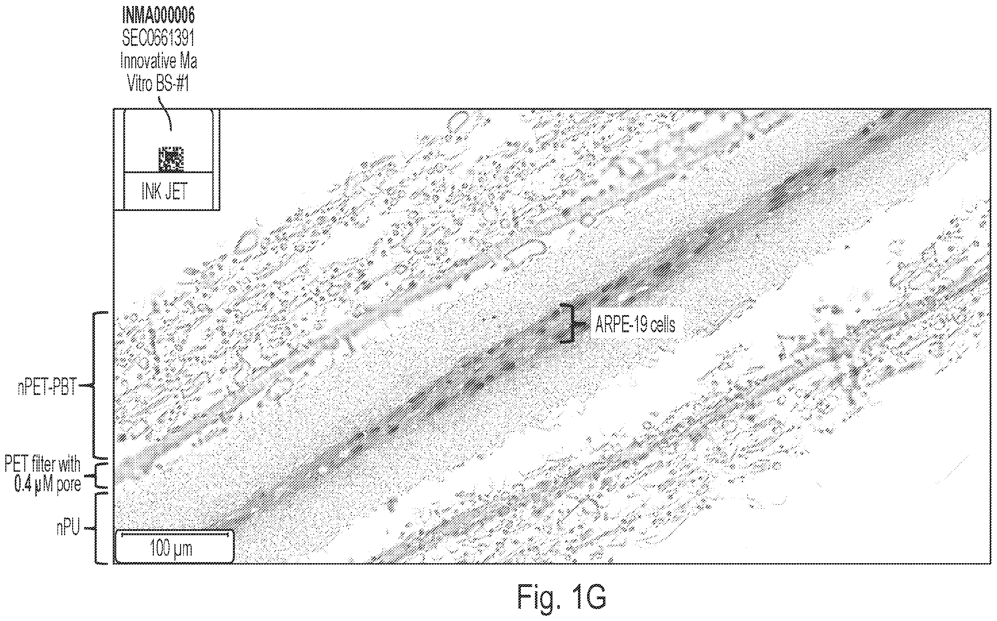

**Fig. 1G** depicts an image of a cell chamber comprising an outer, external-facing layer of electrospun nPET-PBT, a central PET membrane with a 0.4 um pore size, and an inner, internal-facing layer of electrospun nPU. The cell chamber was seeded with ARPE-19 cells and visualized by Hematoxylin & Eosin (H&E) staining 44 days following cell seeding.

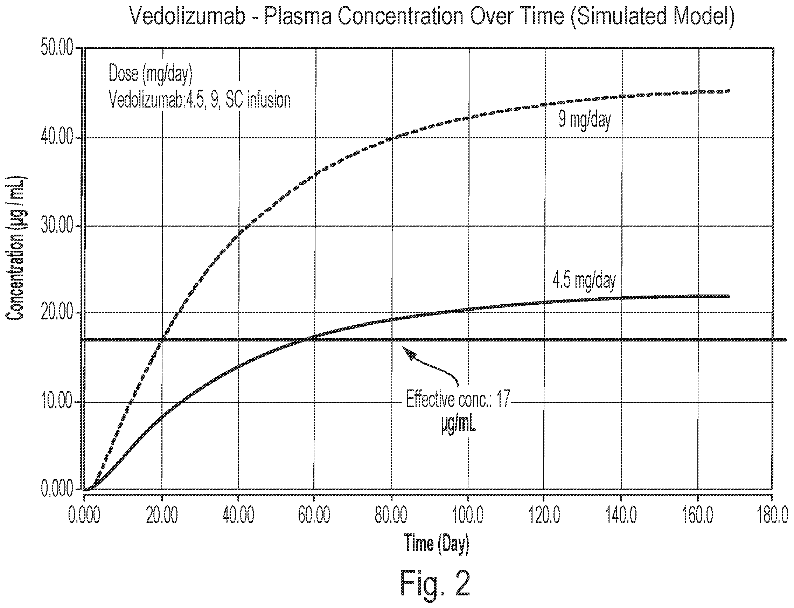

**Fig. 2** shows a simulated model of the predicted plasma concentration of vedolizumab over time that could be attained from a cell chamber device comprising cells that secrete 4.5 mg vedolizumab/day or 9 mg vedolizumab /day. The simulated model was based on the known pharmacokinetics of vedolizumab following subcutaneous infusion. A therapeutically effective plasma concentration of vedolizumab is indicated by the bolded line at 17 µg/mL.

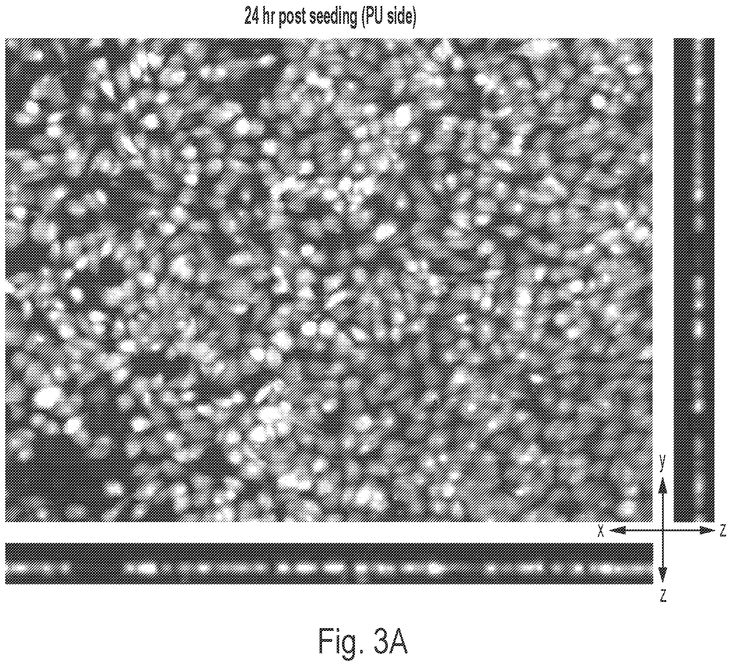

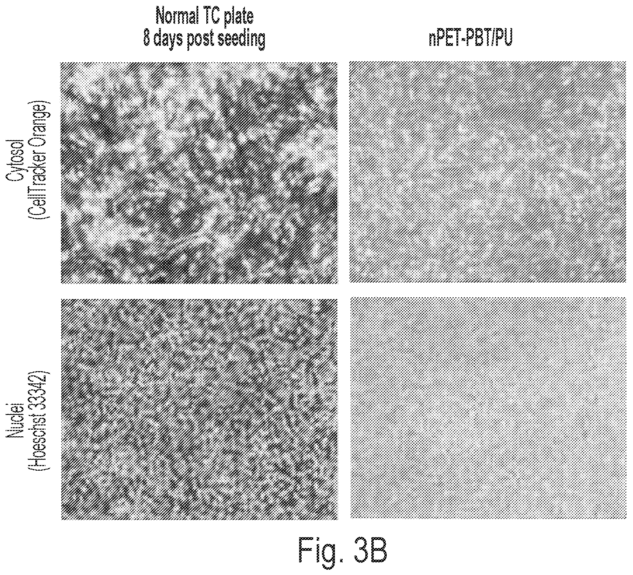

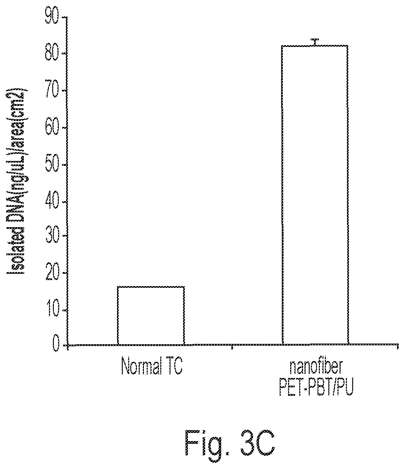

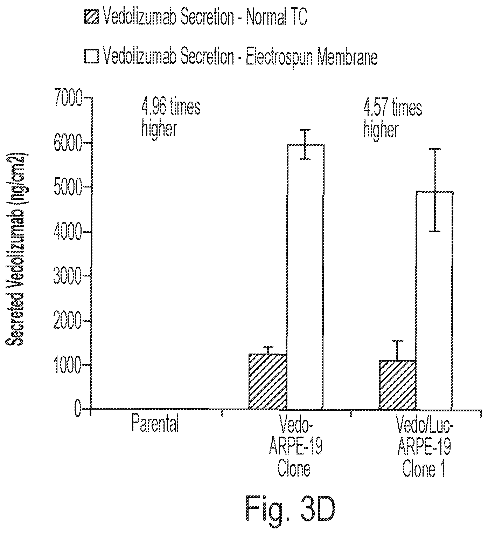

**Figs. 3A-3D** depict the results of an *in vitro* assay to assess the ability of vedolizumab-secreting ARPE-19 cells to grow on a scaffold composed of nanofibrous polybutylene terephthalate (PBT) and polyethylene terephthalate (PET) on one side and nanofibrous polyurethane (PU) polymers on the other side (a nPET-PBT/PU scaffold). **Fig. 3A** shows a fluorescent micrograph of vedolizumab/luciferase-ARPE-19 cells on a nPET-PBT/PU scaffold 24 hours post-seeding on the internal PU side, as imaged from the PU side. **Fig. 3B** shows fluorescent micrographs of the nuclei (Hoescht 33342) or cytosol (Cell Tracker Orange) of vedolizumab/luciferase-ARPE-19 cells 8 days post seeding on a normal tissue culture (TC) plate or a nPET-PBT/PU scaffold. **Fig. 3C** graphically depicts the amount of DNA isolated from vedolizumab/luciferase-ARPE-19 cells on a normal tissue culture plate as compared to a nPBT-PET/PU scaffold 40 days post-seeding. **Fig. 3D** graphically depicts the amount of vedolizumab secreted from ARPE-19 cells on a normal tissue culture plate (left bar) as compared to a nPBT-PET/PU scaffold (right bar).

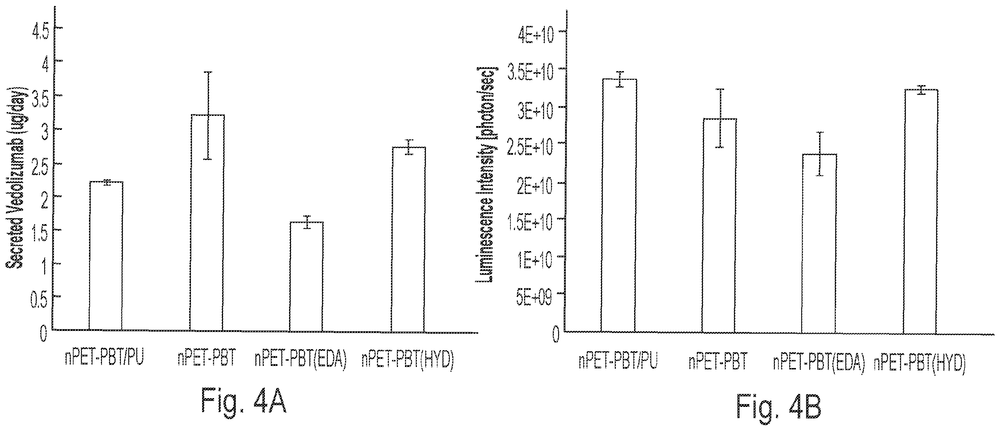

**Figs. 4A-4B** graphically depict the results of an *in vitro* cell seeding assay of vedolizumab/luciferase-ARPE-19 cells seeded on nPET-PBT with charged surface modifications. Discs comprising nPET-PBT/PU were treated with liquid ethylenediamine (EDA) to generate a positively charged surface, or liquid sodium hydroxide (HYD) to generate a negatively charged surface. **Fig. 4A** graphically depicts the amount of vedolizumab secreted from ARPE-19 cells seeded on each of the indicated materials. **Fig. 4B** graphically depicts the level of luminescence generated by vedolizumab/luciferase-ARPE-19 cells seeded on each of the indicated materials.

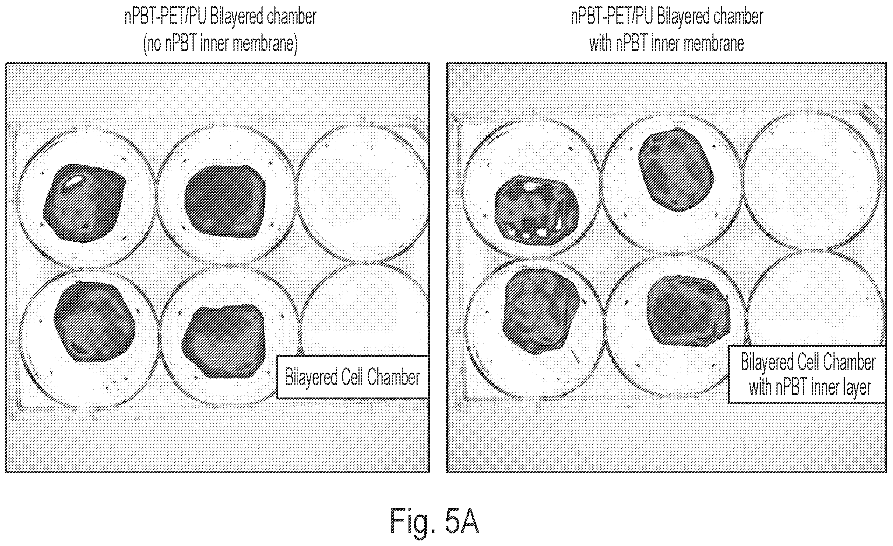

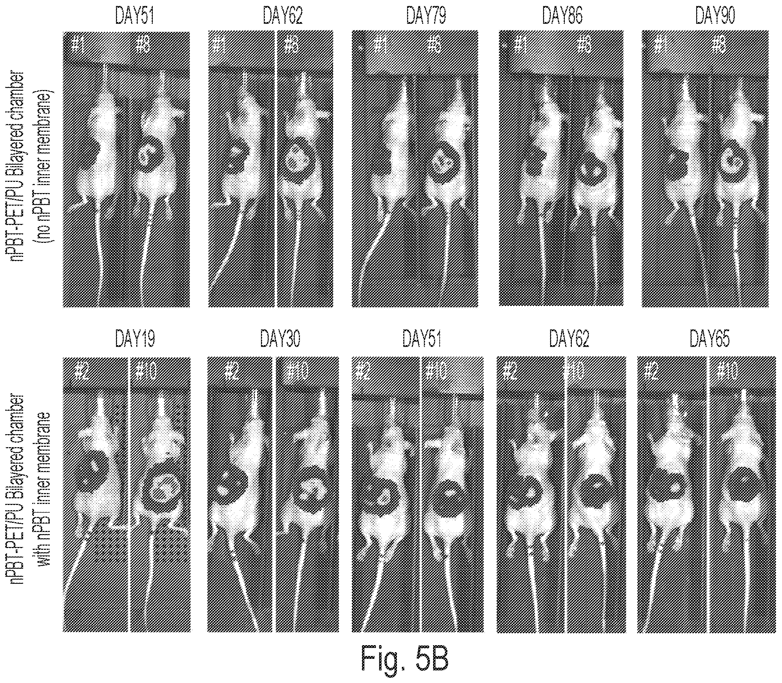

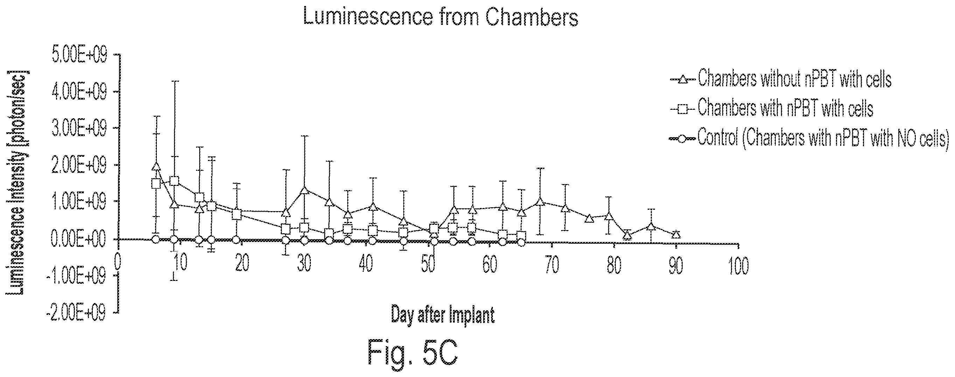

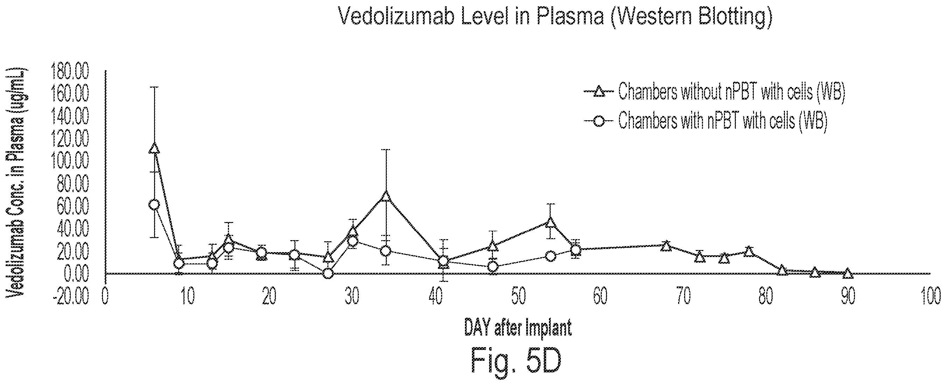

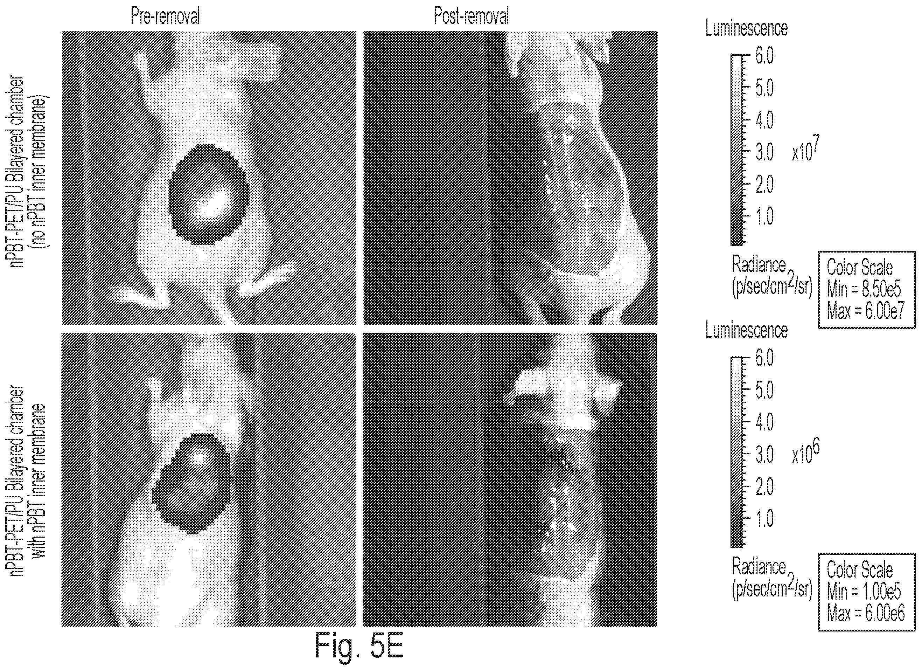

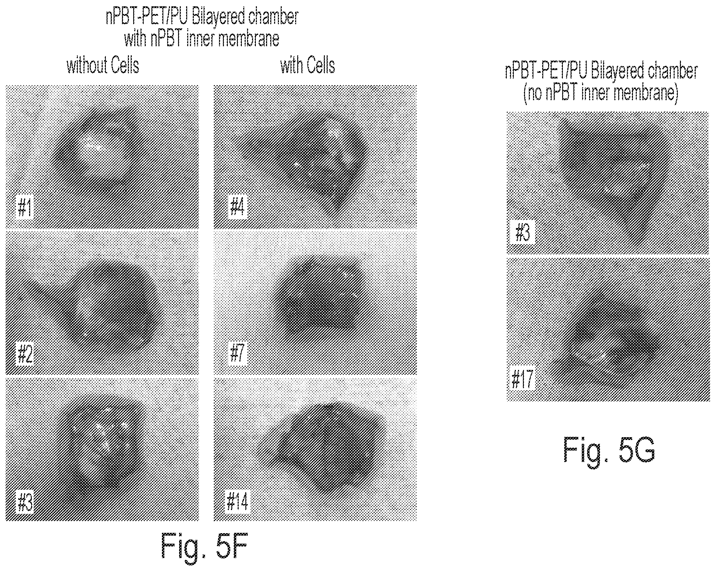

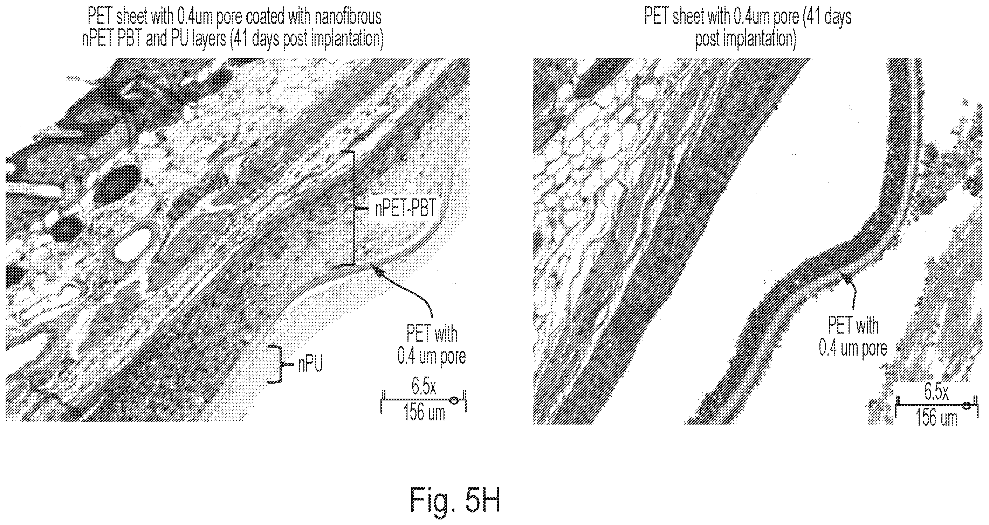

**Figs. 5A - 5H** depict the results of a study assessing *in vitro* and *in vivo* cell distribution in a cell chamber comprising a nPET-PBT/PU scaffold with or without an inner nPBT scaffold layer. **Fig. 5A** shows luminescent images demonstrating the *in vitro* cell distribution of vedolizumab/luciferase-ARPE-19 cells growing on cell chambers comprising a nPBT-PET/PU scaffold with or without an inner nPBT scaffold. **Fig. 5B** shows luminescent imaging of mice, demonstrating the cell distribution of vedolizumab/luciferase-ARPE-19 cells growing in cell chambers comprising a nPBT-PET/PU scaffold with or without an inner nPBT scaffold after the indicated number of days post-implantation. **Fig. 5C** graphically depicts the luminescence intensity over time in mice implanted with cell chambers comprising vedolizumab/luciferase-ARPE-19 cells and a nPBT-PET/PU scaffold with or without an inner nPBT scaffold layer. **Fig. 5D** graphically depicts the plasma concentration (as detected by western blot) over time in mice implanted with cell chambers comprising vedolizumab/luciferase-ARPE-19 cells and a PBT-PET/PU scaffold with or without an inner nPBT scaffold layer. The bottom panel presents the results of a western blot detecting vedolizumab in plasma at days 68-78 following implantation of the cell chamber. **Fig. 5E** shows luminescent imaging of mice 42 days post-transplant prior to removal of the cell chamber (left column) and after removal of the cell chamber (right column). **Figs. 5F and 5G** show images of nPBT-PET/PU cell chambers with an inner nPBT scaffold layer (**Fig. 5F**; without cells (left column) or with cells (right column)) or without an inner nPBT scaffold layer (**Fig. 5G**) after being removed from nude mice 42 days post-transplant, indicating that no significant fibrotic response was observed visually. **Fig. 5H** depicts H&E stained images of the area surrounding a scaffold containing a porous non-nanofibrous PET sheet coated with nanofibrous nPET-PBT and nPU (left panel), and the area surrounding a porous non-nanofibrous PET sheet without a nanofibrous coating (right panel), 41 days following implantation in mice.

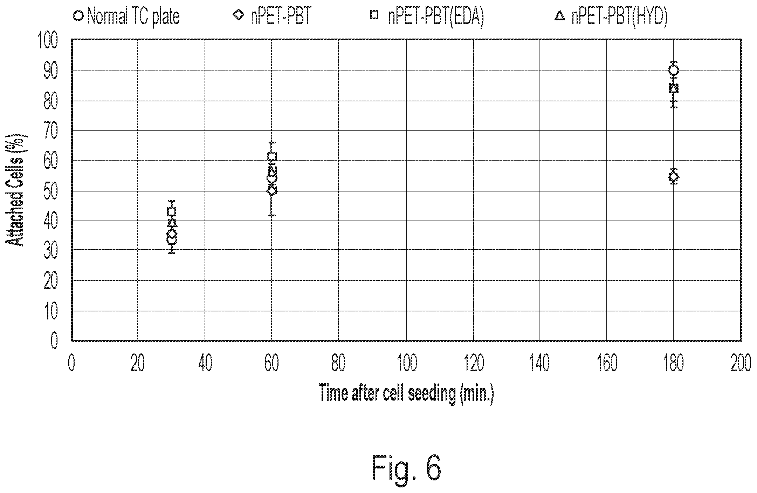

**Fig. 6** graphically depicts the results of an *in vitro* cell attachment assay showing the percentage of attached cells on discs composed of nPET-PBT, nPET-PBT (Ethylene Diamine (EDA)), or nPET-PBT (Sodium Hydroxide (HYD)) as compared to a normal tissue culture (TC) plate as a function of time after cell seeding. 400,000 cells were seeded per disc.

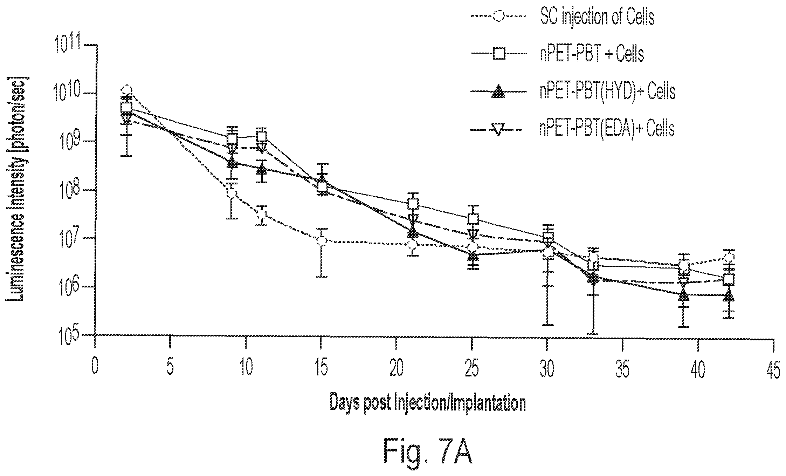

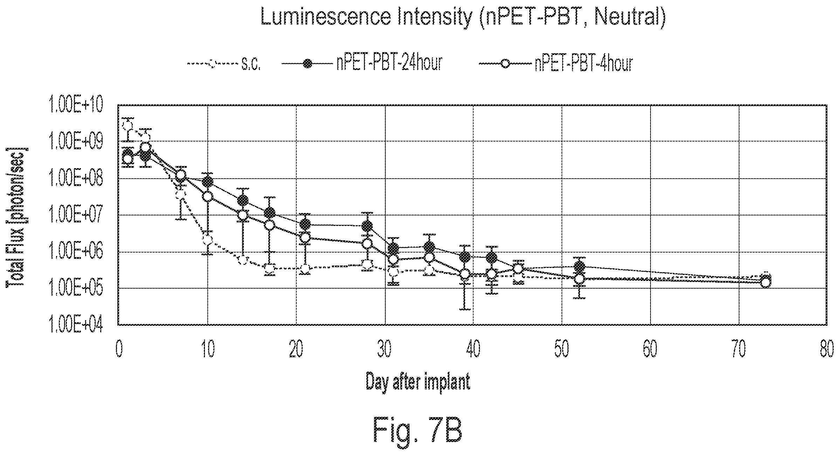

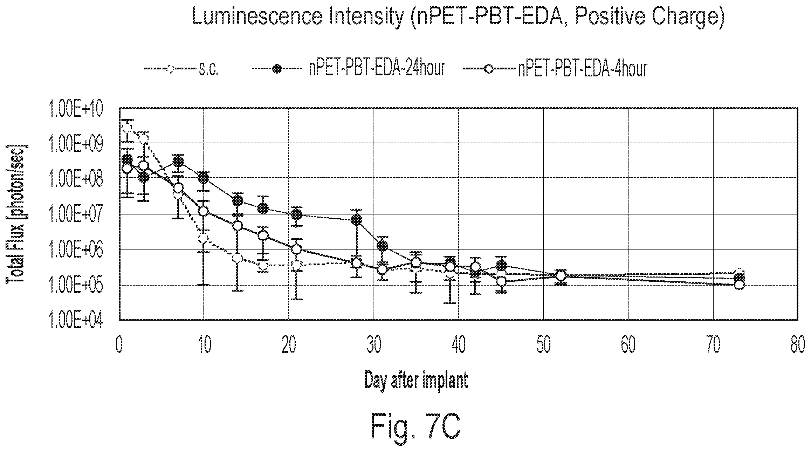

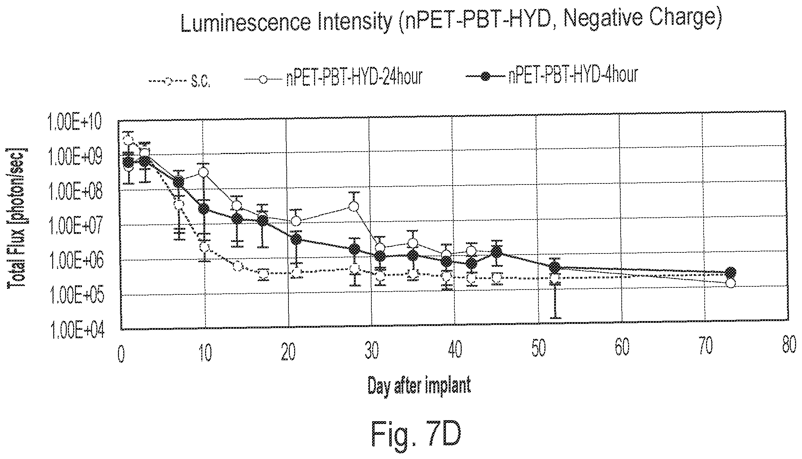

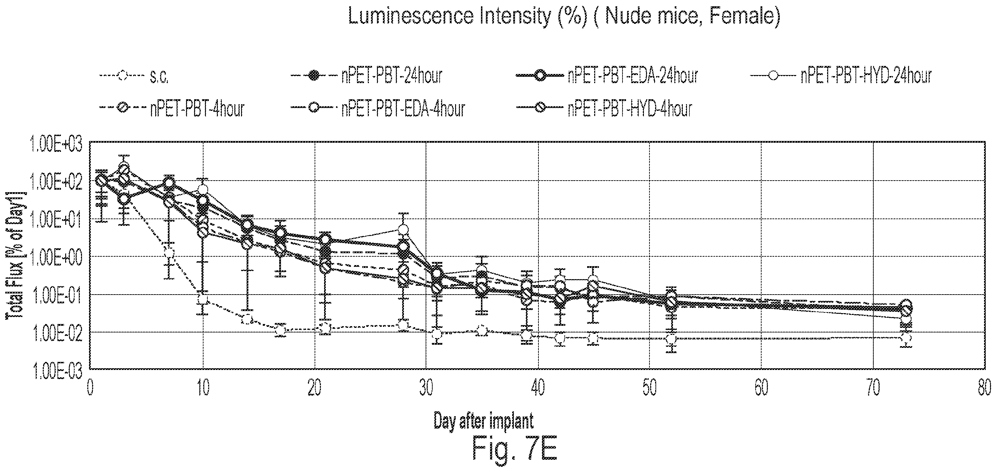

**Figs. 7A-7E** show the results of an *in vivo* study, in which the retention of vedolizumab/luciferase-ARPE-19 cells was assayed by quantifying the luminescence intensity over time (Days post implantation) in nude mice transplanted with discs composed of nPET-PBT (**Figs. 7A****,** **7B**, **and** **7E**), positively-charged nPET-PBT(EDA) (**Figs. 7A****,** **7C****, and** **7E**), or negatively-charged nPET-PBT(HYD) (**Figs. 7A****,** **7D**, **and** **7E**). Twenty-four hours or four hours after loading, the scaffolds were administered into mice by subcutaneous implantation (n=4 mice). Cells administered to mice by subcutaneous injection were assessed as a comparator. **Fig. 7A** depicts the results of the study over the course of 45 days. **Figs. 7B-7E** depict the results of the study over the course of 80 days.

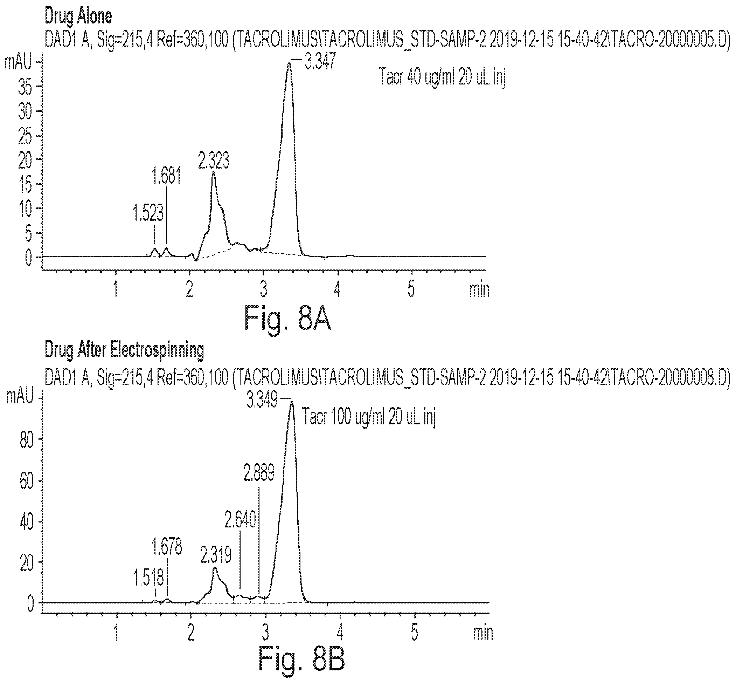

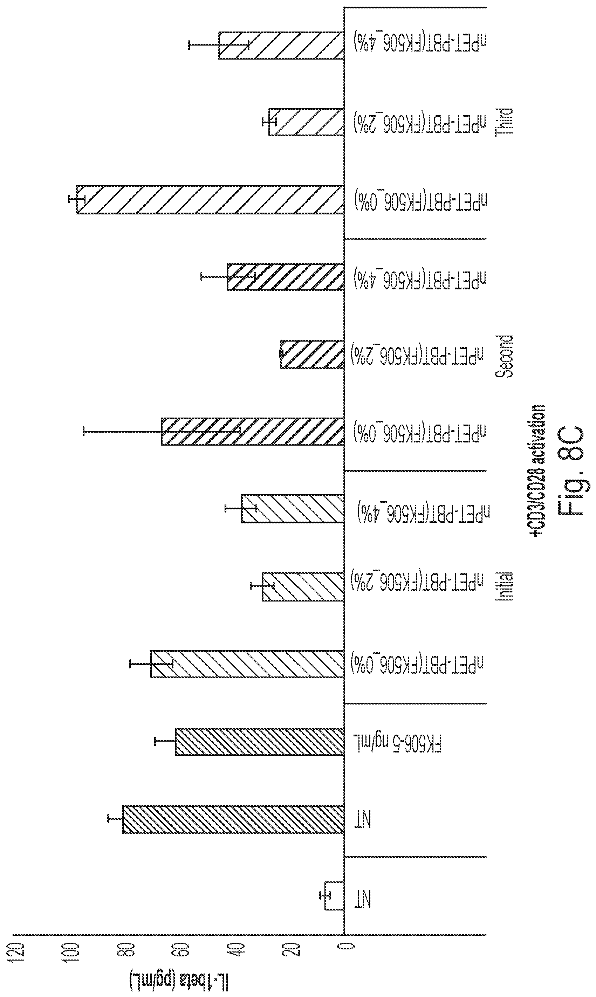

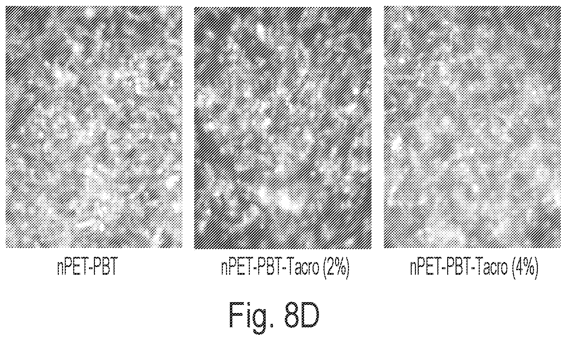

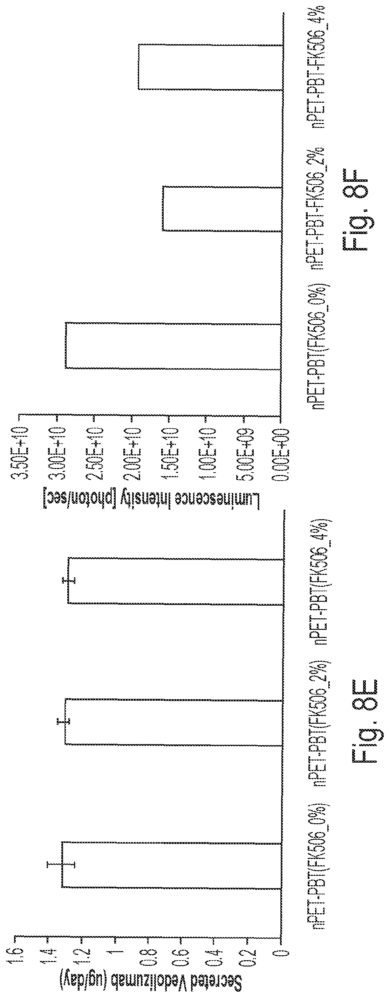

**Figs. 8A-8F** graphically depicts the results of an *in vitro* cell seeding assay of vedolizumab/luciferase-ARPE-19 cells seeded on nPET-PBT loaded with tacrolimus (FK506). Discs comprising nPET-PBT were treated with a solution comprising 0%, 2%, or 4% tacrolimus. **Figs. 8A** and **8B** graphically depict chromatograms from a high performance liquid chromatography (HPLC) analysis of tacrolimus alone (**Fig. 8A**) or after extraction from nPET-PBT treated with tacrolimus (**Fig. 8B**). **Fig. 8C** graphically depicts the results of a T cell activation assay, in which T cell activation (as measured by IL-1β production) was assessed following exposure of human PBMCs to media surrounding tacrolimus-loaded nPET-PBT. **Fig. 8D** shows fluorescent micrographs of vedolizumab/luciferase-ARPE-19 cells on nPET-PBT loaded with 0%, 2%, or 4% tacrolimus. **Fig. 8E** graphically depicts the amount of vedolizumab secreted from vedolizumab/luciferase-ARPE-19 cells seeded on nPET-PBT loaded with 0%, 2%, or 4% tacrolimus. **Fig. 8F** graphically depicts the levels of luminescence generated by vedolizumab/luciferase-ARPE-19 cells seeded on nPET-PBT loaded with 0%, 2%, or 4% tacrolimus.

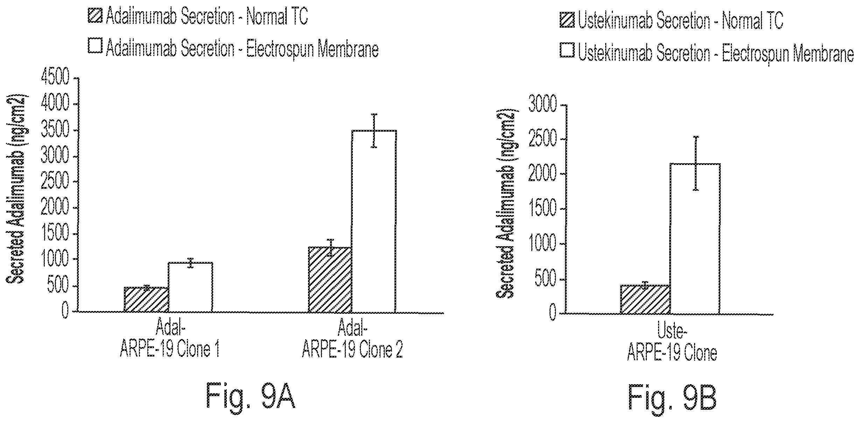

**Figs. 9A and 9B** graphically depict the amount of antibody secretion from adalimumab / ARPE-19 cells (**Fig. 9A**) or ustekinumab/ARPE-19 cells (**Fig. 9B**) on a normal tissue culture plate (left bar) as compared to a nPBT-PET/PU scaffold (right bar).

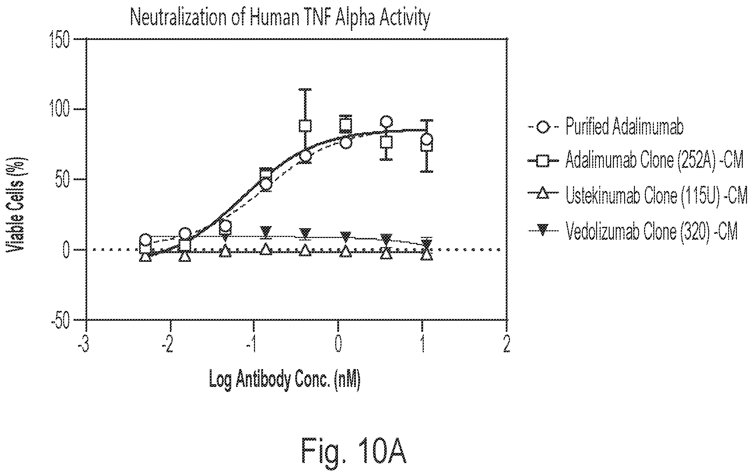

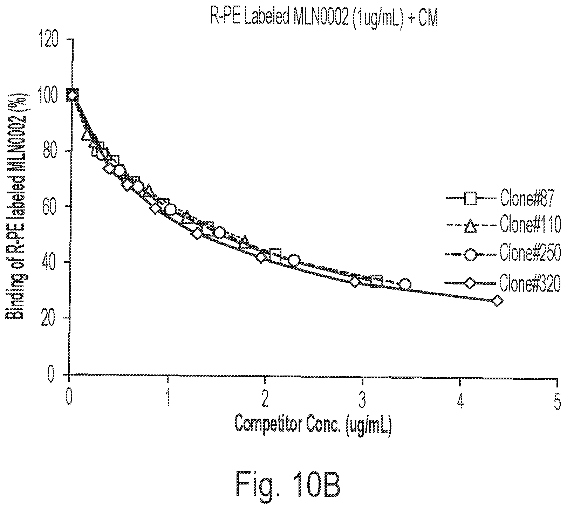

**Figs. 10A** **and** **10B** graphically depict the results of functional assays to test for the activity of adalimumab (**Fig. 10A**) or vedolizumab (**Fig. 10B**), secreted by ARPE-19 cells grown in serum-free media.

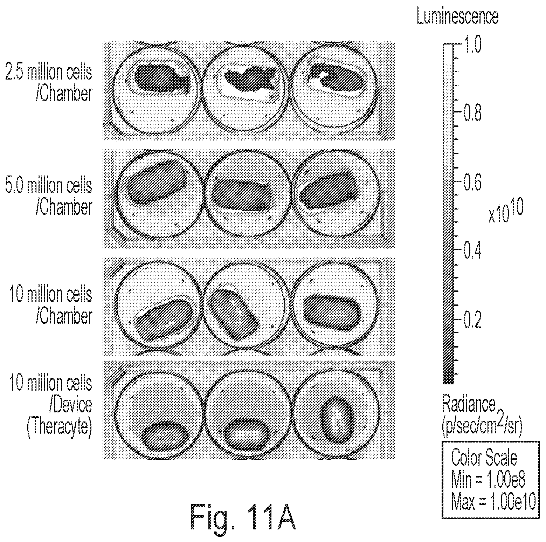

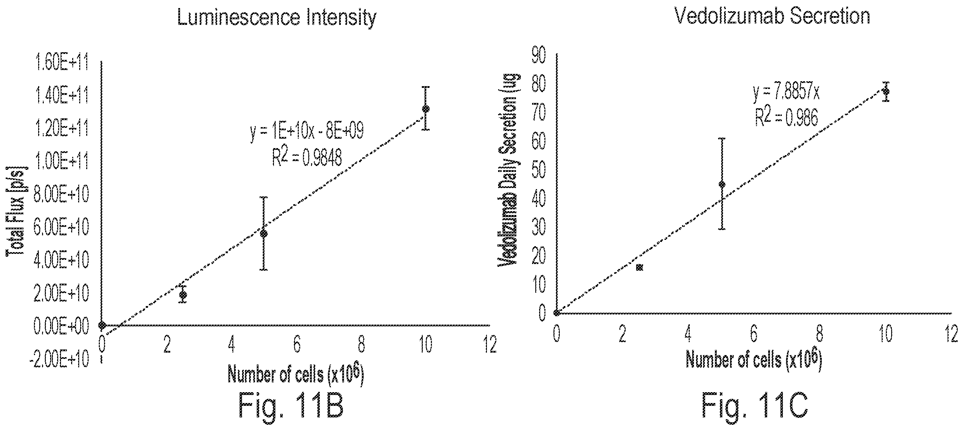

**Figs. 11A-11C** show the results of an *in vitro* study in which ARPE-19 cells stably expressing vedolizumab/luciferase were loaded into cell chambers, as described in Fig. 1E, at three different densities, 2.5 million, 5.0 million, and 10 million cells/chamber. A day after cell loading, the chambers were incubated in fresh MegavirSFM for two hours followed by optical Imaging with IVIS Spectrum Imaging platform (PerkinElmer) by placing the chambers into culture medium containing 150 µg/ml D-luciferin. **Fig. 11A** shows luminescent images of nPET-PBT/PET/nPU cell chamber devices (Rows 1-3) or a Theracyte cell chamber device (Theracyte Cat#:PD20.0s; World Precision Instrument Cat#:505396) (Row 4) with the indicated number of cells. The data were analyzed with Living Image Software (PerkinElmer) by delimiting a constant region of interest (ROI) around the device and quantifying total radiance in photon/sec to assess the linearity of luminescence intensity vs cell numbers. **Fig. 11B** graphically depicts the luminescent intensity of the cells in the chambers shown in Fig. 11A as a function of the number of cells in the chambers. **Fig. 11C** graphically depicts the vedolizumab concentration in the medium as a function of the number of cells, as determined by a vedolizumab ELISA assay.

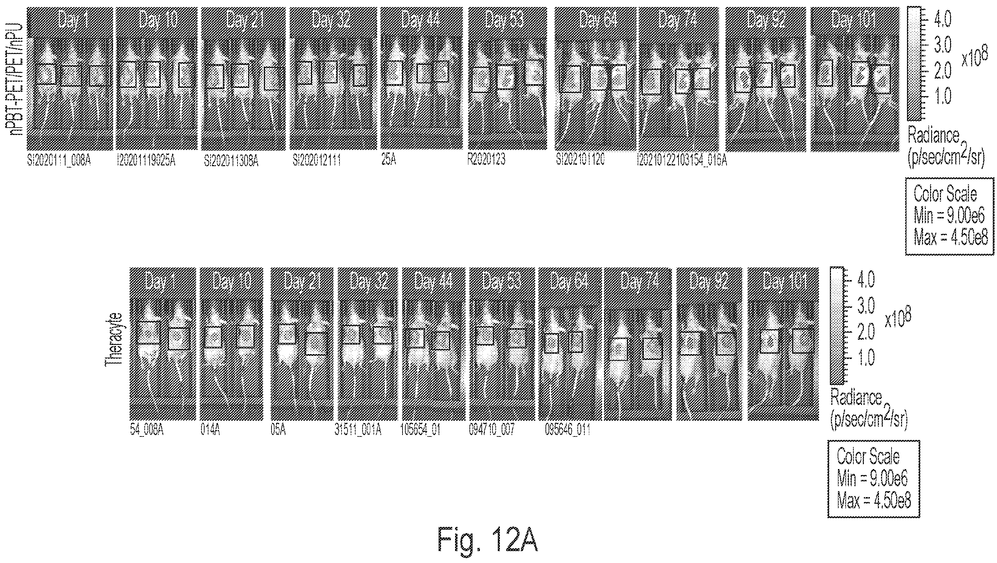

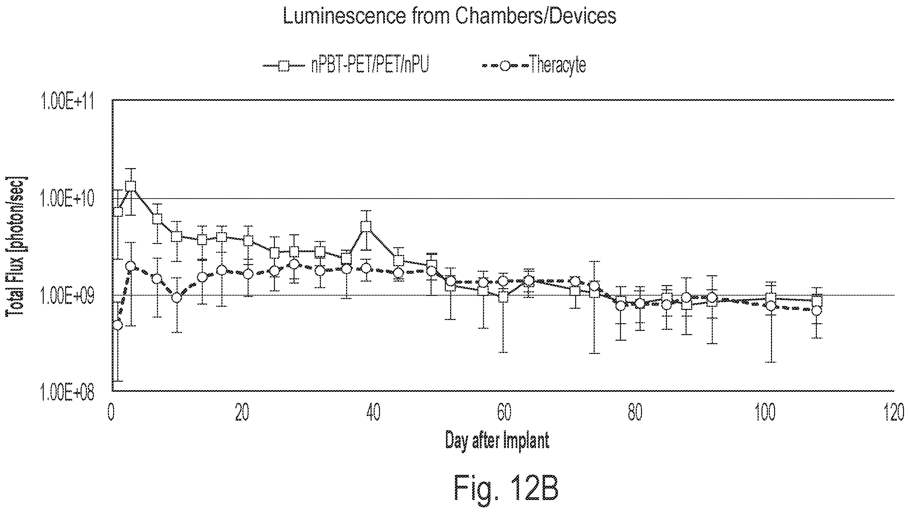

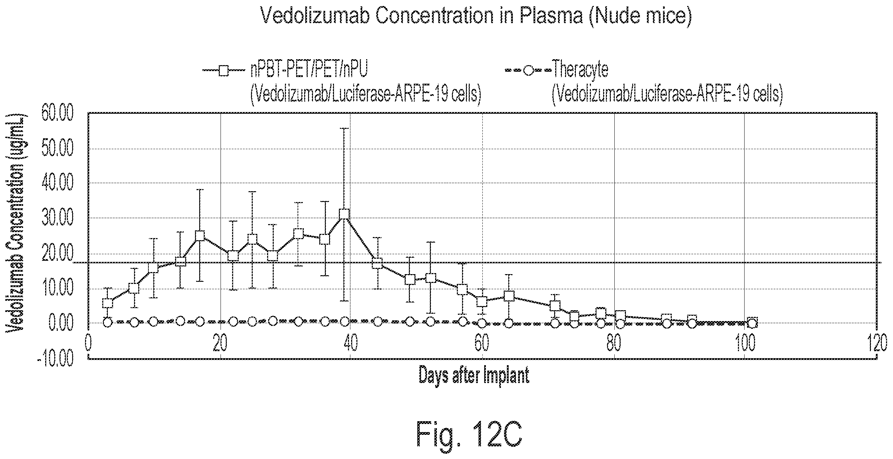

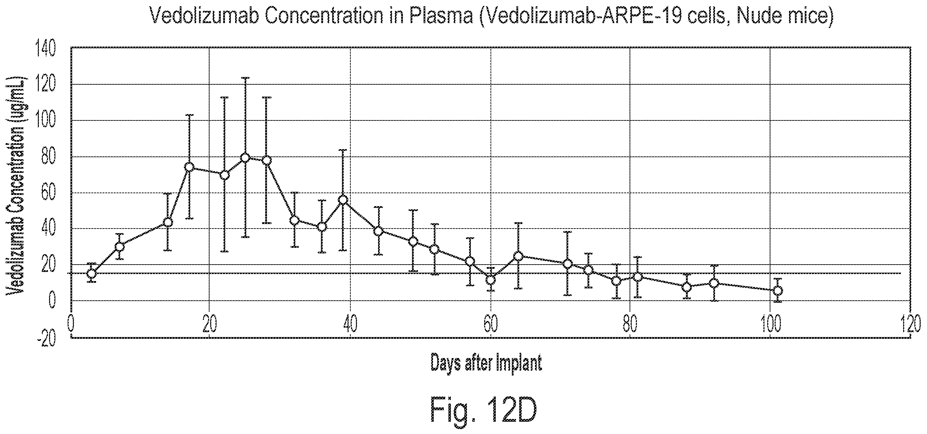

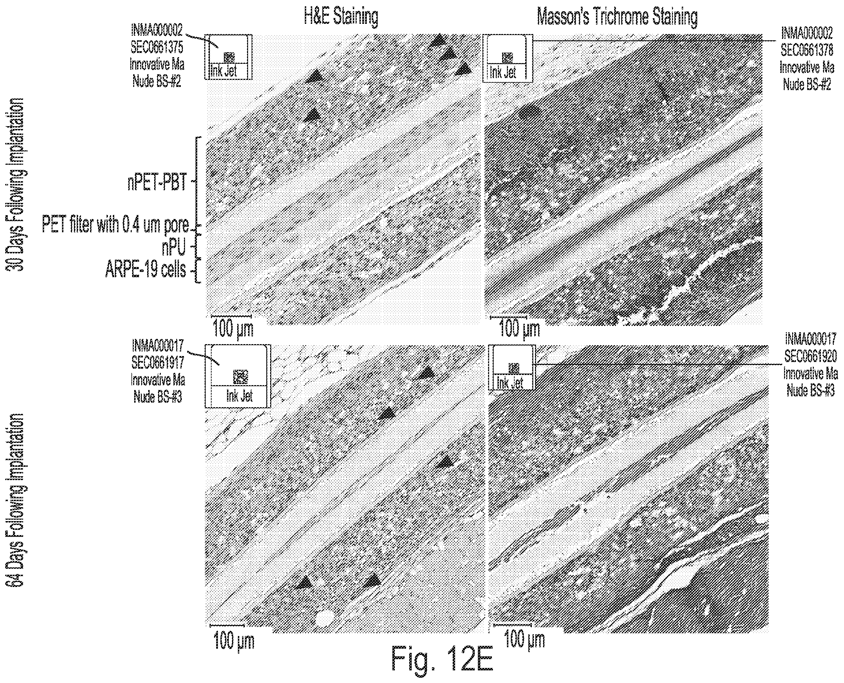

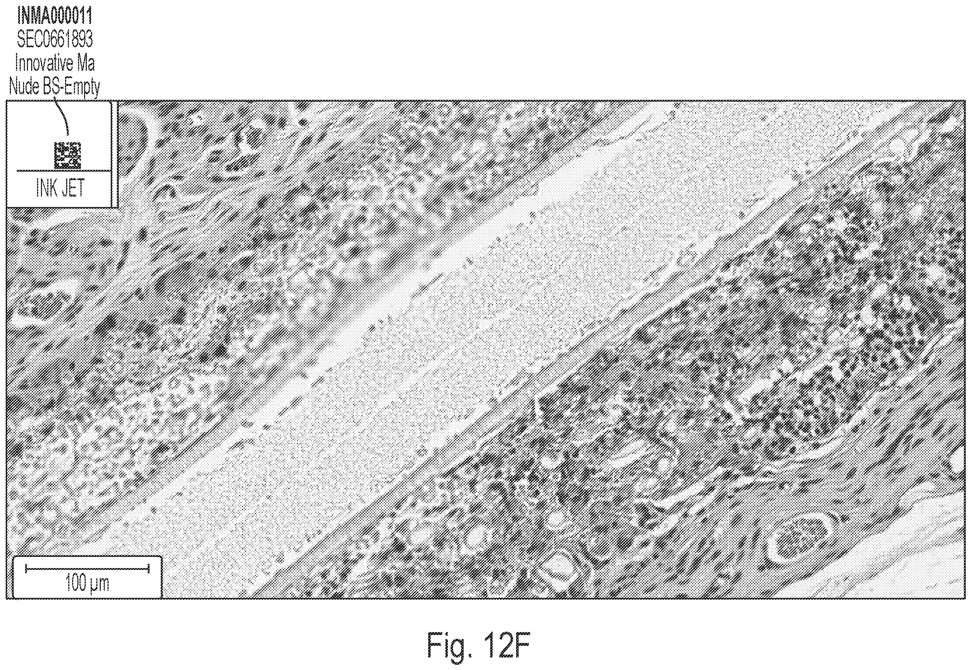

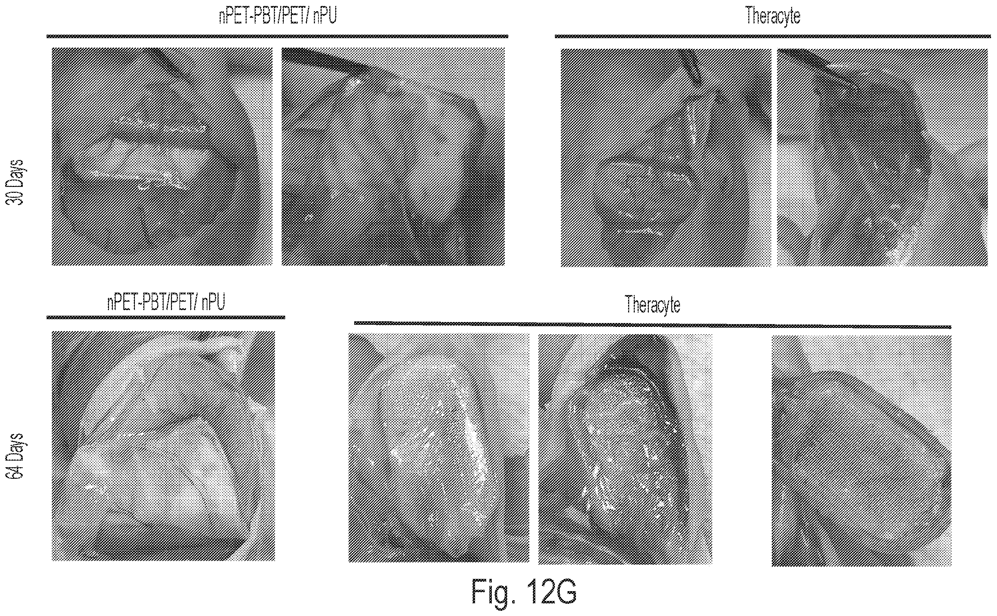

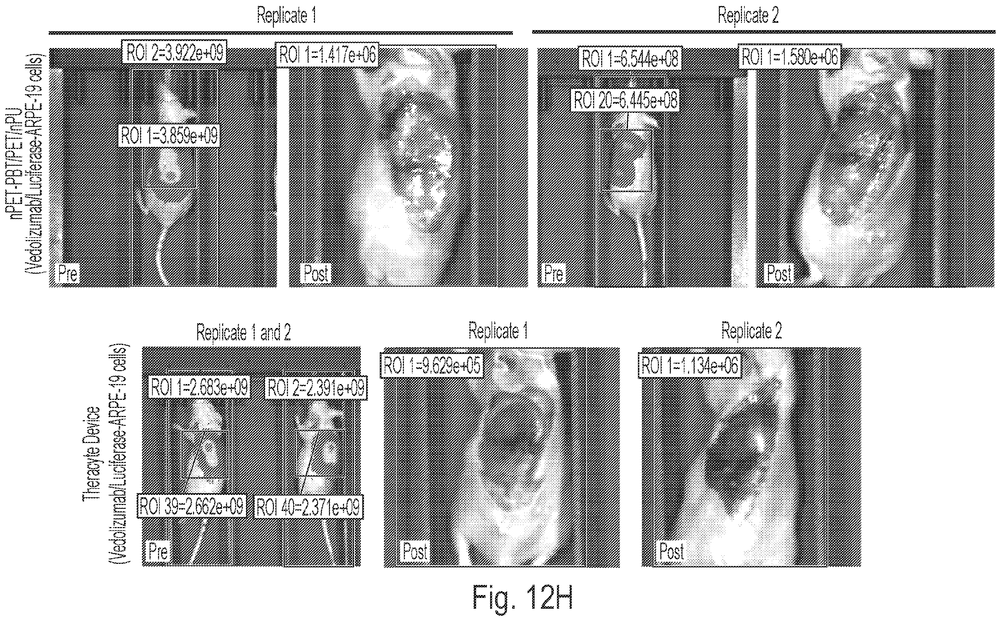

**Figs. 12A-12H** depict the results of a study assessing *in vivo* expression of luciferase and/or vedolizumab by ARPE-19 cells in a cell chamber as described in Fig. 1E, comprising a nPET-PBT/PU scaffold with a central porous PET membrane (0.4µm pore), in which the cell chamber was implanted in immunocompromised mice (nude mouse; no T cells). **Fig. 12A** shows luminescent images of nude mice implanted with nPET-PBT/PET/nPU cell chambers (top row) or Theracyte devices (bottom panel). Data were analyzed with Living Image Software (PerkinElmer) by delimiting a constant region of interest (ROI) around each device and quantifying total radiance in photon/sec. **Fig. 12B** graphically depicts the average luminescence intensity over time in nude mice implanted with cell chambers as described in Fig. 1E, comprising vedolizumab/luciferase-ARPE-19 cells, as compared to the luminescence intensity in nude mice implanted with a Theracyte device comprising vedolizumab/luciferase-ARPE-19 cells (Day1-Day30: N=7; Day31-Day60: N=5; Day64: N=4 for nPET-PBT/PET/nPU, N=5 for Theracyte; Day65-Day108: N=3 for nPET-PBT/PET/nPU, N=2 for Theracyte). **Fig. 12C** graphically depicts the average vedolizumab plasma concentration (as detected by ELISA) over time in nude mice implanted with cell chambers as described in Fig. 1E, comprising vedolizumab/luciferase-ARPE-19 cells, as compared to the vedolizumab plasma concentration over time in nude mice implanted with a Theracyte cell chamber comprising vedolizumab/luciferase-ARPE-19 cells (Day1-Day30: N=7; Day31-Day60: N=5; Day64: N=4 for nPET-PBT/PET/nPU, N=5 for Theracyte; Day65-Day108: N=3 for nPET-PBT/PET/nPU, N=2 for Theracyte). The horizontal line indicates the target therapeutic plasma concentration. **Fig. 12D** graphically depicts the average vedolizumab plasma concentration (as detected by ELISA) over time in nude mice implanted with cell chambers as described in Fig. 1E, comprising vedolizumab-ARPE-19 cells. The horizontal line indicates the target therapeutic plasma concentration. **Fig. 12E** contains H&E stained images (left panels) and Masson's Trichrome stained images (right panels) of the area surrounding a cell chamber as shown in Fig. 1E, comprising a non-nanofibrous PET sheet (membrane) having 0.4 µm pores, coated on one side with electrospun nPET-PBT, and on the other side with electrospun nPU, and seeded with ARPE-19 cells, 30 days (top panels) or 64 days (bottom panel) following implantation in mice. **Fig. 12F** contains an H&E stained image of the area surrounding a nPET-PBT/PET/nPU cell chamber (as shown in Fig. 1E) that has not been seeded with cells, 60 days following implantation in mice. **Fig. 12G** show images of nPBT-PET/PET/nPU cell chambers (as shown in Fig. 1E) or Theracyte cell chambers, each seeded with ARPE-19 cells expressing vedolizumab and luciferase, after being removed from nude mice 30 days (top panels) or 64 days (bottom panels) post-transplant in nude mice. **Fig. 12H** shows luminescent imaging of nude mice before and after removal of PBT-PET/PET/nPU cell chambers (as shown in Fig. 1E) comprising vedolizumab/luciferase-ARPE-19 cells as compared to luminescent imaging of nude mice before and after removal of a Theracyte cell chamber comprising vedolizumab/luciferase-ARPE-19 cells.

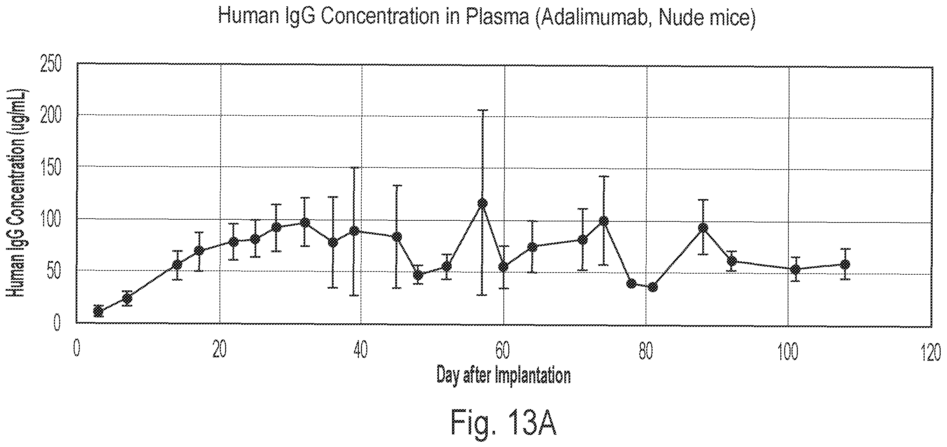

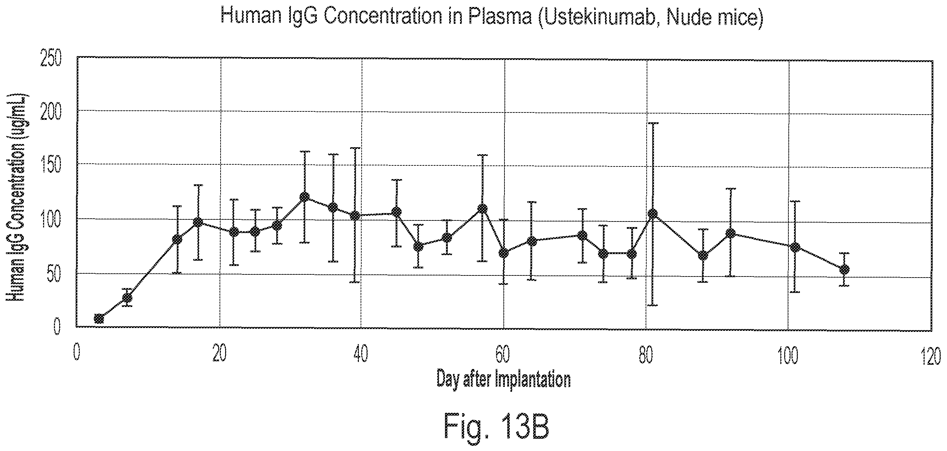

**Figs. 13A** **and** **13B** graphically depicts the average plasma concentration over time of adalimumab (**Fig. 13A**; Day1-Day60: N=5; Day61-Day81: N=4; Day82-92: N=3; Day93-Day108: N=2) or ustekinumab (**Fig. 13B**; N=5) in immune compromised mice implanted with a cell chamber as described in Fig. 1E, comprising adalimumab / ARPE-19 cells (**Fig. 13A**) or ustekinumab/ARPE-19 cells (**Fig. 13B**).

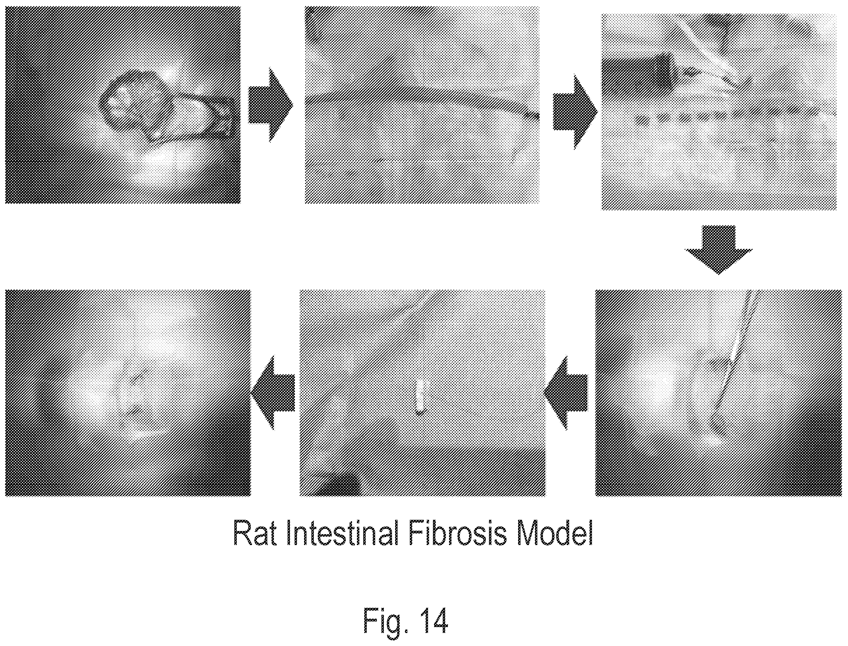

**Fig. 14** depicts the workflow for an experiment testing the ability of tissue explants derived from intestine to grow in a single-layer cell chamber device comprising nanofibrous electrospun polymer.

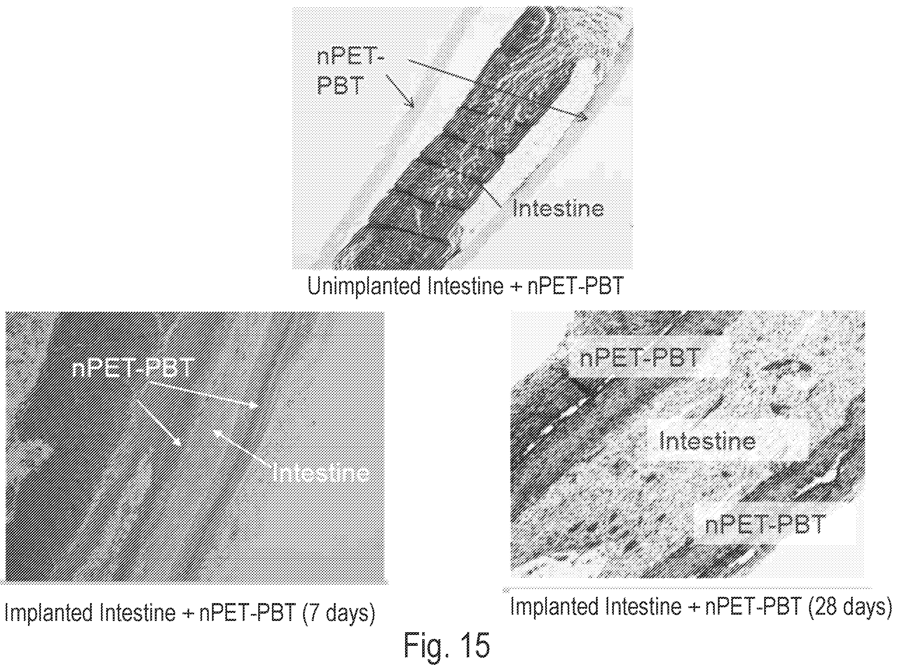

**Fig. 15** presents representative images of a cell chamber device loaded with an intestinal tissue explant prior to implantation (upper panel), 7 days post-implantation (lower left panel), and 28 days after implantation (lower right day panel) in a rat model.

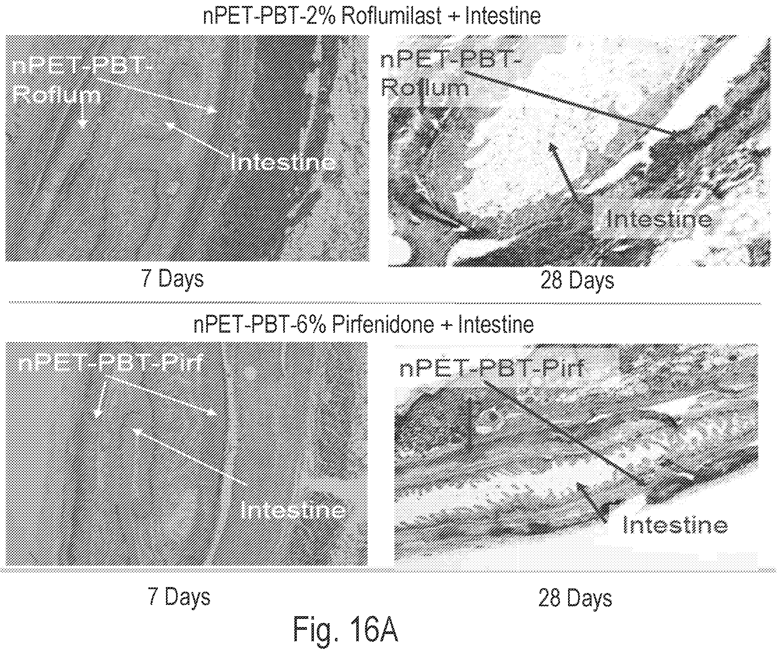

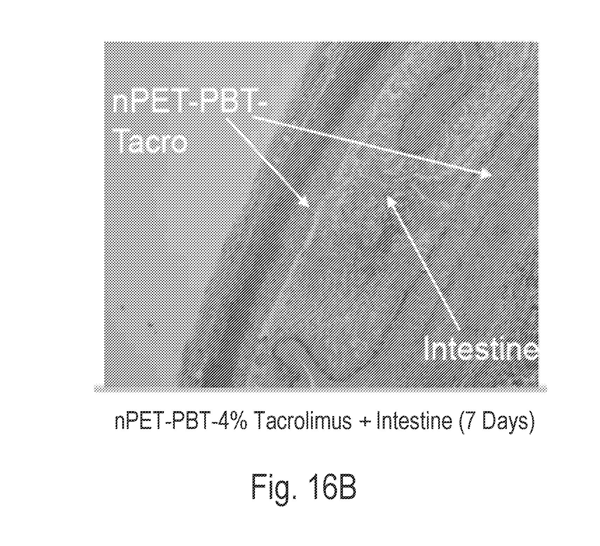

**Figs. 16A-16B** present representative images of a cell chamber device comprising nanofibrous nPET-PBT loaded with Roflumilast (**Fig. 16A****,** top panels) Pirfenidone (**Fig. 16A****,** bottom panels), or Tacrolimus (**Fig. 16B****).** The devices were loaded with an intestinal tissue explant prior to implantation in a rat model, and were imaged 7 days and 28 days post-implantation.

### DETAILED DESCRIPTION OF THE INVENTION

### I. Definitions

As used herein, the term "nanofiber" refers to a fiber with a diameter less than 3.0 micrometers. In exemplary embodiments, a nanofiber has a diameter between 10 nanometers and 3.0 micrometers. The diameter of a nanofiber depends on the type of polymer used and the method of production. Methods of making nanofibers include electrospinning, drawing, self-assembly, template synthesis, and thermal-induced phase separation.

As used herein, the term "electrospinning" refers to a process that uses high voltages in combination with distance from a material source to a base in order to produce a cross-linked mesh. For example, an electric field can be used to draw a solution comprising a polymer from the tip of a capillary to a collector. A high voltage DC current can be applied to the solution, which can cause a jet of the solution to be drawn towards the grounded collector screen. Once ejected out of the capillary orifice, the charged solution jet can be evaporated to form fibers, which can be collected on a collector (e.g., a rotating collector). The size and morphology of the fibers thus obtained depends on a variety of factors such as viscosity of the solution, molecular weight, nature of the polymer and other parameters regarding the electrospinning apparatus. The electrospinning process to form polymer nanofibers has been demonstrated using a variety of polymers (see, e.g., Huang, et al. Composites Science and Technology (2003). 63:2223-2253, which is hereby incorporated by reference in its entirety).

As used herein, an "electrospun polymer" refers to a polymer fiber, e.g., polymer nanofiber, manufactured by electrospinning. Electrospun polymers can include, but are not limited to, polyethylene terephthalate (PET), polybutylene terephthalate (PBT), or polyurethane (PU). The foregoing polymers can be electrospun to produce polymer nanofibers, as described herein.

As used herein, the term "biomolecule" refers to any organic molecule capable of being produced by a living organism, including polypeptides, polysaccharides, and polynucleotides as well as organic molecules such as lipids (e.g., phospholipids, glycolipids and sterols), chemical messengers (e.g., hormones and neurotransmitters), vitamins, sugars (e.g., carbohydrate, disaccharide, oligosaccharides, polysaccharides), amino acids, peptides, oligopeptides, polypeptides, proteins, nucleotides, deoxyribonucleic acid (DNA), or ribonucleic acid (RNA). In some embodiments, biomolecules are produced recombinantly. In some embodiments the biomolecules may be secreted from cells by other means such as extravesicular vesicles, exosomes, or secreted organelles such as mitochondria. For example, secreted biomolecules may include those associated, packaged, and secreted as exosomes, lipid polymers, or viral particles. In some embodiments, the biomolecule can be a "therapeutic biomolecule," which is capable of treating, preventing, and/or ameliorating the symptoms of a disease, disorder, infection or illness in a subject, e.g., a human patient, in need thereof, when provided to the subject at an effective amount or dose. In certain embodiments, cells growing in a cell chamber device described herein can secrete an effective amount of a therapeutic biomolecule in a subject to whom the device has been administered. In some embodiments, the therapeutic biomolecule is an antibody. In other embodiments, the therapeutic biomolecule is a hormone. In other embodiments, the therapeutic biomolecule is an enzyme. In other embodiments, the therapeutic biomolecule is a peptide or protein.

The term "antibody" as used herein, refers to an immunoglobulin molecule comprised of four polypeptide chains, two heavy (H) chains and two light (L) chains inter-connected by disulfide bonds. Each heavy chain is comprised of a heavy chain variable region (abbreviated herein as HCVR or VH) and a heavy chain constant region (CH). The heavy chain constant region is comprised of three domains, CH1, CH2 and CH3. Each light chain is comprised of a light chain variable region (abbreviated herein as LCVR or VL) and a light chain constant region. The light chain constant region is comprised of one domain, CL. The VH and VL regions can be further subdivided into regions of hypervariability, termed complementarity determining regions (CDRs), interspersed with regions that are more conserved, termed framework regions (FR). Each VH and VL is composed of three CDRs and four FRs, arranged from amino-terminus to carboxy-terminus in the following order: FR1, CDR1, FR2, CDR2, FR3, CDR3, FR4. In some embodiments, the antibody has a fragment crystallizable (Fc) region. Antibodies suitable for use in embodiments described herein can include antibodies having an isotype selected from IgG (*e.g.*, IgG1, IgG2, IgG3, IgG4), IgM, IgA1, IgA2, IgD, or IgE. In exemplary embodiments the antibody is an IgG1 antibody, an IgG2 antibody, or an IgG3 antibody. In various embodiments, the antibody can comprise a kappa light chain or a lambda light chain. In certain embodiments, the antibody can have an IgG1 isotype and a kappa light chain.

The cell surface molecule, "α4β7 integrin," or "α4β7" (used interchangeably throughout) is a heterodimer of an α4 chain (CD49D, ITGA4) and a β7 chain (ITGB7). Human α4-integrin and β7-integrin genes GenBank (National Center for Biotechnology Information, Bethesda, Md.) RefSeq Accession numbers NM_000885 and NM_000889, respectively) are expressed by B and T lymphocytes, particularly memory CD4+ lymphocytes. Typical of many integrins, α4β7 can exist in either a resting or activated state. Ligands for α4β7 include vascular cell adhesion molecule (VCAM), fibronectin and mucosal addressin (MAdCAM (*e.g*., MAdCAM-1)). An antibody that binds to α4β7 integrin is referred to herein as an "anti- α4β7 antibody".

As used herein, an antibody, or antigen-binding fragment thereof, that has "binding specificity for the α4β7 complex" binds to α4β7, but not to α4β1 or α_{E}B7. Vedolizumab is an example of an antibody that has binding specificity for the α4β7 complex.

A "CDR" or "complementarity determining region" is a region of hypervariability interspersed within regions that are more conserved, termed "framework regions" (FR).

As used herein, the term "antigen binding fragment" or "antigen binding portion" of an antibody refers to Fab, Fab', F(ab')₂, and Fv fragments, single chain antibodies, functional heavy chain antibodies (nanobodies), as well as any portion of an antibody having specificity toward at least one desired epitope, that competes with the intact antibody for specific binding (*e.g*., an isolated portion of a complementarity determining region having sufficient framework sequences so as to bind specifically to an epitope). Antigen binding fragments can be produced by recombinant techniques, or by enzymatic or chemical cleavage of an antibody. Exemplary antibody fragments include, but are not limited to, a Fab, a F(ab')₂, an scFv, a tandem scFv, a diabody, a minibody, and a single domain antibody.

"Humanized" forms of non-human (e.g., rodent) antibodies are chimeric antibodies that contain minimal sequence derived from the non-human antibody. For the most part, humanized antibodies are human immunoglobulins (recipient antibody) in which residues from a hypervariable region of the recipient are replaced by residues from a hypervariable region of a non-human species (donor antibody) such as mouse, rat, rabbit or nonhuman primate having the desired specificity, affinity, and capacity. In some instances, framework region (FR) residues of the human antibody are replaced by corresponding non-human residues. Furthermore, humanized antibodies may comprise residues that are not found in the recipient antibody or in the donor antibody. These modifications are made to further refine antibody performance. In general, the humanized antibody will comprise substantially all of at least one, and typically two, variable domains, in which all or substantially all of the hypervariable CDR loops correspond to those of a non-human antibody and all or substantially all of the FRs are those of a human antibody sequence. The humanized antibody optionally also will comprise at least a portion of an antibody constant region (Fc), typically that of a human antibody. For further details, see Jones et al., Nature 321:522-525 (1986); Riechmann et al., Nature 332:323-329 (1988); and Presta, Curr. Op. Struct. Biol. 2:593-596 (1992).

As used herein, the terms "pore" or "pores" refer to an opening in a material, e.g., a scaffold material, that allows the passage of biomolecules from one side of the material to the other. Pores can be of any shape or dimension suitable for this purpose. In cases where the material is a nanofibrous polymer, e.g., an electrospun nanofibrous polymer, the spaces between fibers can function as pores. In one embodiment, pores can be of a size that permits the passage of biomolecules, but blocks the passage of cells.

As used herein, the term "recombinant protein" refers to a protein produced as the result of the transcription and translation of a gene(s) carried on a recombinant expression vector(s) that has been introduced into a host cell, *e.g.* a mammalian host cell. In certain embodiments the recombinant protein is a recombinant antibody, or an antigen binding portion thereof.

The term "recombinant host cell" (used interchangeably herein with the term "host cell") includes a cell into which a recombinant expression vector has been introduced, e.g., a recombinant expression vector that encodes a secreted protein. It should be understood that such terms are intended to refer not only to the particular subject cell but to the progeny of such a cell. Because certain modifications may occur in succeeding generations due to either mutation or environmental influences, such progeny may not, in fact, be identical to the parent cell, but are still included within the scope of the term "host cell" as used herein. Further, it should be understood that unless specified otherwise, where the term "cell" is used, *e.g.,* host cell or mammalian cell or mammalian host cell, it is intended to include a population of cells.

The term "about" denotes that the thereafter following value is not an exact value but is the center point of a range that is +/-5% of the value of the value. If the value is a relative value given in percentages the term "about" also denotes that the thereafter following value is not an exact value but is the center point of a range that is +/-5% of the value, whereby the upper limit of the range cannot exceed a value of 100%.

### II. Implantable Cell Chamber Device

Provided herein is a cell chamber device capable of retaining cells, e.g., cells that secrete a therapeutic biomolecule. The cell chamber device of the present disclosure comprises a scaffold comprising nanofibrous polymers, that defines the envelope of a cell chamber. The scaffold permits the passage of biomolecules such as proteins into and out of the cell chamber, but does not permit the passage of cells. Live cells capable of producing a biomolecule can be loaded within the cell chamber, where the cells can propagate and adhere to the inner surface of the scaffold. Biomolecules secreted by cells within the cell chamber can diffuse out of the cell chamber through pores in the scaffold. The scaffold prevents the cells contained therein from migrating out of the cell chamber, and prevents host cells from infiltrating into the cell chamber. Upon implantation of the cell chamber into a host subject, the cells within the cell chamber device can provide steady delivery of a variety of biomolecule therapeutics in a stable form, e.g., for treatment of a host subject in need thereof. Continuous production of a desired biomolecule by cells in the chamber can eliminate the need for repeated dosing of an isolated pharmaceutical composition comprising the biomolecule by traditional means. The cell chamber device provided herein preferably comprises materials that induce little to no fibrotic reaction in mammalian hosts, and induce little to no host immune reaction.

The cell chamber devices provided herein comprise a scaffold comprising nanofibrous polymers that surround a cell chamber. In some embodiments, the scaffold of the cell chamber device has a multi-layer construction. For example, the scaffold can comprise a bilayer of nanofibrous polymers, with an outer layer and an inner layer, e.g., an outer layer of nanofibrous polymer in contact with the external environment surrounding the device, and an inner layer of nanofibrous polymer in contact with a cell chamber. Alternatively, the scaffold may have a trilayer or multilayer construction, by including one or more additional layers between the outer and inner layers. For example, the scaffold can optionally comprise a membrane, e.g., a porous membrane, semi-porous, or a non-porous membrane, positioned between the outer layer and the inner layer. The membrane is alternatively referred to herein as a filter. In some embodiments the one or more additional layers of the scaffold comprise nanofibrous polymers. In some embodiments, the multi-layer scaffold comprises two, three, four, five, six, seven, eight, nine, or ten, or more, layers. The outer layer and inner layer (and any optional additional layers, e.g., a central membrane layer) may comprise the same nanofibrous polymers, or alternatively, may comprise different nanofibrous polymers having different properties. For example, the outer layer of the scaffold can comprise a material that is compatible for implantation within a host tissue (e.g., generates little to no fibrotic reaction or immune reaction in the implant recipient, permits vascularization of the outer scaffold), while the inner layer that comes into direct contact with cells in the cell chamber can comprise a material that can serve as a cell scaffold and optionally, a barrier. In certain embodiments, the inner layer can have smaller pores than the outer layer.

The inner and/or outer scaffold layer of the cell chamber device can be formed from a variety of nanofibrous polymers, such as polyethylene terephthalate (PET, also known as Dacron), polybutylene terephthalate (PBT), or polyurethane (PU). Any biocompatible polymers suitable for use in electrospinning can be used in the cell chamber device described herein. In some embodiments, the biocompatible polymer is a nanofibrous polyester. Other polymers suitable for use in the scaffold layers of the cell chamber device described herein include, but are not limited to poly-lactic acid (PLA), poly-glycolic acid (PGA), poly-lactic-co-glycolic acid (PLGA), polycaprolactone (PCL), polypropylene (PP), poly-tetrafluoroethylene (PTFE), polytrimethylene terephthalate, polyvinyl alcohol (PVA), polyethylene oxide (PEO), polytrimethylene terephthalate (PTT), polyethylene acetate (PEVA), poly-D-lactide (PLDA), polylactic acid (PLLA), or polyethylene glycol (PEG). Other suitable polymers include, but are not limited to, collagen, gelatin, alginate, fibrinogen, silk, elastin, cellulose, chitin, chitosan. The inner and/or outer layers may each comprise a single type of nanofibrous polymer. In such instances, the inner and/or outer layer may each comprise different types of nanofibrous polymer or may comprise the same type of nanofibrous polymer. Alternatively, the inner and/or outer layers may each comprise two or more types of nanofibrous polymers.

For example, in some embodiments, the outer layer can comprise nanofibrous polyethylene terephthalate (nPET) or nanofibrous polybutylene terephthalate (nPBT). In some embodiments, the outer layer can comprise both nanofibrous polyethylene terephthalate and polybutylene terephthalate (nPET-PBT). In some embodiments, the outer layer can comprise nanofibrous polyurethane (nPU).

In some embodiments, the inner layer can comprise nanofibrous polyethylene terephthalate (nPET) or nanofibrous polybutylene terephthalate (nPBT). In some embodiments, the inner layer can comprise both nanofibrous polyethylene terephthalate and polybutylene terephthalate (nPET-PBT). In some embodiments, the inner layer can comprise nanofibrous polyurethane (nPU). In some embodiments, the outer layer can comprise nPET-PBT, and the inner layer can comprise nPU. In other embodiments, the outer layer can comprise nPU. In some embodiments, the inner layer can comprise nPET, nPBT, or nPET-PBT.

The scaffold can additionally or alternatively include other synthetic or biological materials. Examples of synthetic materials include polytetrafluoroethylene (ePTFE) or poly(glycolic acid) (PGA). Biological materials include biological membranes, bovine tissue, collagen scaffolds (e.g., a gel, thread, foam, sheet, matt, or tube), gelatin, alginate, cellulose (e.g., methylcellulose), elastin, glycosaminoglycans, peptidoglycans, chitin, or fibrin (e.g., a gel). The biological material can be optionally treated with a crosslinker, e.g., an aldehyde (e.g., glutaraldehyde or formaldehyde), a carbodiimide (e.g., 1-ethyl-3-(dimethyaminopropyl)carbodiimide), an acrylamide (e.g., N,N'-methylenebisacrylamide), or a diimidmate (e.g., dimethylsuberimidate). In some embodiments, the biological material includes glutaraldehyde-cross linked biological membranes.

There are several different ways of synthesizing tissue scaffolds which may be employed in the fabrication of the cell chamber devices described herein. These include, for example, electrospinning, nanofiber self-assembly, textile technologies, solvent casting and particulate leaching. One exemplary method of creating the scaffold layers is electrospinning, a process that uses an electric field to draw a solution comprising a polymer from the tip of a capillary to a collector. A high voltage DC current is applied to the solution which causes a jet of the solution to be drawn towards the grounded collector screen. Once ejected out of the capillary orifice, the charged solution jet gets evaporated to form fibers and the fibers get collected on the collector. Electrospinning can be used to produce a cross-linked mesh with polymer fibers of nanometer to micrometer size in diameter. The size and morphology of the fibers obtained by electrospinning depends on a variety of factors such as viscosity of the solution, molecular weight, nature of the polymer and other parameters regarding the electrospinning apparatus. The electrospinning process to form polymer nanofibers has been demonstrated using a variety of polymers (see, e.g., Huang, et al. Composites Science and Technology (2003). 63:2223-2253, which is hereby incorporated by reference in its entirety).

In certain embodiments, the inner and/or outer layer of the scaffold is produced by electrospinning, thereby forming a scaffold comprising electrospun polymers. Electrospun polymers are biocompatible, having a web-like, random structure that resembles the body's natural extracellular matrix scaffold, thereby promoting tissue incorporation and preventing rejection. Further, electrospun polymers can enhance cell growth, promote cell differentiation, and attract cellular attachment. In addition, the size of electrospun fibers can be ten to 14 times smaller than the fiber size of other implantable materials (e.g., such as a woven polyester membrane, see **Figs. 1A-1C**).

In exemplary embodiments, the scaffold (e.g., the inner and/or outer layer) can comprise electrospun polymers selected from electrospun polyethylene terephthalate, electrospun polybutylene terephthalate, or electrospun polyurethane. In some embodiments, the outer layer comprises electrospun polyethylene terephthalate. In some embodiments, the outer layer comprises electrospun polybutylene terephthalate. In some embodiments, the outer layer comprises electrospun polyethylene terephthalate and electrospun polybutylene terephthalate (see Fig. 1C, bottom image). In yet further embodiments, the inner layer comprises electrospun polyurethane (see Fig. 1C, top image). In exemplary embodiments, the outer layer comprises electrospun polyethylene terephthalate and electrospun polybutylene terephthalate, and the inner layer comprises electrospun polyurethane.

Electrospun polymers may be generated by electrospinning procedures known in the art or further described herein. In one embodiment, the electrospun polymer is prepared by first preparing a polymer solution, such as a polymer solution comprising the polymer and hexafluoroisopropanol (HFIP). For example, to form a solution of polyethylene terephthalate (PET) and polybutylene terephthalate (PBT), PET and PBT chips or pellets can be placed into a solution of HFIP, and incubated (e.g., on a rotator) until the chips have dissolved (e.g., about 5-7 days of incubation). Similarly, to form a solution of polyurethane (PU), PU chips or pellets can be placed into a solution of HFIP, and incubated (e.g., on a rotator) until the chips have dissolved (e.g., about 5-7 days of incubation).

Next, the polymer solution can be loaded onto an electrospinning unit to produce electrospun polymers. For example, the polyester solution can be loaded into a syringe, which is subsequently connected to an electrospinning unit. The electrospinning unit can then be operated in accordance with standard operating procedures to generate the electrospun polymers (e.g., PET, PBT, or PU). In some embodiments, the electrospinning unit is set to have an electrospinning distance of about 10-25 cm (e.g., about 10 cm, about 11 cm, about 12 cm, about 13 cm, about 14 cm, about 15 cm, about 16 cm, about 17 cm, about 18 cm, about 19 cm, or about 20 cm, about 21 cm, about 22 cm, about 23 cm, about 24 cm, or about 25 cm), an electrospinning voltage of about 15-30 kV (e.g., about 15 kV, about 18 kV, about 20 kV, about 21 kV, about 22 kV, or about 23 kV, about 25 kV, about 28 kV, or about 30 kV), or rotation speed of about 15-150 RPM (e.g., about 15-25 RPM, about 25-50 RPM, about 50-75 RPM, about 75-100 RPM, about 100-125 RPM, or about 125-150RPM). In some embodiments, a second electrospun polymer (e.g., a PU layer) can be layered onto a first electrospun polymer (e.g., a nPET-PBT layer) to generate a material composed of two or more different layers of electrospun polymers. In certain embodiments, the electrospun material comprises nPET-PBT layered onto nPU. In other embodiments, the electrospun material comprises nPU layered onto nPET-PBT. Such bilayered scaffolds can be used to fabricate the cell chamber devices described herein.

One or more additional polymers can optionally be layered on or between the foregoing layers, to form a multi-layered material that can be used to fabricate some embodiments of the cell chamber devices described herein. In some embodiments, the multi-layer scaffold can comprise 1, 2, 3, 4, 5, 6, 7, 8, 9, 10 or more layers of nanofibrous polymers, e.g., nanofibrous electrospun polymers. The layers can each comprise a distinct polymer, or multiple layers can comprise the same polymer.

A cell chamber can be fabricated from the multilayer (e.g., bilayer) electrospun polymer material. The material can be measured and cut to any desired dimensions. Once prepared to the desired shape and size, the edges of the electrospun polymer material can be partially or fully sealed to form a chamber. The polymer material can be sealed at the edges of the chamber in accordance with methods known in the art. In one embodiment, the edges of the chamber are sealed using ultrasonic welding. In some embodiments, at least one portion of the chamber is left unsealed, e.g., to permit subsequent loading of cells. In some embodiments, the chamber is fully sealed, but can later be opened, e.g., by cutting or by piercing with a needle, to create a cell loading port that can be subsequently sealed. In some embodiments, the scaffold material is folded prior to sealing of the edges. In some embodiments, a cell loading port (e.g., a port having a luer lock mechanism that can be attached to a cell injector device) will be incorporated into the chamber membrane design that would allow for cell loading. Post loading, the port can optionally be removed and chamber sealed by ultrasound or heat welding, or by sealing the chamber opening with adhesives.

In some instances, the nanofibrous polymers comprising the inner and/or outer layer of the scaffold of the cell chamber device herein can be further modified with one or more charged surface modifications. For example, in some embodiments the nanofibrous polymers of the inner and/or outer layers of the scaffold are contacted with liquid ethylenediamine. For example, when reacted with PET, treatment with ethylenediamine results in a positively charged surface. In other embodiments the nanofibrous polymers of the inner and/or outer layers of the scaffold are contacted with liquid sodium hydroxide. When reacted with PET, for example, treatment with sodium hydroxide results in a negatively charged surface. Altering the surface charge of the material can be used to modulate the attraction or adherence of cell types having an intrinsic charge on their cell surface proteins. Charged surface material can also attract specific proteins, such as proteins in the blood and surrounding milieu, that promote cell attachment. Ionic surfaces can also improve surface wettability and contact with cells. The charged surface modification may comprise cationic functional groups, anionic functional groups, or both cationic and anionic functional groups. For example, the charged surface modification can include carboxylic acid and/or amine functional groups. In some embodiments, a charged surface modification can be generated by treating a polymer of the scaffold with alkaline hydrolysis (e.g., by sodium hydroxide treatment). In some embodiments, a charged surface modification can be generated by treating a polymer of the scaffold with a diamine, such as ethylene diamine (EDA), 2-methylpentamethylene diamine, 1,2-diaminocyclohexane, or 1,6-hexanediamine. Examples of charged surface modifications and methods of generating charged surface modifications are further described, for example, in U.S. Patent Nos. 6,743,253B2 and 7,037,527B2, which are hereby incorporated by reference in their entirety.

In some instances, the inner and/or outer scaffold layer of the cell chamber device further comprises a substance that decreases transplant injury related inflammation, such as a steroid (e.g., dexamethasone, triamcinilone) or an immune suppressant (e.g., Tacrolimus, Sirolimus, everolimus, and/or paclitaxel). In some embodiments, the inner and/or outer scaffold layer of the cell chamber device comprises the cytokine CCL22, which can promote tolerance to the implant by recruitment of Treg cells to the vicinity of the implant. The substance can be incorporated within the polymers during scaffold production, or coated on the scaffold after production. For example, the substance can be dissolved into the polymer solution prior to electrospinning. In such embodiments, the substance will be incorporated into the nanofibers of the scaffold during production. In other embodiments, a fully or partially formed scaffold can be soaked in a solution comprising the substance, which can be absorbed by the scaffold material. Introduction of a net positive charge or a net negative charge to the scaffold fibers, as described herein, can facilitate adherence of a substance loaded into the scaffold in this manner. Other substances, e.g., antibiotics, growth factors, etc., can be incorporated into the scaffold material in the manner described above.

The nanofibrous polymer scaffolds described herein can comprise pores arising from the random and web-like arrangement of the nanofibrous polymers. In addition, or alternatively, the pores of any desired shape and diameter can be introduced in the scaffold material. In some embodiments, the pores are nanopores (e.g., with a size of 1 µm or less).

The pores can be sized to permit the passage of biomolecules secreted by cells within the chamber out of the scaffold and into the host tissue or blood, while preventing passage of cells into or out of the chamber. For example, the nanopores may be sized to permit the passage of a biomolecule described herein, such as a polypeptide, a polysaccharide, and/or a polynucleotide, as well as organic molecules such as lipids (e.g., phospholipids, glycolipids and sterols), vitamins, sugars (e.g., carbohydrate, disaccharide, oligosaccharides, polysaccharides), and/or amino acids. The pores may be of a size that allows diffusion of polypeptides (e.g., antibodies, hormones, neurotransmitters, etc.), peptides, oligopeptides, nucleotides, deoxyribonucleic acid (DNA), ribonucleic acid (RNA) or miRNA across the scaffold. In certain embodiments, the pores are sized to permit the passage of an antibody, or antigen-binding portion thereof, across the scaffold.

In some embodiments, the nanopores are sized to permit the passage of biomolecules having a molecular weight of about 250 kDa or less (e.g., about 225 kDa or less, about 220 kDa or less, about 200 kDa or less, about 175 kDa or less, about 150 kDa or less, about 125 kDa or less, about 100 kDa or less, about 75 kDa or less, about 50 kDa or less, about 25 kDa or less, or about 10 kDa or less). In some embodiments, the nanopores are sized to permit the passage of a biomolecules having a molecular weight of 10 kDa to 50 kDa, 50 kDa to 100 kDa, 100 kDa to 150 kDa, 130 kDa to 165 kDa, 150 kDa to 200 kDa, or 200 kDa to 250 kDa.

The nanopores may also be sized to prevent the passage of cells and/or to prevent cells on one side of the scaffold from contacting cells on the other side of the scaffold. Accordingly, in some embodiments, the nanopores have a diameter of about 1 µm or less (e.g., about 0.9 µm or less, about 0.8 µm or less, about 0.7 µm or less, about 0.6 µm or less, about 0.5 µm or less, about 0.4 µm or less, about 0.3 µm or less, about 0.2 µm or less, about 0.1 µm or less, or about 0.05 µm or less). In certain embodiments, the nanopores have a diameter of 0.5 µm or less. In some embodiments, the nanopores have a diameter of 0.1 µm to 0.2 µm, 0.2 µm to 0.4 µm, 0.3 µm to 0.5 µm, 0.4 µm to 0.6 µm, 0.6 µm to 0.8 µm, or 0.8 µm to 1 µm. In some embodiments, the nanopores have a diameter of 0.1 µm to 3 µm. In other embodiments, the nanopores have a diameter of 0.2 µm to 2 µm. In some embodiments, the nanopores have a diameter of 1 nm to 10 µm (e.g., 1 nm to 10 nm, 5 nm to 25 nm, 10 nm to 50 nm, 25 nm to 75 nm, 50 nm to 100 nm, 75 nm to 125nm, 100 nm to 200 nm, 150 nm to 250 nm, 200 nm to 300 nm, 250 nm to 500 nm, 300 nm to 400 nm, 350 nm to 450 nm, 400 nm to 500 nm, 450 nm to 550 nm, 500 nm to 600 nm, 500 nm to 1µm, 550 nm to 650 nm, 600 nm to 700 nm, 650 nm to 750 nm, 700 nm to 800 nm, 750 nm to 850 nm, 800 nm to 900 nm, 900 nm to 1 µm, 1 µm to 2 µm, 1 µm to 5 µm, 1 µm to 10 µm, 2 µm to 4 µm, 2 µm to 6 µm, 2 µm to 10 µm, 4 µm to 6 µm, 4 µm to 8 µm, 4 µm to 10 µm, or 5 µm to 10 µm).

Antibodies generally have a length of about 10-15 nm. Many other peptide therapeutics are of equivalent size or smaller. For example, Fab fragments have a length of about 9 nm. Accordingly, a pore size of about 10 nm, 15 nm, 20 nm or more is generally sufficient to allow peptides and antibodies to pass through the device, while a diameter of about 10 µm or smaller (e.g., 9 µm or less, 8 µm or less, 7 µm or less, 6 µm or less, 5 µm or less, 4 µm or less, 3 µm or less, 2 µm or less, or 1 µm or less) is generally sufficient to retain encapsulated cells inside the device. The precise size of the pores can be adjusted based on the size of the encapsulated cells, and the size of the secreted biomolecule.

In some embodiments, the device can be a multi-scaffold device. A multi-scaffold device comprises one or more nanofibrous polymer scaffolds in addition to the multi-layer scaffold described above. For example, the device can comprise (i) a first scaffold surrounding a cell chamber, the first scaffold comprising an outer layer and an inner layer, wherein the outer layer and the inner layer each comprise a nanofibrous polymer, and (ii) a second scaffold positioned adjacent to the inner layer, wherein the second scaffold is in contact with the cell chamber. The second scaffold can optionally provide additional surface area for adherence of cells within the cell chamber. In one embodiment, the second scaffold comprises a nanofibrous polymer, e.g., an electrospun polymer. Any polymer set forth herein for incorporation into the first scaffold is similarly suitable for incorporation into the second scaffold. In exemplary embodiments, the second scaffold can comprise nPET, nPBT, nPU, or combinations thereof, e.g., nPET-PBT. In an exemplary embodiment, the second scaffold comprises nPBT. In some embodiments, the second scaffold comprises pores. As the second scaffold is surrounded by the first scaffold, the pores can be of any size, e.g., nanoporous or macroporous. In one embodiment, the second scaffold comprises pores of about the same size as those in the first scaffold. In another embodiment, the second scaffold comprises pores that are larger than those in the first scaffold. In another embodiment, the second scaffold comprises pores that are smaller than those in the first scaffold. An exemplary multi-scaffold device is depicted in Fig. 1D.

In some embodiments, the outer (first) scaffold comprises pores that are larger in size than the pores of the inner (second) scaffold. For example, in some embodiments, the outer scaffold can comprise pores sized to permit ingrowth of capillaries for perfusion of the inner chamber bioreactor, while the inner scaffold comprises pores sized to permit the passage of biomolecules while blocking the passage of cells. In one embodiment, the outer scaffold comprises pores of about 5-15 µm (e.g., 5-7 µm, 5-10 µm, 5-12 µm, 7-15 µm, 10-15 µm, etc.), reflecting the approximate diameter of capillaries, while the inner scaffold has a diameter of 1 µm or less (e.g., about 0.9 µm or less, about 0.8 µm or less, about 0.7 µm or less, about 0.6 µm or less, about 0.5 µm or less, about 0.4 µm or less, about 0.3 µm or less, about 0.2 µm or less, about 0.1 µm or less, or about 0.05 µm or less) to retain cells in the chamber.

The scaffold of the present cell chamber device can optionally comprise a porous, semi-porous, or non-porous membrane positioned between the inner layer and the outer layer, thereby forming a trilayer scaffold. The membrane is alternatively referred to herein as a filter.

In some embodiments, the membrane can comprise a nanofibrous polymer. For example, in some embodiments, the membrane can comprise nanofibrous polybutylene terephthalate. A nanofibrous membrane can contain pores resulting from the random, web-like arrangement of nanofibers, as described above with respect to the scaffold.

In other embodiments, the membrane is not nanofibrous. For example, the membrane can comprise a solid sheet interspersed with nanopores of a pre-determined size. The non-nanofibrous membrane can be made up of virtually any biocompatible polymer, including but not limited to any of the polymers described herein, such as, for example, PET, PBT, or PU. In some embodiments, the non-nanofibrous membrane can comprise one or more of the following polymers: mixed cellulose esters (MCE), cellulose acetate, coated cellulose acetate, hydrophilic PTFE, hydrophobic PTFE, nylon, or polycarbonate. In some embodiments, electrospun fibers can coat one or both sides of the membrane. For example, a solid (non-nanofibrous) film containing pores of a defined size can be coated with electrospun fibers, e.g., nPET-PBT or nPU. In one embodiment, the scaffold comprises a solid (non-nanofibrous) membrane interspersed with nanopores, that is coated with electrospun nPET-PBT on one side, and electrospun nPU on the other side. The solid membrane interspersed with nanopores can comprise a polymer sheet (e.g., composed of PET, PBT, PU, or other suitable polymer, or a combination thereof) that is not nanofibrous. In an exemplary embodiment, the solid membrane comprises PET. In other exemplary embodiments, the solid membrane comprises PU. Incorporation of a non-nanofibrous membrane into the scaffold can serve as a barrier to block the passage of cells that may otherwise push through a nanofibrous material. In such embodiments, the membrane can comprise pores sized to block the passage of cells. Scaffolds containing a solid (non-nanofibrous) membrane as an external surface can provoke inflammation in a host subject at the site of implantation. Coating a solid (non-nanofibrous) membrane with electrospun fibers can significantly reduce the inflammatory response to the scaffold, relative to a scaffold composed of a solid membrane without a coating of electrospun fibers.

Membrane nanopores can be sized to permit the passage of biomolecules, e.g., proteins, across the membrane, while blocking the passage of cells. In one embodiment, the membrane pores are sized to permit the passage of biomolecules having a molecular weight of about 250 kDa or less (e.g., about 225 kDa or less, about 220 kDa or less, about 200 kDa or less, about 175 kDa or less, about 150 kDa or less, about 125 kDa or less, about 100 kDa or less, about 75 kDa or less, about 50 kDa or less, about 25 kDa or less, or about 10 kDa or less). In some embodiments, the membrane pores are sized to permit the passage of a biomolecules having a molecular weight of 10 kDa to 50 kDa, 50 kDa to 100 kDa, 100 kDa to 150 kDa, 150 kDa to 200 kDa, or 200 kDa to 250 kDa. The membrane pores may also be sized to prevent the passage of cells and/or to prevent cells on one side of the scaffold from contacting cells on the other side of the scaffold. Accordingly, in some embodiments, the membrane comprises nanopores that have a diameter of about 1 µm or less (e.g., about 0.9 µm or less, about 0.8 µm or less, about 0.7 µm or less, about 0.6 µm or less, about 0.5 µm or less, about 0.4 µm or less, about 0.3 µm or less, about 0.2 µm or less, about 0.1 µm or less, or about 0.05 µm or less). In certain embodiments, the membrane nanopores have a diameter of 0.5 µm or less. In some embodiments, the membrane nanopores have a diameter of 0.1 µm to 0.4 µm, 0.4 µm to 0.6 µm, 0.6 µm to 0.8 µm, or 0.8 µm to 1 µm. In some embodiments, the membrane nanopores have a diameter of 0.1-3 µm. In other embodiments, the membrane nanopores have a diameter of 0.2-2 µm. In some embodiments, the membrane nanopores have a diameter of 1 nm to 10 µm (e.g., 1 nm to 10 nm, 5 nm to 25 nm, 10 nm to 50 nm, 25 nm to 75 nm, 50 nm to 100 nm, 75 nm to 125nm, 100 nm to 200 nm, 150 nm to 250 nm, 200 nm to 300 nm, 250 nm to 500 nm, 300 nm to 400 nm, 350 nm to 450 nm, 400 nm to 500 nm, 450 nm to 550 nm, 500 nm to 600 nm, 500 nm to 1µm, 550 nm to 650 nm, 600 nm to 700 nm, 650 nm to 750 nm, 700 nm to 800 nm, 750 nm to 850 nm, 800 nm to 900 nm, 900 nm to 1 µm, 1 µm to 2 µm, 1 µm to 5 µm, 1 µm to 10 µm, 2 µm to 4 µm, 2 µm to 6 µm, 2 µm to 10 µm, 4 µm to 6 µm, 4 µm to 8 µm, 4 µm to 10 µm, or 5 µm to 10 µm). In some embodiments, the membrane pores are about the same size as the pores in the outer and/or inner layer of the nanofibrous polymer scaffold. In other embodiments, the membrane pores are smaller than the pores in the outer and/or inner layer of the nanofibrous polymer scaffold. In other embodiments, the membrane pores are larger than the pores in the outer and/or inner layer of the nanofibrous polymer scaffold.

In some embodiments, one or more electrospun polymers can optionally be applied to a porous, semi-porous, or non-porous membrane to form a tri-layered material, which can be used to fabricate some embodiments of the cell chamber devices described herein. In such embodiments, a pre-set size polyester film or membrane (e.g., about 3 mm to about 300 mm, such as 3 mm to 10 mm, 10 mm to 25 mm, 25 mm to 50 mm, 50 mm to 75 mm, 75 mm to 100 mm, 100 mm to 125 mm, 125 mm to 150 mm, 150 mm to 175 mm, 175 mm to 200 mm, 200 mm to 225 mm, 225 mm to 250 mm, 250 mm to 275 mm, or 275 mm or 300 mm) can be loaded onto the mandrel or a grounded collector of the electrospinning unit. The electrospinning unit can then be operated in accordance with standard operating procedures to coat one or more electrospun polymers (e.g., PET and PBT) on one or both sides of the polyester film or membrane. In certain embodiments, the electrospun material comprises a film or membrane (e.g., PET membrane) coated with nPET-PBT on one side of the membrane and nPET-PBT and/or nPU on the other side of the membrane. For example, the device can comprise a scaffold comprising a film or membrane, with an nPET/PBT layer applied on both sides of the membrane, and an nPU layer applied on top the nPET/PBT layer on one side of the membrane. In another embodiment, the device can comprise a scaffold comprising a film or membrane, with an nPET/PBT layer applied on one side of the membrane, and an nPU layer applied on the other side of the membrane. Optionally, an adhesive can be applied to one or both sides of the membrane to help bind the layers together. In some embodiments, an electrospun adhesive is applied between the membrane and the nanofibrous polymer layers deposited thereon. In some embodiments, the electrospun adhesive can comprise the same electrospun fiber materials as one or more of the other layers.

Similar to the inner layer or outer layer of the scaffold, the nanoporous film or membrane may comprise membrane nanopores sized to permit the passage of biomolecules secreted by cells in the cell chamber across the scaffold and into the host tissue or blood and/or to permit the passage of biomolecules (e.g., nutrients) from the host into the chamber to feed the cells seeded in the device. For example, the membrane nanopores may be sized to permit the passage of any biomolecules described herein, such as polypeptides, polysaccharides, and polynucleotides as well as organic molecules such as lipids (e.g., phospholipids, glycolipids and sterols), chemical messengers (e.g., hormones and neurotransmitters), vitamins, sugars (e.g., carbohydrate, disaccharide, oligosaccharides, polysaccharides), amino acids, peptides, oligopeptides, polypeptides, proteins, nucleotides, deoxyribonucleic acid (DNA), or ribonucleic acid (RNA). In certain embodiments, the membrane nanopores are sized to permit the passage of a protein, such as an antibody, or antigen-binding portion thereof.

In some embodiments, the membrane nanopores are sized to permit the passage of biomolecules having a molecular weight of about 250 kDa or less (e.g., about 225 kDa or less, about 220 kDa or less, about 200 kDa or less, about 175 kDa or less, about 150 kDa or less, about 125 kDa or less, about 100 kDa or less, about 75 kDa or less, about 50 kDa or less, about 25 kDa or less, or about 10 kDa or less). In some embodiments, the membrane nanopores are sized to permit the passage of a biomolecules having a molecular weight of 10 kDa to 50 kDa, 50 kDa to 100 kDa, 100 kDa to 150 kDa, 130 to 165 kDa, 150 kDa to 200 kDa, or 200 kDa to 250 kDa.

In exemplary embodiments, the cell chamber device comprises a multilayer scaffold surrounding a cell chamber, wherein the multilayer scaffold comprises an outer layer comprising nanofibrous polyethylene terephthalate and polybutylene terephthalate, and an inner layer comprising nanofibrous polyurethane, wherein the outer layer and the inner layer comprise nanopores having a diameter of 1 nm to 10 um (e.g., 1 nm to 10 nm, 5 nm to 25 nm, 10 nm to 50 nm, 25 nm to 75 nm, 50 nm to 100 nm, 75 nm to 125nm, 100 nm to 200 nm, 150 nm to 250 nm, 200 nm to 300 nm, 250 nm to 500 nm, 300 nm to 400 nm, 350 nm to 450 nm, 400 nm to 500 nm, 450 nm to 550 nm, 500 nm to 600 nm, 500 nm to 1µm, 550 nm to 650 nm, 600 nm to 700 nm, 650 nm to 750 nm, 700 nm to 800 nm, 750 nm to 850 nm, 800 nm to 900 nm, 900 nm to 1 µm, 1 µm to 2 µm, 1 µm to 5 µm, 1 µm to 10 µm, 2 µm to 4 µm, 2 µm to 6 µm, 2 µm to 10 µm, 4 µm to 6 µm, 4 µm to 8 µm, 4 µm to 10 µm, or 5 µm to 10 µm). In other exemplary embodiments, the cell chamber device comprises a trilayer scaffold surrounding a cell chamber, wherein the trilayer scaffold comprises an outer layer comprising nanofibrous polyethylene terephthalate and polybutylene terephthalate, an inner layer comprising nanofibrous polyurethane, and a membrane positioned between the inner layer and outer layer comprising nanofibrous polybutylene terephthalate, wherein the outer layer, the inner layer, and the membrane comprise nanopores having a diameter of 1 nm to 10 um (e.g., 1 nm to 10 nm, 5 nm to 25 nm, 10 nm to 50 nm, 25 nm to 75 nm, 50 nm to 100 nm, 75 nm to 125nm, 100 nm to 200 nm, 150 nm to 250 nm, 200 nm to 300 nm, 250 nm to 500 nm, 300 nm to 400 nm, 350 nm to 450 nm, 400 nm to 500 nm, 450 nm to 550 nm, 500 nm to 600 nm, 500 nm to 1µm, 550 nm to 650 nm, 600 nm to 700 nm, 650 nm to 750 nm, 700 nm to 800 nm, 750 nm to 850 nm, 800 nm to 900 nm, 900 nm to 1 µm, 1 µm to 2 µm, 1 µm to 5 µm, 1 µm to 10 µm, 2 µm to 4 µm, 2 µm to 6 µm, 2 µm to 10 µm, 4 µm to 6 µm, 4 µm to 8 µm, 4 µm to 10 µm, or 5 µm to 10 µm).

In some embodiments, one or more layers of the scaffold comprise pores having a diameter of 1 µm or less.

A cell chamber device described herein can be of any size or shape suitable for administration to a host subject (e.g., administration by surgery, such as a minimally invasive surgery e.g., laparoscopic surgery or endoscopic surgery). The size and shape can be altered as needed to accommodate the desired number of cells that can be loaded into the device, to tailor the device to the site of implantation, and/or adjust the dosage of the therapeutic biomolecule secreted by cells within the device into a recipient subject (e.g., as measured by mg of biomolecule secreted from the device per day).

In some embodiments, the cell chamber is sized to accommodate up to about 1x10¹¹ cells (e.g., up to about 1x10¹¹ cells, up to about 1x10¹⁰ cells, up to about 1x10⁹ cells, up to about 1x10⁸ cells, up to about 1x10⁷ cells, up to about 1x10⁶ cells, up to about 1x10⁵ cells, up to about 1x10⁴ cells, or up to about 1x10³ cells). In some embodiments, the cell chamber is sized to accommodate up to 1x10⁷ cells. In some embodiments, the cell chamber is sized to accommodate up to 1x10¹¹ cells. The number of cells loaded into the device can readily be determined by one of ordinary skill in the art based on the desired dose of the biomolecule to be delivered and the amount of the biomolecule produced by the cells. For example, if a 10 µg dose per day is needed, and cells make 1 pg/cell/day, the chamber can be designed to accommodate 1 x 10⁷ cells.

The cell chamber device can be fabricated in any configuration appropriate for stable implantation at the desired site of delivery of the biomolecule. Accordingly, in various embodiments, the device can be substantially cylindrical, planar, disk-shaped, patch-shaped, ovoid, stellate, tubular, or spherical. In some embodiments, the cell chamber device is planar or substantially planar. In some embodiments, the cell chamber device is planar or substantially planar, and may be shaped as or approximating a rectangle, a square, a triangle, circle, a pentagon, a hexagon, a heptagon, or an octagon. In other embodiments, the device is not planar. For example, in some embodiments, the device can be shaped as or approximating a sphere, a cylinder, a rod, a cube, etc. In some embodiments, the cell chamber shape can be arranged as a spiral, or folded to maximize surface area.

In certain embodiments, the cell chamber device is a rectangle. For example, in some embodiments, the rectangular cell chamber device can have a length of about 15 cm or less, about 12 cm or cell, about 10 cm or less, about 8 cm or less, about 6 cm or less, about 4 cm or less, about 2 cm or less, about 1 cm or less, about 0.1 cm (100 mm) or less, or about 0.01 cm (10 mm) or less. In some embodiments, a rectangular cell chamber device has a width of about 15 cm or less, about 12 cm or cell, about 10 cm or less, about 8 cm or less, about 6 cm or less, about 4 cm or less, about 2 cm or less, about 1 cm or less, about 0.1 cm (100 mm) or less, or about 0.01 cm (10 mm) or less. In some embodiments, the cell chamber device has a width of about 5-10 cm and a length of about 10-15 cm. In some embodiments, the cell chamber device has a width of about 3-5 cm and a length of about 5-10 cm. In some embodiments, the cell chamber device has a width of about 1-3 cm and a length of about 3-5 cm. In some embodiments, the cell chamber device has a width of about 0.01-1 cm and a length of about 1-3 cm. In some embodiments, the cell chamber device has a first dimension of about 0.01 cm, about 0.1 cm, about 1 cm, about 2 cm, about 3 cm, about 4 cm, about 5 cm, about 6 cm, about 7 cm, about 8 cm, about 9 cm, about 10 cm, about 11 cm, about 12 cm, about 13 cm, about 14 cm or about 15 cm, and a second dimension of about 0.01 cm, about 0.1 cm, about 1 cm, about 2 cm, about 3 cm, about 4 cm, about 5 cm, about 6 cm, about 7 cm, about 8 cm, about 9 cm, about 10 cm, about 11 cm, about 12 cm, about 13 cm, about 14 cm or about 15 cm. In certain embodiments, the cell chamber device has a width of about 8 cm and a length of about 10 cm. In other embodiments, the cell chamber device has a width of about 5 cm and a length of about 9 cm. In other embodiments, the cell chamber device has a width of about 3 cm and a length of about 5 cm. In other embodiments, the cell chamber device has a width of about 1 cm and a length of about 3 cm.

In some embodiments, the device has a surface area of about 250 cm² or less, for example, about 225 cm² or less, about 220 cm² or less, about 200 cm² or less, about 175 cm² or less, about 150 cm² or less, about 125 cm² or less, about 100 cm² or less, about 75 cm² or less, about 50 cm² or less, about 25 cm² or less, or about 10 cm² or less.

In some embodiments, the device has a volume of about 5 cm³ or less, for example, about 4.5 cm³ or less, about 4 cm³ or less, about 3.5 cm³ or less, about 3 cm³ or less, about 2.5 cm³ or less, about 2 cm³ or less, about 1.5 cm³ or less, about 1 cm³ or less, or about 0.5 cm³ or less.

The device may be of any suitable thickness to accommodate the desired site of implantation. In some embodiments, the device is substantially planar, and comprises a total thickness that is 20% or less, 15% or less, 10% or less, 9% or less, 8% or less, 7% or less, 6% or less, 5% or less, 4% or less, 3% or less, 2% or less or 1% or less of the next smallest dimension, e.g., width, of the device. In some embodiments, the device comprises a total thickness of about 1 mm or less, 0.9 mm or less, 0.8 mm or less, 0.7 mm or less, 0.6 mm or less, 0.5 mm or less, 0.4 mm or less, 0.3 mm or less, 0.2 mm or less, or 0.1 mm or less. In some embodiments, the device comprises a total thickness of about 250 µm or less (e.g., about 225 µm or less, about 220 µm or less, about 200 µm or less, about 175 µm or less, about 150 µm or less, about 125 µm or less, about 100 µm or less, about 75 µm or less, about 50 µm or less, about 25 µm or less, or about 10 µm or less). In some embodiments, the device comprises a total thickness of 10 µm to 50 µm, 10 µm to 100 µm, 50 µm to 100 µm, 50 µm to 200 µm,100 µm to 150 µm, 100 µm to 250 µm, 100 µm to 500 µm, 150 µm to 200 µm, 200 µm to 250 µm, 250 µm to 300 µm, 250 µm to 500 µm, 300 µm to 350 µm, 350 µm to 400 µm, 400 µm to 450 µm, 450 µm to 500 µm, 500 µm to 550 µm, 500 µm to 1 mm, 550 µm to 600 µm, 600 µm to 650 µm, 650 µm to 700 µm, 700 µm to 750 µm, 750 µm to 800 µm, 750 µm to 1 mm, 800 µm to 850 µm, 850 µm to 900 µm, 900 µm to 950 µm, or 950 µm to 1 mm. In certain embodiments, the device comprises a total thickness of 150 µm or less.

The cell chamber device disclosed herein may optionally comprise a loading port to facilitate the loading of cells into the cell chamber. Such a loading port can traverse the layers of the multi-layer scaffold. The loading port can include an external opening facing the outside of the cell chamber device (e.g., for inserting a cell loading or injection instrument) and an internal opening facing the inside of the cell chamber (e.g., to deposit cells in the cell chamber via the cell loading or injection instrument). The loading port may be sealed to prevent loaded cells inside the cell chamber from escaping from the cell chamber. Following cell loading, the opening in the chamber can be sealed by a variety of methods, such as ultrasonic welding. Other examples of sealing processes known in the art include thermal staking (e.g., cold staking or heat steaking), swaging, spin welding, hot-plate welding, vibration welding, or laser welding.

### III. Cells

The device disclosed herein can accommodate cells in the cell chamber encased by the multilayer scaffold. Accordingly, in some embodiments, the device can optionally comprise cells. Cells can be loaded into the cell chamber via a cell loading port, or through an opening in the bilayer scaffold that can subsequently be sealed, e.g., using the methods described herein. The cell chamber is contacted by the inner layer of the bilayer scaffold such that cells loaded into the cell chamber can optionally adhere to the inner layer of the scaffold.

In some embodiments, cell lines selected for use in the cell chamber device can have one or more of the following characteristics: (1) the cells can be hardy under stringent conditions; (2) the cells can be able to be genetically modified to produce (e.g., secrete) a desired therapeutic biomolecule; (3) the cells can have a relatively long life span or shelf life (e.g., greater than 1 month); (4) in instances where the subject is human, the cells can be of human origin to increase compatibility between the encapsulated cells and the host; (5) the cells can exhibit high viability in the device to ensure long-term delivery (e.g., greater than 80% viability for a period of more than one month *in vivo* in device); (6) the encapsulated cells can deliver an efficacious quantity of a useful biological product; (7) the cells can have a low level of host immune reaction to ensure the longevity of the graft; and/or (8) the cells can be non-tumorigenic to provide added safety to the host, in the event of device leakage.

Mammalian cells known in the art for the production of mammalian proteins can be suitable for use in the cell chamber device, in some embodiments. Chinese hamster ovary (CHO) cells, and cell lines obtained from various other mammalian sources, such as, for example, mouse myeloma (NS0), baby hamster kidney (BHK), human embryonic kidney (HEK-293) and human retinal cells have been approved by regulatory agencies for the production of biopharmaceutical products, including therapeutic antibodies. Examples of mammalian host cells include CHO, BHK, HEK293 COS, PC12, HiB5, RN33b, C2C12, HepG2, and ARPE-19 cells.

In certain embodiments, the cells are human cells, including recombinant cells of human origin. In one embodiment, the cells comprise human retinal pigment epithelial (RPE) cells, or cells derived therefrom. In exemplary embodiments, the cells comprise ARPE-19 cells. The ARPE-19 cell line (see, e.g., Dunn et al, 62 Exp. Eye Res. 155-69 (1996), Dunn et al, 39 Invest. Ophthalmol. Vis. Sci. 2744-9 (1998), Finnemann et al., 94 Proc. Natl. Acad. Sci. USA 12932-7 (1997), Handa et al., 66 Exp. Eye. 411-9 (1998), Holtkamp et al., 112 Clin. Exp. Immunol. 34-43 (1998), Maidji et al., 70 J. Virol. 8402-10 (1996); United States Patent No. 6,361,771) possesses many characteristics of a platform cell line for use with the cell chamber devices described herein. The ARPE-19 cell line is available from the American Type Culture Collection (ATCC Number CRL-2302). ARPE-19 cells are normal retinal pigmented epithelial (RPE) cells and express the retinal pigment epithelial cell- specific markers CRALBP and RPE-65. ARPE-19 cells form stable monolayers, which exhibit morphological and functional polarity. The ARPE-19 cell line is viable under stringent conditions, such as during implantation in a host subject; can be genetically modified to secrete a biomolecule of therapeutic interest, has a relatively long life, and is of human origin. In addition, encapsulated ARPE-19 cells have good viability *in vivo* in devices, cause an insignificant immune reaction in a human host, and are not tumorigenic. In other embodiments, the cells are human hepatocytes. In other embodiments, the cells are human islet cells.

The cells can be transformed or non-transformed. Further, the cells can grow in suspension or adherent. In some embodiments, the cells are contact inhibited cells, e.g., immortalized contact inhibited human cells (e.g., hTERT immortalized cell lines (Evercyte, Vienna, AT). Cells are known in the art for recombinant protein production. For example, mammalian cell lines that may be used in the devices described herein include, in some embodiments, monkey kidney CVI line transformed by SV40 (COS-7, ATCC^{™} CRL 1651); baby hamster kidney cells (BHK, ATCC^{™} CCL 10); mouse sertoli cells (TM4, Mather, Biol. Reprod., 23:243 (1980)); monkey kidney cells (CVI-76, ATCC^{™} CCL 70); African green monkey kidney cells (VERO-76, ATCC^{™} CRL-1587); canine kidney cells (MDCK, ATCC^{™} CCL 34); buffalo rat liver cells (BRL 3A, ATCC.RTM. CRL 1442); mouse mammary tumor cells (MMT 060562, ATCCV CCL 51); rat hepatoma cells (HTC, MI.54, Baumann et al., J. Cell Biol., 85:1 (1980)), 3T3 cells; 293T cells (Pear, W. S., et al., Proc. Natl. Acad. Sci. U.S.A., 90:8392-8396 (1993)); NS0 cells (Sato et al. Tissue Culture Association, 24:1223 (1988)); SP2/0 (Sato et al. J. Exp. Med., 165:1761 (1987)); TR-1 cells (Mather et al., Annals N.Y. Acad. Sci., 383:44 (1982)), and hybridoma cell lines. In some embodiments, the cell line is a human cell line, such as human embryonic kidney line 293S (Graham et al., J. Gen. Virolo., 36:59 (1977)); human cervical carcinoma cells (HELA, ATCC^{™} CCL 2); human lung cells (W138, ATCC^{™} CCL 75); human liver cells (Hep G2. HB 8065); hTERT immortalized cell lines (Evercyte, Vienna, AT), or human retinal cells. In some embodiments, the cell line is an oncogenic cell line that has been modified or treated to be rendered safe for use in a cell chamber device provided herein. In some embodiments, the cell chamber device can contain stem cells, e.g., human stem cells, such as induced pluripotent stem cells (iPS), embryonic stem cells (ES) or mesenchymal stem cells (MSC), or differentiated cells derived from such stem cells.

In some instances, the cells loaded into the cell chamber device of the present disclosure may be genetically modified cells, e.g., recombinant cells, that have been engineered to produce, e.g., secrete, a biomolecule of interest. Biomolecules that can be secreted by the cells of the device include, for example and without limitation, polypeptides, polysaccharides, and polynucleotides, as well as organic molecules such as lipids (e.g., phospholipids, glycolipids and sterols), chemical messengers (e.g., neurotransmitters and hormones, such as insulin), vitamins, sugars (e.g., carbohydrate, disaccharide, oligosaccharides, polysaccharides), amino acids, peptides, oligopeptides, polypeptides, proteins, nucleotides, deoxyribonucleic acid (DNA), or ribonucleic acid (RNA). Other secreted biomolecules may include those associated, packaged, and secreted as exosomes, lipid polymers, or viral particles. In some embodiments, the cells produce a therapeutic biomolecule, as described herein.

In some embodiments, the cells in the device can be engineered to secrete one or more proteins or peptides, e.g., one or more recombinant proteins or peptides, e.g., one or more therapeutic proteins or peptides. For example, the cells in the device can secrete one or more therapeutic proteins such as an antibody or antigen-binding fragment thereof, a growth factor, a hormone (e.g., insulin), a cytokine, a clotting factor (e.g., Factor VIII, or Factor IX, or variants thereof, e.g., Recombinate, Kogenate, Refacto, Advate, Alprolix, BeneFIX, Rixubis, Ixinity, Idelvion, etc.), or a combination thereof. The protein can be, in some instances, a recombinant protein or peptide.

In some embodiments, cells in the device can secrete one protein or peptide, e.g., one recombinant or therapeutic protein or peptide. In other embodiments, the cells in the device can secrete two or more proteins, e.g., 2, 3, 4, 5, 6, 7, 8, 9, 10 or more recombinant or therapeutic proteins or peptides. The device can, in some embodiments, be loaded with a single cell type, e.g., a single cell line, that secretes multiple proteins or peptides. In other embodiments, the device can be loaded with two or more, e.g., 2, 3, 4, 5, 6, 7, 8, 9, 10 or more, cell types, e.g., cell lines, each of which secretes one or more proteins or peptides. For example, in one embodiment in which the cell chamber device is used to provide two secreted proteins to a subject, the device can be loaded with a cells that secrete two recombinant proteins or peptides. In another embodiment, the device can be loaded with two cell lines, each of which secretes a single recombinant protein or peptide.

In some embodiments, the cells in the device can secrete one or more growth factors, including but not limited to fibroblast growth factor (FGF), epidermal growth factor (EGF), platelet derived growth factor (PDGF), insulin-like growth factor (IGF), transforming growth factor (TGF), vascular endothelial growth factor (VEGF), liver growth factor (LGF), bone morphogenetic protein (BMP), colony stimulating factor (CSF), hepatocyte growth factor (HGF), or nerve growth factor (NGF), or combinations thereof.

In some embodiments, the cells in the device secrete one or more cytokines, including but not limited to bone morphogenetic protein (BMP), erythropoietin (EPO), granulocyte colony-stimulating factor (G-CSF), granulocyte macrophage colony-stimulating factor (GM-CSF), interferon alfa, interferon beta, interleukin 2 (IL-2), interleukin 11 (IL-11), or interferon gamma, or combinations thereof.

In some embodiments, the cells in the device secrete one or more hormones, including but not limited to insulin, estrogen, progestogen, thyroxine (as levothyroxine), or steroids, or combinations thereof.

In some embodiments, the cells in the device secrete one or more enzymes. For example, the cells in the device can secrete an enzyme that provides enzyme replacement therapy (ERT) to a subject. In some embodiments, the cells can produce enzymes that complement lysosomal storage disease deficiencies, including but not limited to hexosaminidase A, alpha-galactosidase A, glucocerebrosidase, arylsulfatase A, galactocerebrosidase, and sphingomyelinase. In some embodiments, the cells can produce enzymes that complement Hunter syndrome, also known as mucopolysaccharidosis type II (MPSII), including but not limited to idursulfase. In some embodiments, the cells can produce enzymes that complement metachromatic leukodystrophy (MLD), including but not limited to arylsulfatase A. In some embodiments, the cells can produce enzymes that complement mucopolysaccharidosis type I (MPSI), including but not limited to laronidase.

In some embodiments, the cells in the device can secrete one or more antibodies, or antigen-binding portion thereof. The antibody or antigen-binding portion thereof, described herein can be in the form of a full-length antibody, a bispecific antibody, a dual variable domain antibody, a multiple chain or single chain antibody, and/or antigen binding fragments that specifically bind an extracellular molecule, including but not limited to Fab, Fab', (Fab')2, Fv, scFv (single chain Fv), surrobodies (including surrogate light chain construct), single domain antibodies, camelized antibodies and the like. They also can be of, or derived from, any isotype, including, for example, IgA (e.g., IgA1 or IgA2), IgD, IgE, IgG (e.g. IgG1, IgG2, IgG3 or IgG4), or IgM. In some embodiments, the cells secrete an antibody fragment selected from the group consisting of a Fab, a F(ab')2, an scFv, a tandem scFv, a diabody, a minibody, and a single domain antibody. In some embodiments, the cells secrete a full length antibody.

In some embodiments, the cells secrete a chimeric antibody, or antigen-binding portion thereof. The term "chimeric antibody" is intended to refer to antibodies in which the variable region sequences are derived from one species and the constant region sequences are derived from another species, such as an antibody in which the variable region sequences are derived from a mouse antibody and the constant region sequences are derived from a human antibody.

In some embodiments, the cells secrete a humanized antibody, or antigen-binding portion thereof. "Humanized" forms of non-human (e.g., rodent) antibodies are chimeric antibodies that contain minimal sequence derived from the non-human antibody. For the most part, humanized antibodies are human immunoglobulins (recipient antibody) in which residues from a hypervariable region of a human "recipient" antibody are replaced by residues from a hypervariable region of a "donor" antibody from a non-human species such as mouse, rat, rabbit or nonhuman primate having the desired specificity, affinity, and capacity. In some instances, framework region (FR) residues of the human antibody can be replaced by corresponding non-human residues. Furthermore, in some instances, humanized antibodies can comprise residues that are not found in the recipient antibody or in the donor antibody. These modifications can further refine antibody performance. In some embodiments, a humanized antibody can comprise substantially all of at least one, and typically two, variable domains, in which all or substantially all of the hypervariable CDR loops correspond to those of a non-human antibody and all or substantially all of the FRs are those of a human antibody sequence. The humanized antibody optionally also will comprise at least a portion of an antibody constant region (Fc), typically that of a human antibody. For further details, see Jones et al., Nature 321:522-525 (1986); Riechmann et al., Nature 332:323-329 (1988); and Presta, Curr. Op. Struct. Biol. 2:593-596 (1992).

In some embodiments, the cells secrete a human antibody, or antigen-binding portion thereof. The term "human antibody", as used herein, refers to an antibody having variable regions in which both the framework and CDR regions are derived from human germline immunoglobulin sequences. Furthermore, if the antibody contains a constant region, the constant region also is derived from human germline immunoglobulin sequences. The human antibodies of the invention may include amino acid residues not encoded by human germline immunoglobulin sequences (e.g., mutations introduced by random or site-specific mutagenesis in vitro or by somatic mutation in vivo). However, the term "human antibody", as used herein, is not intended to include antibodies in which CDR sequences derived from the germline of another mammalian species, such as a mouse, have been grafted onto human framework sequences.

In some embodiments, the cells secrete a monoclonal antibody. The term "monoclonal antibody" as used herein refers to an antibody obtained from a population of substantially homogeneous antibodies, i.e., the individual antibodies comprising the population are identical in sequence and specificity, except for possible variant antibodies, e.g., containing naturally occurring mutations or arising during production of a monoclonal antibody preparation, such variants generally being present in minor amounts. In contrast to polyclonal antibody preparations, which typically include different antibodies directed against different determinants (epitopes), each monoclonal antibody of a monoclonal antibody preparation is directed against a single determinant on an antigen. Thus, the modifier "monoclonal" indicates the character of the antibody as being obtained from a substantially homogeneous population of antibodies and is not to be construed as requiring isolation of the antibody by any particular method. For example, the monoclonal antibodies to be used in accordance with the present invention may be derived using a variety of techniques, including but not limited to the hybridoma method, recombinant DNA methods, phage-display methods, and methods utilizing transgenic animals containing all or part of the human immunoglobulin loci, such methods and other exemplary methods for making monoclonal antibodies being known in the art and described herein.

In instances where the cells in the cell chamber secrete an antibody or antigen-binding portion thereof, the antibody, or antigen-binding portion thereof, can specifically bind any antigen of interest. In some embodiments, the antibody or antigen binding portion thereof can specifically bind to (i) tumor-associated antigens; (ii) cell surface receptors, (iii) CD proteins and their ligands, such as CD3, CD4, CD8, CD19, CD20, CD22, CD25, CD32, CD33, CD34, CD40, CD44, CD47, CD54, CD59, CD70, CD74, CD79a (CD79a), and CD79P (CD79b); (iv) members of the ErbB receptor family such as the EGF receptor, HER2, HER3 or HER4 receptor; (v) cell adhesion molecules such as LFA-1, Mac1, p150,95, VLA-4, ICAM-1, VCAM and αv/β3 integrin including either alpha or beta subunits thereof (e.g. anti-CD11a, anti-CD18 or anti-CD11b antibodies); or (vi) growth factors such as PDGF, FGF, VEGF; IgE; blood group antigens; flk2/flt3 receptor; obesity (OB) receptor; mpl receptor; CTLA4; protein C, BR3, c-met, tissue factor etc. In other exemplary embodiments, the antibody or antigen-binding portion thereof can specifically bind an antigen including, but not limited to, 4-1BB, 5T4, ACVR2B, ADAM-9, alpha-V integrin, AMHRII, AXL, BAFF, BAFF-R, basigin, BCMA, C242 antigen, c-Met, CA9, CA-125, CanAg, CCR2, CCR4, CCR5, CD2, CD3, CD3 epsilon, CD3E, CD4, CD5, CD6, CD11, CD11a, CD15, CD18, CD19, CD20, CD22, CD23, CD25, CD27, CD28, CD30, CD33, CD37, CD38, CD40, CD40L, CD41, CD44 v6, CD45, CD49b, CD51, CD52, CD54, CD56, CD70, CD74, CD79B, CD80, CD97B, CD98, CD99, CD117, CD123, CD125, CD134, CD137, CD138, CD147, CD152, CD154, CD163, CD166, CD184, CD200, CD205, CD221, CD248, CD276, CD278, CD279, CD319, CD352, CDH-6, CEA, CEA-CAM4, CEA-CAM5, CEA-Cide, CEA-related antigen, CEACAM1, CEACAM6, CFC1B, Claudin 18 Isoform 2, CLDN6, CLDN18.2, CSF1R, CTLA4, CXCR4, dendritic cell-associated lectin 2, DLK1, DLL3, DLL4, DR5, EFNA4, EGFR, EGFR extracellular domain III, EGFRviii, endoglin, endothelin receptor ETB, ENPP3, EpCAM, EphA, EPHA3, ephrin receptor A3, EphrinA4, episialin, ER-alpha36, ERBB1, ERBB2, ERBB3, FCGRT, FGFR, FGFR2, fibronectina extra domain-B, FLT3, folate receptor, folate receptor1, folate receptor alpha, FOLH1, Frizzled receptor, FXYD5, Ganglioside GD3, GCC, GCGR, GCL, GD2 ganglioside, GD3 ganglioside, Globo H, glypican 3, GMCSF receptor α-chain, GPC2, GPNMB, granulocyte antigen, GUCY2C, H-Ferritin, hepatitis B surface antigen, HER1, HER2, HER2/neu, HER3, HGFR, HLA-DR, human scatter factor receptor kinase, ICAM-1, ICOS, IgE receptor, IGF1R, IL6R, IL31RA, IL 3 receptor, IL-4Rα, IL-6R, IL-12/23, IL-17 receptor, integrin α4, integrin α4β7, integrin α5β1, integrin αIIbβ3, integrin αvβA, integrin β7, interferon receptor, interferon α/β receptor, ITGA2, ITGB2, KAAG-1, KIR2D, L-selectin, CD62L, LAG3, LAMP1, Le(y), LFA-1, LINGO-1, LIV-1, LRRC15, LY6E, LYPD3, MCAM, mesothelin, MS4A1, MSLN, MST1R, MT1-MMP, MTX3, MTX5, MUC1, mucin 16, mucosal addressin cell adhesion molecule (MAdCAM), myelin-associated glycoprotein, NCA-90, Nectin-4, NGNA ganglioside, NKA, NKG2A, Notch3, Notch 1, Notch receptor, NRP1, OFP, NaPi2b, OX-40, P-cadherin, P. aeruginosa antigen, PCDC1, PD-1, PD-L1, PDCD1, PDGF-R α, PDGFRA, phosphate-sodium co-transporter, phosphatidylserine, platelet-derived growth factor receptor beta, PRLR, PSMA, PTK7, RGMA, RHD, Rhesus factor, Rhesus factor, ROR1, Ror2, RSVFR, SAIL, SDC1, selectin P, SLAMF7, SLC34A2, SLC44A4, SLeA, SLITRK5, SLITRK6, soluble IL-6, SOST, SSTR2, STEAP1, STn, T-cell receptor, TACSTD2, TAG-72, TEM1, TF, TIGIT, TIM-1, TM4SF1, TNF-α, TNFR superfamily member 4, TNFRSF17, TRAIL-R1, TRAIL-R2, TROP2, TWEAK receptor, VEGFA, VEGFR2, VEGFR-1, VSIR, or VWF. Other examples of antigens that can be targeted by the antibody, or an antigen-binding fragment thereof, include cell surface receptors such as those described in Chen and Flies. Nature reviews immunology. 13.4 (2013): 227, which is incorporated herein by reference.

In some embodiments, the antibody secreted by cells in the device is 3F8 (binds GD2 ganglioside), abciximab (ReoPro; binds CD41), abituzumab (binds CD51), alemtuzumab (Lemtrada, Campath; binds CD52), abrilumab (binds integrin α4β7), adalimumab (Humira; binds TNF-α), adecatumumab (binds EpCAM), alacizumab pegol (binds VEGFR2), alemtuzumab (Lemtrada, Campath; binds CD52), altumomab pentetate (Hybri-ceaker; binds CEA), amatuximab (binds mesothelin), anatumomab mafenatox (binds TAG-72), anetumab ravtansine (binds MSLN), anifrolumab (binds interferon α/β receptor), apolizumab (binds HLA-DR), aprutumab ixadotin (binds FGFR2); arcitumomab (binds CEA), aselizumab (binds L-selectin or CD62L), atezolizumab (Tecentriq; binds PD-L1), atorolimumab (binds Rhesus factor), avelumab (Bavencio; binds PD-L1), avicixizumab (binds DLL4; VEGFA), azintuxizumab vedotin (binds CD319); basiliximab (Simulect; binds CD25), bavituximab (binds phosphatidylserine), BCD-100 (binds PD-1), bectummomab (LymphoScan; binds CD22), belantamab mafodotin (binds BCMA); belimumab (Benlysta; binds BAFF), bemarituzumab (binds FGFR2), benralizumab (Fasenra; binds CD125), bersanlimab (binds ICAM-1), besilesomab (Scintimun; binds CEA-related antigen), bimagrumab (binds ACVR2B), bivatuzumab mertansine (binds CD44 v6), bleselumab (binds CD40), blinatumomab (Blincyto; binds CD19), blosozumab (binds SOST); brentuximab vedotin (Adcentris; binds CD30), brontictuzumab (binds Notch 1), brodalumab (Siliq; binds IL-17 receptor), cabiralizumab (binds CSF1R), camidanlumab tesirine (binds CD25), camrelizumab (binds PD-1), carotuximab (binds endoglin), catumaxomab (Removab; binds EpCAM/CD3), cantuzumab ravtansine (binds MUC1), caplacizumab (Cablivi; binds VWF), cedelizumab (binds CD4); cemipilimab (Libtayo; binds PCDC1), cetrelimab (binds PD-1), certolizumab (binds TNF-α), cergutuzumab amunaleukin (binds CEA), cetuximab (Erbitux; binds EGFR), cibisatamab (binds CEACAM5), cirmtuzumab (binds ROR1), cixutumumab (binds IGF-1 receptor; CD221), clenoliximab (binds CD4), clivatuzumab tetraxetan (binds hPAM4-Cide; MUC1), codrituzumab (binds glypican 3), coltuximab ravtansine (binds CD19), conatumumab (binds TRAIL-R2), crizanlizumab (binds selectin P), crotedumab (binds GCGR), dacetuzumab (binds CD40), daclizumab (Zenapax; binds CD25), dalotuzumab (binds IGF-1 receptor; CD221), dapirolizumab pegol (binds CD154; CD40L), daratumumab (Darzalex; binds CD38), demcizumab (binds DLL4), denintuzumab mafodotin (binds CD19), depatuxizumab mafodotin (binds EGFR), drozitumab (binds DR5); DS-8201 (binds HER2), deligotuzumab (binds ERBB3; HER3), dinutuximab (Unituxin; binds GD2 ganglioside), dupilumab (binds IL-4Rα), durvalumab (Imfinzi; binds PD-L1), duvortuxizumab (binds CD19; CD3E), ecromeximab (binds GD3 ganglioside), edrecolomab (binds EpCAM); elezanumab (binds RGMA), elgemtumab (binds ERBB3, HER3); elotuzumab (binds SLAMF7), emactuzumab (binds CSF1R), enapotamab vedotin (binds AXL), enavatuzumab (binds TWEAK receptor), enlimonomab pegol (binds ICAM-1; CD54), enoblituzumab (binds CD276), enoticumab (binds DLL4), epratuzumab (binds CD22), erlizumab (binds ITGB2; CD18), ertumaxomab (Rexomun; binds HER2/neu; CD3), etaracizumab (Abergin; binds integrin αᵥβ₃), etigilimab (binds TIGIT), etrolizumab (binds integrin β₇), exbivirumab (binds hepatitis B surface antigen), fanolesomab (NeutroSpec; binds CD15), faralimomab (binds interferon receptor), farletuzumab (binds folate receptor1), FBTA05 (Lymphomun; binds CD20), fgatipotuzumab (binds MUC1), fibatuzumab (binds ephrin receptor A3), figitumumab (binds IGF-1 receptor; CD221), flotetuzumab (binds IL 3 receptor); foralumab (binds CD3 epsilon); futuximab (binds EGFR), galiximab (binds CD80), gancotamab (binds HER2/neu), ganitumab (binds IGF-1 receptor; CD221), gavilimomab (binds CD147; basigin), gemtuzumab ozogamicin (Mylotarg; binds CD33), glembatumumab vedotin (binds GPNMB), golimumab (Simponi; binds TNF-α), gomiliximab (binds CD23; IgE receptor), ianalumab (binds BAFF-R), ibalizumab (Trogarzo; binds CD4), IBI308 (binds PD-1), ibritumomab tiuxetan (binds CD20), icrucumab (binds VEGFR-1), ifabotuzumab (binds EPHA3), igovomab (Indimacis-125; binds CA-125), IMAB362 (binds CLDN18.2), imaprelimab (binds MCAM), inclacumab (binds selectin P), indatuximab ravtansine (binds SDC1), iladatuzumab vedotin (binds CD97B), imgatuzumab (binds EGFR), indusatumab vedotin (binds GUCY2C), inebilizumab (binds CD19), infliximab (Remicade; binds TNF-α), intetumumab (binds CD51), inolimomab (binds CD25), inotuzumab ozogamicin (Besponsa; binds CD22), ipilimumab (Yervoy; binds CD152), iomab-B (binds CD45), iratumumab (binds CD30), isatuximab (binds CD38), iscalimab (binds CD40), istiratumab (binds IGF1R; CD221), itolizumab (Alzumab; binds CD6), keliximab (binds CD4), laprituximab emtansine (binds EGFR), labetuzumab (binds CEA-Cide; CEA), lifastuzumab vedotin (binds phosphate-sodium co-transporter), lemalesomab (binds NCA-90; granulocyte antigen), lenvervimab (binds hepatitis B surface antigen), leronlimab (binds CCR5), lexatumumab (binds TRAIL-R2), libivirumab (binds hepatitis B surface antigen), loncastuximab tesirine (binds CD19), losatuxizumab vedotin (binds EGFR; ERBB1; HER1), lilotomab satetraxetan (binds CD37), lintuzumab (binds CD33), lirilumab (binds KIR2D), lorvotuzumab mertansine (binds CD56), lucatumumab (binds CD40), lulizumab pegol (binds CD28), lumiliximab (binds CD23; IgE receptor), lumretuzumab (binds ERBB3; HER3), lupartumab amadotin (binds LYPD3), mapatumumab (binds TRAIL-R1), margetuximab (binds HER2), maslimomab (binds T-cell receptor), mavrilimumab (binds GMCSF receptor α-chain), matuzumab (binds EGFR), milatuzumab (binds CD74), minretumomab (binds TAG-72), mirvetuximab soravtansine (binds folate receptor alpha), mitumomab (binds GD3 ganglioside), morolimumab (binds Rhesus factor), modotuximab (binds EGFR extracellular domain III), mogamulizumab (binds CCR4), monalizumab (binds NKG2A), mosunetuzumab (binds CD3E; MS4A1; CD20), moxetumomab pasudotox (binds CD22), muromonab-CD3 (binds CD3), nacolomab tafenatox (binds C242 antigen), naptumomab estafenatox (binds 5T4), naratuximab emtansine (binds CD37), narnatumab (binds MST1R), natalizumab (Tysabri; binds integrin α₄), naxitamab (binds c-Met), necitumumab (binds EGFR), nemolizumab (binds IL31RA), nimotuzumab (Theracim; Theraloc; binds EGFR), nirsevimab (binds RSVFR), nivolumab (binds PD-1), obinutuzumab (binds CD20), ocaratuzumab (binds CD20), ocrelizumab (binds CD20), odulimomab (binds LFA-1; CD11a), ofatumumab (binds CD20), olaratumab (binds PDGF-R α), omburtamab (binds CD276), onartuzumab (binds human scatter factor receptor kinase), ontuxizumab (binds TEM1), onvatilimab (binds VSIR), opicinumab (binds LINGO-1), oportuzumab monatox (binds EpCAM), oregovomab (binds CA-125), otelixizumab (binds CD3), otlertuzumab (binds CD37), oxelumab (binds OX-40), panitumumab (binds EGFR), pankomab (binds tumor specific glycosylation of MUC1), patitumab (binds ERBB3; HER3), PDR001 (binds PD-1), pembrolizumab (Keytruda; binds PD-1), pemtumomab (Theragyn; binds MUC1), pertuzumab (Omnitarg; binds HER2/neu), pidilizumab (binds PD-1), pinatuzumab vedotin (binds CD22), plozalizumab (binds CCR2), pogalizumab (binds TNFR superfamily member 4), polatuzumab vedotin (binds CD79B), prilizimab (binds CD4), PRO 140 (binds CCR5), ramucirumab (Cyramza; binds VEGFR2), ravagalimab (binds CD40), relatlimab (binds LAG3), rinucumab (binds platelet-derived growth factor receptor beta); rituximab (binds CD20), rituzimab (MabThera; Rituzan; binds CD20), robatumumab (IGF-1 receptor; binds CD221), racotumomab (Vaxira, binds NGNA ganglioside), radretumab (binds fibronectina extra domain-B), refanezumab (binds myelin-associated glycoprotein), roledumab (binds RHD), rovelizumab (LeukArrest; binds CD11; CD18), rozanolixizumab (binds FCGRT), ruplizumab (Antova; binds CD154; CD40L), SA237 (binds IL-6R), sacituzumab govitecan (binds TROP-2), samalizumab (binds CD200), samrotamab vedotin (binds LRRC15), sarilumab (Kevzara; binds IL-6R), satralizumab (binds IL6 receptor), satumomab pendetide (binds TAG-72), seribantumab (binds ERBB3; HER3), setrusumab (binds SOST), SGN-CD19A (binds CD19), SHP647 (binds mucosal addressin cell adhesion molecule), siltuximab (Sylvant; binds soluble IL-6, IL-6R), siplizumab (binds CD2), sirtratumab vedotin (binds SLITRK6), sontuzumab (binds episialin), sofituzumab vedotin (binds CA-125), solitomab (binds EpCAM), spartalizumab (binds PDCD1; CD279), sulesomab (binds NCA-90; granulocyte antigen), suptavumab (binds RSVFR), tabalumab (binds BAFF), tadocizumab (binds integrin α_{IIb}β₃) talacotuzumab (binds CD123), taplitumomab paptox (binds CD19), tarextumab (binds Notch receptor), tavolimab (binds CD134), telisotuzumab vedotin (binds HGFR), teneliximab (binds CD40), tepoditamab (binds dendritic cell-associated lectin 2), teprotumomab (binds IGF-1 receptor; CD221), tetulomab (binds CD37), TGN1412 (binds CD28), tibulizumab (binds BAFF), tigatuzumab (binds TRAIL-R2), timigutuzumab (binds HER2), tiragotumab (binds TIGIT), tislelizumab (binds PCDC1; CD279), tocilizumab (Actemra; RoActemra; binds IL-6 receptor), tomuzotuximab (binds EGFR; HER1), toralizumab (binds CD154; CD40L), tositumomab (Bexxar; binds CD20), tovetumab (binds PDGFRA), trastuzumab (Herceptin; binds HER2/neu); trastuzumab emtansine (Kadcyla; binds HER2/neu); tregalizumab (binds CD4), tremelimumab (binds CTLA4), TRBS07 (binds GD2 ganglioside), tucotuzumab celmoleukin (binds EpCAM), ublituximab (binds MS4A1), ulocuplumab (binds CXCR4; CD184), urelumab (binds 4-1BB; CD137), ustekinumab (Stellera; binds IL-12/23), utomilumab (binds 4-1BB; CD137), vadastuximab talirine (binds CD33), vanalimab (binds CD40), vantictumab (binds Frizzled receptor), varisacumab (binds VEGFR2), varlilumab (binds CD27), vatelizumab (binds ITGA2; CD49b), vedolizumab (Entyvio; binds integrin α₄β₇), veltuzumab (binds CD20), vesencumab (binds NRP1), visilizumab (Nuvion; binds CD3), vobarilizumab (binds IL6R), volociximab (binds integrin α₅β₁), vonlerolizumab (binds CD134), vopratelimab (binds CD278; ICOS), XMAB-5574 (binds CD19), zalutumumab (HuMax-EGFr; binds EGFR), zanolimumab (HuMax-CD4; binds CD4), zatuximab (binds HER1), zenocutuzumab (binds ERBB3; HER3), ziralimumab (binds CD147; basigin), zolbetuximab (binds Claudin 18 Isoform 2), or zolimomab aritox (binds CD5), or an antigen binding portion thereof.

Other antibodies that can be secreted by the cells in the device include, but are not limited to, anetumab (binds mesothelin), aorutumab (binds FGFR2), azintuxizumab (binds SLAMF7), belantamab (binds TNFRSF17), bivatuzumab (binds CD44v6), brentuximab (binds CD30), camidanlumab (binds CD25), cantuzumab (binds CanAg), cantuzumab (binds CanAg), clivatuzumab (binds MUC1), cofetuzumab (binds PTK7), coltuximab (binds CD19), denintuzumab (binds CD19), depatuxizumab (binds EGFR), enapotamab (binds AXL), enfortumab (binds Nectin-4), epratuzumab (binds CD22), gemtuzumab (binds CD33), glembatumumab (binds GPNMB), hertuzumab (binds HER2), iladatuzumab (binds CD79B), indatuximab (binds CD138), industuzumab (binds GCC), inotuzumab (binds CD22), labetuzumab (binds CEA-CAM4), ladiratuzumab (binds LIV-1), laprituximab (binds EGFR), lifastuzumab (binds SLC34A2), loncastuximab (binds CD19), lorvotuzumab (binds CD56), losatuximab (binds EGFR), lupartumab (binds LYPD3), iratumumab (binds CD30), milatuzumab (binds CD74), mirvetuximab (binds PSMA), naratuximab (binds CD37), pinatuzumab (binds CD22), polatuzumab (binds CD79B), rovalpituzumab (binds DLL3), sacituzumab (binds TACSTD2), samrotamab (binds LRRC15), sirtratumab (binds SLTRK6), sofituzumab (binds mucin 16), telisotuzumab (binds c-Met), tisotumab (binds TF), trastuzumab (binds ERBB2), vadastuximab (binds CD33), vandortuzumab (binds STEAP1), or vorsetuzumab (binds CD70), or an antigen binding portion thereof.

In some embodiments, cells in the cell chamber device secrete an antibody, or antigen-binding portion thereof, that specifically binds α4β7. In certain embodiments, the cells secrete vedolizumab, or an antigen-binding portion thereof. In some embodiments, the cells in the device are mammalian host cells engineered to stably express an anti-α4β7 antibody, such as vedolizumab, or a binding molecule comprising antigen binding regions of vedolizumab. Vedolizumab is also known by the trade name ENTYVIO^{®} (Millennium Pharmaceuticals, Inc.). Vedolizumab is a humanized monoclonal antibody that specifically binds to the α4β7 integrin, e.g., the α4β7 complex, and blocks the interaction of α4β7 integrin with mucosal addressin cell adhesion molecule-1 (MAdCAM-1) and fibronectin and inhibits the migration of lymphocytes, e.g., CD4, CD8 or memory T-lymphocytes across the endothelium into inflamed gastrointestinal parenchymal tissue. Vedolizumab does not bind to or inhibit function of the α4β1 and αEβ7 integrins and does not antagonize the interaction of α4 integrins with vascular cell adhesion molecule-1 (VCAM-1).

The α4β7 integrin is expressed on the surface of a discrete subset of memory T-lymphocytes that preferentially migrate into the gastrointestinal tract. MAdCAM-1 is mainly expressed on gut endothelial cells and plays a critical role in the homing of T-lymphocytes to gut lymph tissue. The interaction of the α4β7 integrin with MAdCAM-1 has been implicated as an important contributor to mucosal inflammation, such as the chronic inflammation that is a hallmark of ulcerative colitis and Crohn's disease. Vedolizumab may be used to treat inflammatory bowel disease, including Crohn's disease and ulcerative colitis, pouchitis (e.g., including chronic pouchitis), graft-versus host disease, celiac disease, HIV, primary sclerosing cholangitis, and mucosal inflammation from lymphocyte trafficking, such as after adhesion to α4β7 ligands such as MAdCAM or fibronectin.

In one embodiment, cells in the cell chamber device secrete an antibody, or antigen binding portion thereof, that comprises a heavy chain variable region of SEQ ID NO:1, and/or a light chain variable region of SEQ ID NO:5. In one embodiment, cells in the cell chamber device secrete an antibody, or antigen binding portion thereof, that comprises a heavy chain CDR1 of SEQ ID NO:2, a heavy chain CDR2 of SEQ ID NO:3, and a heavy chain CDR3 of SEQ ID NO:4, and/or a light chain CDR1 of SEQ ID NO:6, a light chain CDR2 of SEQ ID NO:7, and a light chain CDR3 of SEQ ID NO:8. In one embodiment, cells in the cell chamber device secrete an antibody, or antigen binding portion thereof, that comprises a heavy chain comprising the amino acid sequence of SEQ ID NO: 9, and/or a light chain comprising the amino acid sequence of SEQ ID NO: 10. Vedolizumab and the sequences of vedolizumab are also described in U.S. Patent Publication No. 2014/0341885 and U.S. Patent Publication No. 2014-0377251, the entire contents of each which are expressly incorporated herein by reference. The cells in the device herein can be engineered to secrete an antibody comprising binding regions, *e.g.,* CDRs or variable regions, set forth above and in the enclosed sequence table.

In certain embodiments, the cells in the cell chamber device secrete an antibody, or an antigen-binding portion thereof, comprising a heavy chain variable region comprising an amino acid sequence of SEQ ID NO:1, and a light chain variable region comprising an amino acid sequence of SEQ ID NO:5. In some embodiments, the cells in the cell chamber device secrete an antibody, or an antigen-binding portion thereof, comprising a heavy chain variable region comprising a CDR1 of SEQ ID NO:2, a CDR2 of SEQ ID NO:3, and a CDR3 of SEQ ID NO:4 and a light chain variable region comprising a CDR1 of SEQ ID NO:6, a CDR2 of SEQ ID NO:7 and CDR3 of SEQ ID NO:8. In some embodiments, the cells in the cell chamber device secrete an antibody, or an antigen-binding portion thereof, comprising a heavy chain comprising the amino acid sequence of SEQ ID NO: 9, and a light chain comprising the amino acid sequence of SEQ ID NO: 10. In certain embodiments, the cells in the cell chamber device secrete vedolizumab, or an antigen binding portion thereof.

In one embodiment, cells in the cell chamber device secrete an antibody, or antigen binding portion thereof, that specifically binds to tumor necrosis factor alpha (TNFα). In one embodiment, cells in the cell chamber device secrete an antibody, or antigen binding portion thereof, that comprises a heavy chain variable region of SEQ ID NO:22, and/or a light chain variable region of SEQ ID NO:23. In one embodiment, cells in the cell chamber device secrete an antibody, or antigen binding portion thereof, that comprises a heavy chain CDR1 of SEQ ID NO:24, a heavy chain CDR2 of SEQ ID NO:25, and a heavy chain CDR3 of SEQ ID NO:26, and/or a light chain CDR1 of SEQ ID NO:27, a light chain CDR2 of SEQ ID NO:28, and a light chain CDR3 of SEQ ID NO:29. In one embodiment, cells in the cell chamber device secrete an antibody, or antigen binding portion thereof, that comprises a heavy chain comprising the amino acid sequence of SEQ ID NO: 30, and/or a light chain comprising the amino acid sequence of SEQ ID NO: 31. The cells in the device herein can be engineered to secrete an antibody comprising binding regions, *e.g*., CDRs or variable regions, set forth above and in the enclosed sequence table.

In one embodiment, cells in the cell chamber device secrete an antibody, or antigen binding portion thereof, that specifically binds to Interleukin 12 (IL-12). In one embodiment, cells in the cell chamber device secrete an antibody, or antigen binding portion thereof, that comprises a heavy chain variable region of SEQ ID NO:32, and/or a light chain variable region of SEQ ID NO:33. In one embodiment, cells in the cell chamber device secrete an antibody, or antigen binding portion thereof, that comprises a heavy chain CDR1 of SEQ ID NO:34, a heavy chain CDR2 of SEQ ID NO:35, and a heavy chain CDR3 of SEQ ID NO:36, and/or a light chain CDR1 of SEQ ID NO:37, a light chain CDR2 of SEQ ID NO:38, and a light chain CDR3 of SEQ ID NO:39. In one embodiment, cells in the cell chamber device secrete an antibody, or antigen binding portion thereof, that comprises a heavy chain comprising the amino acid sequence of SEQ ID NO: 40, and/or a light chain comprising the amino acid sequence of SEQ ID NO: 41. The cells in the device herein can be engineered to secrete an antibody comprising binding regions, *e.g*., CDRs or variable regions, set forth above and in the enclosed sequence table.

In some embodiments, the cell chamber device comprises a cell comprising one or more nucleic acid molecules encoding an antibody or antigen binding portion thereof, e.g., encoding an antibody or antigen binding portion thereof as set forth above, operably linked to a promoter and/or other elements needed for gene expression. In one embodiment, the cell chamber device comprises a recombinant host cell comprising one or more expression vector(s) comprising one or more nucleic acid(s) that encode an antibody heavy chain, and/or an antibody light chain, or a portion thereof.

For example, in some embodiments, the cell chamber device can comprise a cell that comprises one or more nucleic acid molecules that encode 3F8 (binds GD2 ganglioside), abciximab (ReoPro; binds CD41), abituzumab (binds CD51), alemtuzumab (Lemtrada, Campath; binds CD52), abrilumab (binds integrin α4β7), adalimumab (Humira; binds TNF-α), adecatumumab (binds EpCAM), alacizumab pegol (binds VEGFR2), alemtuzumab (Lemtrada, Campath; binds CD52), altumomab pentetate (Hybri-ceaker; binds CEA), amatuximab (binds mesothelin), anatumomab mafenatox (binds TAG-72), anetumab ravtansine (binds MSLN), anifrolumab (binds interferon α/β receptor), apolizumab (binds HLA-DR), aprutumab ixadotin (binds FGFR2); arcitumomab (binds CEA), aselizumab (binds L-selectin or CD62L), atezolizumab (Tecentriq; binds PD-L1), atorolimumab (binds Rhesus factor), avelumab (Bavencio; binds PD-L1), avicixizumab (binds DLL4; VEGFA), azintuxizumab vedotin (binds CD319); basiliximab (Simulect; binds CD25), bavituximab (binds phosphatidylserine), BCD-100 (binds PD-1), bectummomab (LymphoScan; binds CD22), belantamab mafodotin (binds BCMA); belimumab (Benlysta; binds BAFF), bemarituzumab (binds FGFR2), benralizumab (Fasenra; binds CD125), bersanlimab (binds ICAM-1), besilesomab (Scintimun; binds CEA-related antigen), bimagrumab (binds ACVR2B), bivatuzumab mertansine (binds CD44 v6), bleselumab (binds CD40), blinatumomab (Blincyto; binds CD19), blosozumab (binds SOST); brentuximab vedotin (Adcentris; binds CD30), brontictuzumab (binds Notch 1), brodalumab (Siliq; binds IL-17 receptor), cabiralizumab (binds CSF1R), camidanlumab tesirine (binds CD25), camrelizumab (binds PD-1), carotuximab (binds endoglin), catumaxomab (Removab; binds EpCAM/CD3), cantuzumab ravtansine (binds MUC1), caplacizumab (Cablivi; binds VWF), cedelizumab (binds CD4); cemipilimab (Libtayo; binds PCDC1), cetrelimab (binds PD-1), certolizumab (binds TNF-α), cergutuzumab amunaleukin (binds CEA), cetuximab (Erbitux; binds EGFR), cibisatamab (binds CEACAM5), cirmtuzumab (binds ROR1), cixutumumab (binds IGF-1 receptor; CD221), clenoliximab (binds CD4), clivatuzumab tetraxetan (binds hPAM4-Cide; MUC1), codrituzumab (binds glypican 3), coltuximab ravtansine (binds CD19), conatumumab (binds TRAIL-R2), crizanlizumab (binds selectin P), crotedumab (binds GCGR), dacetuzumab (binds CD40), daclizumab (Zenapax; binds CD25), dalotuzumab (binds IGF-1 receptor; CD221), dapirolizumab pegol (binds CD154; CD40L), daratumumab (Darzalex; binds CD38), demcizumab (binds DLL4), denintuzumab mafodotin (binds CD19), depatuxizumab mafodotin (binds EGFR), drozitumab (binds DR5); DS-8201 (binds HER2), deligotuzumab (binds ERBB3; HER3), dinutuximab (Unituxin; binds GD2 ganglioside), dupilumab (binds IL-4Rα), durvalumab (Imfinzi; binds PD-L1), duvortuxizumab (binds CD19; CD3E), ecromeximab (binds GD3 ganglioside), edrecolomab (binds EpCAM); elezanumab (binds RGMA), elgemtumab (binds ERBB3, HER3); elotuzumab (binds SLAMF7), emactuzumab (binds CSF1R), enapotamab vedotin (binds AXL), enavatuzumab (binds TWEAK receptor), enlimonomab pegol (binds ICAM-1; CD54), enoblituzumab (binds CD276), enoticumab (binds DLL4), epratuzumab (binds CD22), erlizumab (binds ITGB2; CD18), ertumaxomab (Rexomun; binds HER2/neu; CD3), etaracizumab (Abergin; binds integrin αᵥβ₃), etigilimab (binds TIGIT), etrolizumab (binds integrin β₇), exbivirumab (binds hepatitis B surface antigen), fanolesomab (NeutroSpec; binds CD15), faralimomab (binds interferon receptor), farletuzumab (binds folate receptor1), FBTA05 (Lymphomun; binds CD20), fgatipotuzumab (binds MUC1), fibatuzumab (binds ephrin receptor A3), figitumumab (binds IGF-1 receptor; CD221), flotetuzumab (binds IL 3 receptor); foralumab (binds CD3 epsilon); futuximab (binds EGFR), galiximab (binds CD80), gancotamab (binds HER2/neu), ganitumab (binds IGF-1 receptor; CD221), gavilimomab (binds CD147; basigin), gemtuzumab ozogamicin (Mylotarg; binds CD33), glembatumumab vedotin (binds GPNMB), golimumab (Simponi; binds TNF-α), gomiliximab (binds CD23; IgE receptor), ianalumab (binds BAFF-R), ibalizumab (Trogarzo; binds CD4), IBI308 (binds PD-1), ibritumomab tiuxetan (binds CD20), icrucumab (binds VEGFR-1), ifabotuzumab (binds EPHA3), igovomab (Indimacis-125; binds CA-125), IMAB362 (binds CLDN18.2), imaprelimab (binds MCAM), inclacumab (binds selectin P), indatuximab ravtansine (binds SDC1), iladatuzumab vedotin (binds CD97B), imgatuzumab (binds EGFR), indusatumab vedotin (binds GUCY2C), inebilizumab (binds CD19), infliximab (Remicade; binds TNF-α), intetumumab (binds CD51), inolimomab (binds CD25), inotuzumab ozogamicin (Besponsa; binds CD22), ipilimumab (Yervoy; binds CD152), iomab-B (binds CD45), iratumumab (binds CD30), isatuximab (binds CD38), iscalimab (binds CD40), istiratumab (binds IGF1R; CD221), itolizumab (Alzumab; binds CD6), keliximab (binds CD4), laprituximab emtansine (binds EGFR), labetuzumab (binds CEA-Cide; CEA), lifastuzumab vedotin (binds phosphate-sodium co-transporter), lemalesomab (binds NCA-90; granulocyte antigen), lenvervimab (binds hepatitis B surface antigen), leronlimab (binds CCR5), lexatumumab (binds TRAIL-R2), libivirumab (binds hepatitis B surface antigen), loncastuximab tesirine (binds CD19), losatuxizumab vedotin (binds EGFR; ERBB1; HER1), lilotomab satetraxetan (binds CD37), lintuzumab (binds CD33), lirilumab (binds KIR2D), lorvotuzumab mertansine (binds CD56), lucatumumab (binds CD40), lulizumab pegol (binds CD28), lumiliximab (binds CD23; IgE receptor), lumretuzumab (binds ERBB3; HER3), lupartumab amadotin (binds LYPD3), mapatumumab (binds TRAIL-R1), margetuximab (binds HER2), maslimomab (binds T-cell receptor), mavrilimumab (binds GMCSF receptor α-chain), matuzumab (binds EGFR), milatuzumab (binds CD74), minretumomab (binds TAG-72), mirvetuximab soravtansine (binds folate receptor alpha), mitumomab (binds GD3 ganglioside), morolimumab (binds Rhesus factor), modotuximab (binds EGFR extracellular domain III), mogamulizumab (binds CCR4), monalizumab (binds NKG2A), mosunetuzumab (binds CD3E; MS4A1; CD20), moxetumomab pasudotox (binds CD22), muromonab-CD3 (binds CD3), nacolomab tafenatox (binds C242 antigen), naptumomab estafenatox (binds 5T4), naratuximab emtansine (binds CD37), narnatumab (binds MST1R), natalizumab (Tysabri; binds integrin α₄), naxitamab (binds c-Met), necitumumab (binds EGFR), nemolizumab (binds IL31RA), nimotuzumab (Theracim; Theraloc; binds EGFR), nirsevimab (binds RSVFR), nivolumab (binds PD-1), obinutuzumab (binds CD20), ocaratuzumab (binds CD20), ocrelizumab (binds CD20), odulimomab (binds LFA-1; CD11a), ofatumumab (binds CD20), olaratumab (binds PDGF-R α), omburtamab (binds CD276), onartuzumab (binds human scatter factor receptor kinase), ontuxizumab (binds TEM1), onvatilimab (binds VSIR), opicinumab (binds LINGO-1), oportuzumab monatox (binds EpCAM), oregovomab (binds CA-125), otelixizumab (binds CD3), otlertuzumab (binds CD37), oxelumab (binds OX-40), panitumumab (binds EGFR), pankomab (binds tumor specific glycosylation of MUC1), patitumab (binds ERBB3; HER3), PDR001 (binds PD-1), pembrolizumab (Keytruda; binds PD-1), pemtumomab (Theragyn; binds MUC1), pertuzumab (Omnitarg; binds HER2/neu), pidilizumab (binds PD-1), pinatuzumab vedotin (binds CD22), plozalizumab (binds CCR2), pogalizumab (binds TNFR superfamily member 4), polatuzumab vedotin (binds CD79B), prilizimab (binds CD4), PRO 140 (binds CCR5), ramucirumab (Cyramza; binds VEGFR2), ravagalimab (binds CD40), relatlimab (binds LAG3), rinucumab (binds platelet-derived growth factor receptor beta); rituximab (binds CD20), rituzimab (MabThera; Rituzan; binds CD20), robatumumab (IGF-1 receptor; binds CD221), racotumomab (Vaxira, binds NGNA ganglioside), radretumab (binds fibronectina extra domain-B), refanezumab (binds myelin-associated glycoprotein), roledumab (binds RHD), rovelizumab (LeukArrest; binds CD11; CD18), rozanolixizumab (binds FCGRT), ruplizumab (Antova; binds CD154; CD40L), SA237 (binds IL-6R), sacituzumab govitecan (binds TROP-2), samalizumab (binds CD200), samrotamab vedotin (binds LRRC15), sarilumab (Kevzara; binds IL-6R), satralizumab (binds IL6 receptor), satumomab pendetide (binds TAG-72), seribantumab (binds ERBB3; HER3), setrusumab (binds SOST), SGN-CD19A (binds CD19), SHP647 (binds mucosal addressin cell adhesion molecule), siltuximab (Sylvant; binds soluble IL-6, IL-6R), siplizumab (binds CD2), sirtratumab vedotin (binds SLITRK6), sontuzumab (binds episialin), sofituzumab vedotin (binds CA-125), solitomab (binds EpCAM), spartalizumab (binds PDCD1; CD279), sulesomab (binds NCA-90; granulocyte antigen), suptavumab (binds RSVFR), tabalumab (binds BAFF), tadocizumab (binds integrin α_{IIb}β₃) talacotuzumab (binds CD123), taplitumomab paptox (binds CD19), tarextumab (binds Notch receptor), tavolimab (binds CD134), telisotuzumab vedotin (binds HGFR), teneliximab (binds CD40), tepoditamab (binds dendritic cell-associated lectin 2), teprotumomab (binds IGF-1 receptor; CD221), tetulomab (binds CD37), TGN1412 (binds CD28), tibulizumab (binds BAFF), tigatuzumab (binds TRAIL-R2), timigutuzumab (binds HER2), tiragotumab (binds TIGIT), tislelizumab (binds PCDC1; CD279), tocilizumab (Actemra; RoActemra; binds IL-6 receptor), tomuzotuximab (binds EGFR; HER1), toralizumab (binds CD154; CD40L), tositumomab (Bexxar; binds CD20), tovetumab (binds PDGFRA), trastuzumab (Herceptin; binds HER2/neu); trastuzumab emtansine (Kadcyla; binds HER2/neu); tregalizumab (binds CD4), tremelimumab (binds CTLA4), TRBS07 (binds GD2 ganglioside), tucotuzumab celmoleukin (binds EpCAM), ublituximab (binds MS4A1), ulocuplumab (binds CXCR4; CD184), urelumab (binds 4-1BB; CD137), ustekinumab (Stellera; binds IL-12/23), utomilumab (binds 4-1BB; CD137), vadastuximab talirine (binds CD33), vanalimab (binds CD40), vantictumab (binds Frizzled receptor), varisacumab (binds VEGFR2), varlilumab (binds CD27), vatelizumab (binds ITGA2; CD49b), vedolizumab (Entyvio; binds integrin α₄β₇), veltuzumab (binds CD20), vesencumab (binds NRP1), visilizumab (Nuvion; binds CD3), vobarilizumab (binds IL6R), volociximab (binds integrin α₅β₁), vonlerolizumab (binds CD134), vopratelimab (binds CD278; ICOS), XMAB-5574 (binds CD19), zalutumumab (HuMax-EGFr; binds EGFR), zanolimumab (HuMax-CD4; binds CD4), zatuximab (binds HER1), zenocutuzumab (binds ERBB3; HER3), ziralimumab (binds CD147; basigin), zolbetuximab (binds Claudin 18 Isoform 2), or zolimomab aritox (binds CD5), or an antigen binding portion thereof . Preferably, the nucleic acid molecule is operably coupled to a promoter and/or other regulatory elements necessary for expression and secretion of the protein encoded by the nucleic acid by cells present in the chamber.

In some embodiments, the cells in the cell chamber device disclosed herein comprise one or more nucleic acids that encode an antibody, or antigen-binding portion thereof, that specifically binds α4β7. In certain embodiments, the cells comprise one or more nucleic acid that encode vedolizumab, or an antigen binding portion of vedolizumab. A nucleic acid sequence encoding the light chain variable region is set forth in SEQ ID NO:11. A nucleic acid sequence encoding the heavy chain variable region is set forth in SEQ ID NO:12. A full length nucleic acid sequence encoding the light chain of vedolizumab is set forth as SEQ ID NO:13 and a full length nucleic acid sequence encoding the heavy chain of vedolizumab is set forth as SEQ ID NO:14. Alternative nucleic acid sequences encoding vedolizumab are described in U.S. Patent Publication No. 2010/0297699, the entire contents of which are incorporated herein. Accordingly, in certain embodiments, the cells in the cell chamber device can comprise the nucleic acid sequence set forth in SEQ ID NO:11 and/or the nucleic acid sequence set forth in SEQ ID NO:12. In some embodiments, the cells in the cell chamber device comprise a nucleic acid comprising the nucleic acid sequence in SEQ ID NO:13 and the nucleic acid sequence in SEQ ID NO:14.

In some embodiments, the cells in the cell chamber device disclosed herein express one or more nucleic acids that encode an antibody, or antigen-binding portion thereof, that specifically binds α4β7. In certain embodiments, the cells express one or more nucleic acid that encode vedolizumab, or an antigen binding portion of vedolizumab. In certain embodiments, the cells in the cell chamber device express the nucleic acid sequence set forth in SEQ ID NO:11 and/or the nucleic acid sequence set forth in SEQ ID NO:12. In some embodiments, the cells in the cell chamber device express a nucleic acid comprising the nucleic acid sequence in SEQ ID NO:13 and the nucleic acid sequence in SEQ ID NO:14.

In some embodiments, the cells in the cell chamber device express a nucleic acid comprising the nucleic acid sequence of an immature humanized anti- α4β7 immunoglobulin chain containing a signal peptide (e.g., SEQ ID NO:15, SEQ ID NO:16, SEQ ID NO:17, SEQ ID NO:18, SEQ ID NO:19, or SEQ ID NO:20). In other embodiments, the cells in the cell chamber device express a nucleic acid comprising the nucleic acid sequence of a mature humanized anti-α4β7 immunoglobulin chain that does not contain a signal peptide (e.g., nucleotides 77-1429 of SEQ ID NO:15, nucleotides 79-735 of SEQ ID NO:16, nucleotides 76-1428 of SEQ ID NO:17, nucleotides 78-734 of SEQ ID NO:18, nucleotides 58-714 of SEQ ID NO:19, or nucleotides 58-1410 of SEQ ID NO:20).

In one embodiment, the cells in the cell chamber device comprise a recombinant expression vector, wherein the recombinant expression vector comprises a first nucleic acid that encodes an immunoglobulin heavy chain and a second nucleic acid that encodes an immunoglobulin light chain, wherein the first nucleic acid comprises nucleotides 77-1429 of SEQ ID NO:15, and the second nucleic acid comprises nucleotides 79-735 of SEQ ID NO:16.

In one embodiment, the cells in the cell chamber device comprise a recombinant expression vector, wherein the recombinant expression vector comprises a first nucleic acid that encodes an immunoglobulin heavy chain and a second nucleic acid that encodes an immunoglobulin light chain, wherein the first nucleic acid comprises nucleotides 76-1428 of SEQ ID NO:17, and the second nucleic acid comprises nucleotides 78-734 of SEQ ID NO:18.

In one embodiment, the cells in the cell chamber device comprise a recombinant expression vector, wherein the recombinant expression vector comprises a first nucleic acid that encodes an immunoglobulin heavy chain and a second nucleic acid that encodes an immunoglobulin light chain, wherein the first nucleic acid comprises nucleotides 58-1410 of SEQ ID NO:20, and the second nucleic acid comprises nucleotides 58-714 of SEQ ID NO:19.

Additional examples of nucleic acid sequences that can be expressed by cells in the cell chamber device are also described in WO 2008/115504, the entire contents of which are incorporated by reference herein.

In exemplary embodiments, the cell chamber device comprises ARPE-19 cells that secrete an antibody, or an antigen-binding portion thereof, comprising a heavy chain variable region comprising an amino acid sequence of SEQ ID NO:1, and a light chain variable region comprising an amino acid sequence of SEQ ID NO:5. In some embodiments, the cell chamber device comprises ARPE-19 cells that secrete an antibody, or an antigen-binding portion thereof, comprising a heavy chain variable region comprising a CDR1 of SEQ ID NO:2, a CDR2 of SEQ ID NO:3, and a CDR3 of SEQ ID NO:4 and a light chain variable region comprising a CDR1 of SEQ ID NO:6, a CDR2 of SEQ ID NO:7 and CDR3 of SEQ ID NO:8. In some embodiments, the cell chamber device comprises ARPE-19 cells that secrete an antibody, or an antigen-binding portion thereof, comprising a heavy chain comprising the amino acid sequence of SEQ ID NO: 9, and a light chain comprising the amino acid sequence of SEQ ID NO: 10. In some embodiments, the cell chamber device comprises ARPE-19 cells that comprise a nucleic acid comprising the nucleic acid sequence in SEQ ID NO:11 and the nucleic acid sequence in SEQ ID NO:12. In some embodiments, the cell chamber device comprises ARPE-19 cells that comprise a nucleic acid comprising the nucleic acid sequence in SEQ ID NO:13 and the nucleic acid sequence in SEQ ID NO:14. In certain embodiments, the cell chamber device comprises ARPE-19 cells that secrete vedolizumab.

In some embodiments, the cells in the cell chamber device disclosed herein express one or more nucleic acids that encode an antibody, or antigen-binding portion thereof, that specifically binds TNFα. In certain embodiments, the cells in the cell chamber device express the nucleic acid sequence set forth in SEQ ID NO:42 and/or the nucleic acid sequence set forth in SEQ ID NO:43. In some embodiments, the cells in the cell chamber device express a nucleic acid comprising the nucleic acid sequence in SEQ ID NO:44 and/or the nucleic acid sequence in SEQ ID NO:45. In one embodiment, the cells in the cell chamber device comprise a recombinant expression vector, wherein the recombinant expression vector comprises a first nucleic acid that encodes an immunoglobulin heavy chain and a second nucleic acid that encodes an immunoglobulin light chain, wherein the first nucleic acid comprises SEQ ID NO:44, and the second nucleic acid comprises SEQ ID NO:45.

In some embodiments, the cells in the cell chamber device disclosed herein express one or more nucleic acids that encode an antibody, or antigen-binding portion thereof, that specifically binds IL-12. In certain embodiments, the cells in the cell chamber device express the nucleic acid sequence set forth in SEQ ID NO:46 and/or the nucleic acid sequence set forth in SEQ ID NO:47. In some embodiments, the cells in the cell chamber device express a nucleic acid comprising the nucleic acid sequence in SEQ ID NO:48 and/or the nucleic acid sequence in SEQ ID NO:49. In one embodiment, the cells in the cell chamber device comprise a recombinant expression vector, wherein the recombinant expression vector comprises a first nucleic acid that encodes an immunoglobulin heavy chain and a second nucleic acid that encodes an immunoglobulin light chain, wherein the first nucleic acid comprises SEQ ID NO:48, and the second nucleic acid comprises SEQ ID NO:49.

In certain embodiments, cells in the cell chamber device can secrete a peptide therapeutic for gastrointestinal use, such as for the treatment of short bowel syndrome. Examples of peptide therapeutics useful for treating gastrointestinal disorders are also described in US 9,125,882 (e.g., SEQ ID NO. 54); US 9,742,455 (e.g., SEQ ID NO:1); and US 7,737,251 (e.g., SEQ ID NO: 8). Each of the foregoing patents and patent applications is incorporated by reference herein in its entirety.

The amino acid sequence of an exemplary peptide therapeutic for gastrointestinal use is provided herein as SEQ ID NO:21. In certain embodiments, the cells in the cell chamber secrete a peptide having the amino acid sequence of SEQ ID NO:21. In some embodiments, the cells in the cell chamber device disclosed herein comprise a nucleic acid that encodes a peptide having the amino acid sequence of SEQ ID NO:21.

In certain embodiments, the cell chamber device comprises ARPE-19 cells that secrete a peptide having the amino acid sequence of SEQ ID NO:21. In some embodiments, the cell chamber device comprises ARPE-19 cells that comprise a nucleic acid that encodes a peptide having the amino acid sequence of SEQ ID NO:21.

Cells can be engineered to produce a biomolecule, such as one described herein, by inserting an expression construct encoding the biomolecule into the cells using standard techniques. For example, a vector comprising a polynucleotide encoding a polypeptide of interest can be inserted into a cell, to produce a cell line that produces the polypeptide of interest. The term "vector," as used herein, is intended to refer to a vehicle, e.g., a nucleic acid molecule, capable of transporting genetic material into a cell, where it can then be replicated and/or integrated into the cellular genome, and expressed. One type of vector is a plasmid, which refers to a circular double stranded DNA into which additional DNA segments may be ligated. Other common vectors include phage vectors and viral vectors. Certain vectors are capable of autonomous replication in a host cell into which they are introduced (e.g., episomal mammalian vectors). Other vectors (e.g., non-episomal mammalian vectors) can be integrated into the genome of a host cell upon introduction into the host cell, and thereby are replicated along with the host genome. Moreover, certain vectors are capable of directing the expression of genes to which they are operatively linked. Such vectors are referred to herein as "recombinant expression vectors," or simply, "expression vectors" or "expression constructs." Various vectors suitable for recombinant expression of therapeutic proteins, e.g., therapeutic antibodies, are publicly available and well known to those in the art. Vector components generally include, but are not limited to, one or more of the following: a signal sequence, an origin of replication, one or more marker genes, an enhancer element, a promoter, and a transcription termination sequence. Optional signal sequences, origins of replication, marker genes, enhancer elements and transcription terminator sequences that may be employed are known in the art and described in further detail in, for example, US Patent No. 7,053,202. In certain instances, the promoter element guiding expression of the biomolecule is a constitutive promoter element to ensure long-term expression of the biomolecule in the cells of the device. Expression of the biomolecule can also be increased by a variety of other methods known in the art, such as by increasing the copy number of the nucleic acid expressing the biomolecule, removing premature termination or splicing signals in the nucleic acid expressing the biomolecule, or a selecting a promoter of site of genome integration that enables increased expression of the biomolecule. Another example of increasing expression level is targeted integration to transcriptional hot-spots, e.g., by homologous recombination, determined either empirically by genome sequencing or computationally by expression probability algorithms.

### IV. Three Dimensional Cell Culture

Also provided herein are cell chamber devices comprising nanofibrous (e.g., electrospun) polymers that can accommodate a three dimensional cell culture. Accordingly, in some embodiments, the device can comprise a tissue or portion thereof (e.g., a tissue explant), or a cluster of cells having a three dimensional architecture (e.g., an organoid or spheroid). Cell chamber devices suitable for three dimensional cell cultures may be useful, for example, in methods of treatment where secretion of a biomolecule endogenously produced by the cells or tissue is desired. The cells or tissues may be grown in three-dimensional cell culture (i.e., 3D cell culture) according to methods known in the art (see, e.g., Edmondson, et al. (2014). Assay and drug development technologies, 12(4), 207-218, which is hereby incorporated by reference), and then added to the cell chamber device. Cells or tissue with a three dimensional architecture can also be obtained, for example, from tissue explants, biopsies, or harvested from a living donor, cell culture, or autopsy, using art-recognized techniques.

**In** some embodiments, the cell chamber device comprises a three dimensional cell culture comprising a tissue, or a portion thereof. Examples of tissues that can be included in the devices herein include liver tissue, pancreatic tissue, intestinal tissue, or kidney tissue. The tissue can optionally be a tissue explant (e.g., a piece or pieces of a tissue or organ removed from an animal). In one embodiment, the cell chamber comprises liver tissue (e.g., a liver tissue explant). In one embodiment, the cell chamber comprises pancreas tissue (e.g., a pancreatic tissue explant, such as pancreatic islet tissue explant). In one embodiment, the cell chamber comprises kidney tissue (e.g., a kidney tissue explant). In some embodiments, the cell chamber comprises a reproductive tissue (e.g., an ovarian or testicular tissue). In some embodiments, the tissue is a human tissue, such as a human kidney tissue explant, a human liver tissue explant, or a human pancreatic tissue. In other embodiments, the tissue is a human reproductive tissue, such as a human ovarian or human testicular explant.

The cell chamber device may alternatively comprise a cluster of cells having a three dimensional architecture. For example, in some embodiments, the three-dimensional cell culture can comprise organoids or spheroids. In some embodiments, the cluster of cells (e.g., organoids or spheroids) comprises hepatocytes (e.g., human hepatocytes), kidney cells, or pancreatic islet cells. The cluster of cells (e.g., organoids or spheroids) may be organized around a structure to maintain the three dimensional architecture of the cell culture. For example, in some embodiments, the cell chamber comprises organized groups of cells organized around a structure, such as a duct or a sinusoid.

The cell chamber device for use with three dimensional cell culture may comprise a multilayer scaffold comprising nanofibrous, e.g., electrospun, polymers, as described herein (see, e.g., Section II). Optionally, the scaffold layers may be tuned to promote vascularization toward tissues or cells within the chamber.

In alternative embodiments, the cell chamber device can comprise a single nanofibrous, e.g., electrospun, polymer layer. The single-layer scaffold can be formed from a variety of polymers (e.g., nanofibrous polymers), such as polyester, polyethylene terephthalate (PET, also known as Dacron), polybutylene terephthalate (PBT), or polyurethane (PU). For example, in some embodiments, the single-layer scaffold can comprise nanofibrous polyester, nanofibrous polyethylene terephthalate (nPET), nanofibrous polybutylene terephthalate (nPBT), and/or nanofibrous polyurethane (nPU). In one embodiment, the single-layer scaffold comprises nPET. In another embodiment, single-layer scaffold comprises nPBT. In one embodiment, the single-layer scaffold comprises nPET-nPBT. In a further embodiment, the single-layer scaffold comprises nPU. A single-layer cell chamber device can be fabricated essentially as described with respect to the multi-layer device provided herein, using polymer sheets that comprise a single layer, for example, a homogenous layer, of nanofibrous polymer. The single-layer device can, in some embodiments, permit greater contact between cells in the chamber and the environment outside the chamber, relative to a multi-layer scaffold device.

In some embodiments, the nanofibrous polymer scaffold can be loaded with an anti-inflammatory agent. For example, the nanofibrous polymer scaffold can be loaded with tacrolimus, pirfenidone, and/or roflumilast. Without wishing to be bound by theory, inclusion of an anti-inflammatory agent may aid in the preservation of three-dimensional architecture of cells growing inside the chamber, by discouraging infiltration of cells (e.g., immune or inflammatory cells) from outside the chamber. Anti-inflammatory agents can also reduce the immune response of a subject to the device following implantation. In some embodiments, the cell chamber device comprises a single layer scaffold comprising a nanofibrous polymer, wherein the scaffold comprises an anti-inflammatory agent (e.g. tacrolimus, pirfenidone, and/or roflumilast). In other embodiments, the cell chamber device comprises a multi-layer scaffold comprising a nanofibrous polymer, wherein the scaffold comprises an anti-inflammatory agent (e.g. tacrolimus, pirfenidone, and/or roflumilast).

Tissue explants or cells having a three dimensional architecture can optionally secrete a biomolecule. In some embodiments, the biomolecule is a biomolecule endogenously produced by the tissue or cells. In alternative embodiments, the cells loaded into the cell chamber device of the present disclosure may be genetically modified cells, e.g., recombinant cells, that have been engineered to produce, e.g., secrete, a biomolecule of interest. Biomolecules that can be secreted by the cells of the device include, for example and without limitation, polypeptides, polysaccharides, and polynucleotides, as well as organic molecules such as lipids (e.g., phospholipids, glycolipids and sterols), chemical messengers (e.g., neurotransmitters and hormones, such as insulin), vitamins, sugars (e.g., carbohydrate, disaccharide, oligosaccharides, polysaccharides), amino acids, peptides, oligopeptides, polypeptides, proteins, nucleotides, deoxyribonucleic acid (DNA), or ribonucleic acid (RNA). Other secreted biomolecules may include those associated, packaged, and secreted as exosomes, lipid polymers, or viral particles. In some embodiments, the tissue explants or cells secrete a therapeutic biomolecule, as described herein.

In some embodiments, the tissue or cells (e.g., cells having a three dimensional structure) within the device secrete one or more proteins or peptides, e.g., one or more therapeutic proteins or peptides. For example, the tissue or cells in the device can secrete one or more therapeutic proteins such as an antibody or antigen-binding fragment thereof, a growth factor, a hormone (e.g., insulin), a cytokine, a clotting factor (e.g., Factor VIII, or Factor IX, or variants thereof, e.g., Recombinate, Kogenate, Refacto, Advate, Alprolix, BeneFIX, Rixubis, Ixinity, Idelvion, etc.), or a combination thereof.

The protein secreted by the tissues or cells in the device can be, in some instances, a protein endogenously produced by the tissue or cells. For example, in one embodiment, the cell chamber device comprises pancreatic tissue or pancreatic cells having a three dimensional architecture (e.g., pancreatic islets), wherein the pancreatic tissue or cells secrete insulin, amylin, glucagon, somatostatin ghrelin, and/or other metabolism related enzymes. In certain embodiments, the cell chamber comprises pancreatic tissue or cells having a three dimensional architecture (e.g., pancreatic islet cells), wherein the pancreatic tissue or cells secrete insulin. In certain embodiments, the cell chamber comprises reproductive tissue (e.g., ovarian tissue or testicular tissue), or cells derived therefrom, wherein the reproductive tissue or cells derived therefrom secretes a hormone, or an agent capable of hormonal regulation in a subject to whom the device is administered.

In other embodiments, the cell chamber device can comprise cells that perform functions such as detoxification or metabolism.

In another embodiment, the cell chamber device comprises liver tissue or hepatocytes having a three dimensional architecture, wherein the liver tissue or hepatocytes secrete albumin, transferrin, plasminogen, fibrinogen, α-fetoprotein, and/or a clotting factor. In another embodiment, the liver tissue or hepatocytes can secrete digestive enzymes and products such as bile, alanine transaminase (ALT), aspartate transaminase (AST), alkaline phosphatase (ALP), and/or gamma-glutamyl transpeptidase (GGT).

In another embodiment, the cell chamber device comprises kidney tissue or kidney cells having a three dimensional architecture, wherein the kidney tissue or cells secrete erythropoietin, calcitriol, prostaglandins, and/or renin.

In some embodiments, tissues or cells in the device can secrete one protein or peptide, e.g., one endogenous or recombinant protein or peptide. In other embodiments, the cells in the device can secrete two or more proteins, e.g., 2, 3, 4, 5, 6, 7, 8, 9, 10 or more endogenous or recombinant proteins. The device can, in some embodiments, be loaded with a single tissue or cluster of cells having a three dimensional architecture, e.g., a single tissue or cluster of cells, that secretes multiple proteins or peptides.

### V. Administration and Methods of Treatment

Also provided herein is a method of delivering a biomolecule to a subject, comprising administering to the subject a cell chamber device disclosed herein, wherein the cell chamber of the device comprises cells secreting a biomolecule. The biomolecule may be, for example, a recombinant peptide or a recombinant protein, including those described above. In certain instances, the method can be used to deliver to a subject an antibody, such as an anti-α4β7 antibody (e.g., vedolizumab), or antigen-binding portion thereof. The dosage of the biomolecule delivered by a cell chamber device comprising biomolecule-producing cells may be controlled by varying the dimensions (length, diameter, volume) of the cell chamber device, adjusting the number of cells in the device by geometry, adjusting the level of expression of the biomolecule by the cells (e.g., by alterations in copy number, selection of promoter, etc.) and/or by adjusting the number of devices delivered to a subject (e.g., between 1 and 10 devices per patient). Accordingly, a subject may be administered one or more of the cell chamber devices described herein. In some embodiments, a subject is administered 1 cell chamber device. In other embodiments, a subject is administered 2, 3, 4, 5, 6, 7, 8, 9, 10 or more cell chamber devices. Multiple devices can be administered at the same (or approximately the same) site, or can be administered to multiple sites in the body.

Prior to administering the device, the device is loaded with cells that secrete a biomolecule to be delivered to the subject. The cells can be loaded, for example, by injecting cells in the cell chamber by way of an opening or loading port in the scaffold surrounding the chamber. The device can be loaded with cells prior to administration. Alternatively, the device may be provided pre-loaded with cells. The number of cells in the device administered to a subject can vary depending on the dimensions of the device, the amount of biomolecule secreted by the cells, and the desired dosage of the biomolecule to be delivered into a recipient subject (e.g., as measured by mg of biomolecule secreted from the device per day, or by the desired concentration of the biomolecule in the serum or plasma of the recipient subject). The desired number of cells in the cell chamber device can be readily determined in accordance with methods known in the art and outlined herein (see, e.g., Example 1). For example, the amount of a biomolecule secreted per cell per day can be measured using standard cell count and biomolecule quantification assays (e.g., western blot or ELISA). Based on this determination, the size of the cell chamber can be adjusted to alter the number of cells in the cell chamber device (i.e., the number of cells in the device following growth and saturation) and accordingly, the dosage of the biomolecule secreted per day from the device. As cells in the chamber can proliferate until the device reaches capacity, the number of cells seeded into the device may be less than the number of cells in the device following implantation.

In some embodiments, the device is loaded with, and can therefore comprise, about 1x10⁴ cells to about 1x10¹² cells (e.g., about 1x10⁴ cells to about 1x10⁵ cells, about 1x10⁵ cells to about 1x10⁶ cells, about 1x10⁶ cells to about 1x10⁷ cells, about 1x10⁷ cells to about 1x10⁹ cells, or about 1x10⁹ cells to about 1x10¹² cells) prior to implantation. In some embodiments, the device comprises about 1x10⁶ cells to about 1x10⁷ cells (e.g., about 1x10⁶ cells to about 1x10⁷ cells, about 2x10⁶ cells to about 1x10⁷ cells, about 3x10⁶ cells to about 1x10⁷ cells, about 4x10⁶ cells to about 1x10⁷ cells, about 5x10⁶ cells to about 1x10⁷ cells, about 6x10⁶ cells to about 9x10⁶ cells, about 7x10⁶ cells to about 9x10⁶ cells, or about 8x10⁶ cells to about 9x10⁶ cells). For example, in certain embodiments, the device comprises about 8.5 x 10⁶ cells. In some embodiments, the device comprises about 1x10⁷ cells to about 1x10⁸ cells. In some embodiments, the device comprises about 1x10⁶ cells to about 1x10⁹ cells. In some embodiments, the device comprises about 1x10⁹ cells to about 1x10¹⁰ cells. In some embodiments, the device comprises about 1x10¹⁰ cells to about 1x10¹¹ cells. In some embodiments, the device comprises about 1x10¹¹ cells to about 1x10¹² cells. In exemplary embodiments, the device comprises about 1x10⁴ cells, 1x10⁵ cells, 1x10⁶ cells, 1x10⁷ cells, 1x10⁸ cells, 1x10⁹ cells, 1x10¹⁰ cells, 1x10¹¹ cells, or 1x10¹² cells. In some embodiments, the device is loaded with about 1x10⁴ cells, 1x10⁵ cells, 1x10⁶ cells, 1x10⁷ cells, 1x10⁸ cells, 1x10⁹ cells, 1x10¹⁰ cells, 1x10¹¹ cells, or 1x10¹² cells prior to implantation in a subject.

In some embodiments, the device comprises about 1x10⁴ cells/cm² to about 1 x10⁶ cells/cm², 1x10⁵ cells/cm² to about 1 x10⁶ cells/cm², about 1x10⁵ cells/cm² to about 9 x10⁵ cells/cm², about 2x10⁵ cells/cm² to about 8 x10⁵ cells/cm², about 3x10⁵ cells/cm² to about 7 x10⁵ cells/cm², about 5 x10⁵ cells/cm² to about 7 x10⁵ cells/cm², about 6 x10⁵ cells/cm² to about 7x10⁵ cells/cm², about 6 x10⁵ cells/cm² to about 6.5x10⁵ cells/cm², or about 6.25 x10⁵ cells/cm².

In other embodiments the device has a capacity to accommodate about 1x10⁴ cells to about 1x10¹² cells (e.g., about 1x10⁴ cells to about 1x10⁵ cells, about 1x10⁵ cells to about 1x10⁶ cells, about 1x10⁶ cells to about 1x10⁷ cells, about 1x10⁷ cells to about 1x10⁹ cells, or about 1x10⁹ cells to about 1x10¹² cells) after implantation in a subject (e.g., after a period of time sufficient for cells in the chamber to proliferate). In some embodiments, the device can accommodate about 1x10⁶ cells to about 1x10⁷ cells (e.g., about 1x10⁶ cells to about 1x10⁷ cells, about 2x10⁶ cells to about 1x10⁷ cells, about 3x10⁶ cells to about 1x10⁷ cells, about 4x10⁶ cells to about 1x10⁷ cells, about 5x10⁶ cells to about 1x10⁷ cells, about 6x10⁶ cells to about 9x10⁶ cells, about 7x10⁶ cells to about 9x10⁶ cells, or about 8x10⁶ cells to about 9x10⁶ cells). For example, in certain embodiments, the device can accommodate about 8.5 x 10⁶ cells. In some embodiments, the device can accommodate about 1x10⁷ cells to about 1x10⁸ cells. In some embodiments, the device can accommodate about 1x10⁸ cells to about 1x10⁹ cells. In some embodiments, the device can accommodate about 1x10⁹ cells to about 1x10¹⁰ cells. In some embodiments, the device can accommodate about 1x10¹⁰ cells to about 1x10¹¹ cells. In some embodiments, the device can accommodate about 1x10¹¹ cells to about 1x10¹² cells. In exemplary embodiments, the device can accommodate about 1x10⁴ cells, 1x10⁵ cells, 1x10⁶ cells, 1x10⁷ cells, 1x10⁸ cells, 1x10⁹ cells, 1x10¹⁰ cells, 1x10¹¹ cells, or 1x10¹² cells. In some embodiments, the device is loaded with about 1x10⁴ cells, 1x10⁵ cells, 1x10⁶ cells, 1x10⁷ cells, 1x10⁸ cells, 1x10⁹ cells, 1x10¹⁰ cells, 1x10¹¹ cells, or 1x10¹² cells following implantation in a subject.

The implantation device can be administered to a subject by, for example, through a surgical incision at the site of implantation in the subject. The site of implantation can vary depending on configuration of the device, the disorder to be treated, and desired biodistribution of the biomolecule secreted by cells in the device. In some embodiments, the device is administered to the subject by implantation at a site selected from under the skin (subcutaneous implantation), on the omentum, or in or adjacent to the liver. In some embodiments, the cell chamber device is implanted on or under the skin; a mucosal surface, a body cavity, the peritoneal cavity; the central nervous system, e.g., the brain, cortex, ventricles, or spinal cord; an organ, e.g., the heart, liver, kidney, spleen, lung, pancreas, lymphatic system, vasculature, the oral cavity, the nasal cavity, the teeth, the gums, the GI tract; bone; hip; fat tissue; muscle tissue; circulating blood; the eye (e.g., intraocular); breast, vagina; uterus, a joint, e.g., the knee or hip joint, or the spine. In some embodiments, is cell chamber device is implanted at a certain part or tissue of the body, e.g., blood, eye, brain, skin, lung, stomach, mouth, ear, leg, foot, hand, liver, heart, kidney, bone, a reproductive organ, testis, pancreas, spleen, large intestine, small intestine, spinal cord, or muscle. In some embodiments, the cell chamber device is administered so as to deliver biomolecules to a certain system of the body, e.g. the vascular system, nervous system (e.g., peripheral nervous system (PNS) or central nervous system, (CNS)), skeletal system, respiratory system, endocrine system, lymph system, reproductive system, or gastrointestinal tract. In some embodiments, the cell chamber device is implanted under the skin of the subject, on the omentum of a subject, into the subcutaneous fat of a subject, or into or adjacent to the muscle tissue of a subject. In exemplary embodiments, the device can be implanted subcutaneously.

In some embodiments, the cell chamber is implanted into the peritoneal cavity (e.g., the omentum). In some embodiments, the cell chamber is implanted into or onto the lesser sac (also known as the omental bursa or bursalis omentum) the greater omentum, lesser omentum, stomach, small intestine, large intestine, liver, spleen, gastrosplenic ligament, adrenal glands, or pancreas.

The cell chamber device can, in some instances, be implanted so as to be easily retrievable from a subject, e.g., without causing injury to the subject or without causing significant disruption of the surrounding tissue. In one embodiment, the device can be retrieved with minimal or no surgical separation of the device from surrounding tissue, e.g., via minimally invasive surgical approach, extraction, or resection.

The length of time that the implant is maintained in the subject will vary depending on the disease being treated and the therapeutic dosage of the biomolecule secreted by the cells in the device. In some embodiments, the cell chamber device is maintained in the subject for at least 2 days, 3 days, 4 days, 5 days, 6 days, 7 days, 1 week, 2 weeks, 3 weeks, 4 weeks, 5 weeks, 10 weeks, 12 weeks, 1 month, 2 months, 3 months, 4 months, 5 months, 6 months, 7 months, 8 months, 9 months, 10 months, 11 months, 12 months, 13 months, 14 months, 15 months, 16 months, 17 months, 18 months, 19 months, 20 months, 21 months, 22 months, 23 months, 24 months, 1 year, 1.5 years, 2 years, 2.5 years, 3 years, 3.5 years, or 4 years, 4.5 years, 5 years, 6 years, 7 years, 8 years, 9 years, 10 years, 11 years, 12 years, 13 years, 14 years, 15 years or more. In some embodiments, the cell chamber device can be maintained in the subject for an average duration of about 1 day to 2 days, 1 day to 7 days, 2 days to 4 days, 3 days to 5 days, 4 days to 1 week, 1 week to 2 weeks, 1 week to 4 weeks, 2 weeks to 4 weeks, 2 weeks to 6 weeks, 3 weeks to 5 weeks, 4 weeks to 1 month, 1 month to 2 months, 1 month to 4 months, 1 month to 6 months, 2 months to 4 months, 3 months to 5 months, 4 months to 6 months, 5 months to 7 months, 6 months to 8 months, 6 months to 12 months, 7 months to 9 months, 8 months to 10 months, 9 months to 11 months, 10 months to 12 months, 11 months to 13 months, 12 months to 15 months, 15 months to 18 months, 18 months to 21 months, 21 months to 24 months, 2 years to 2.5 years, 2.5 years to 3 years, 3 years to 3.5 years, 3.5 years to 4 years, 4 years to 5 years, 5 years to 7 years, 7 years to 9 years, 9 years to 11 years, 11 years to 13 years, or 13-15 years following implantation. In certain embodiments, the device is maintained in the subject for at least 30 days. In certain embodiments, the device is implanted in the subject for at least 90 days. In certain embodiments, the device is implanted in the subject for at least 120 days. In certain embodiments, the device is implanted in the subject for at least 1 year. In certain embodiments, the device is implanted in the subject for at least 2 years. In certain embodiments, the device is implanted in the subject for at least 3 years. In certain embodiments, the device is implanted in the subject for at least 5 years. In certain embodiments, the device is implanted in the subject for at least 7 years. In certain embodiments, the device is implanted in the subject for at least 10 years. In certain embodiments, the device is implanted in the subject for at least 12 years. In certain embodiments, the device is implanted in the subject for at least 15 years. In some embodiments, the device is implanted in the subject permanently.

In some embodiments, the cell chamber device is maintained in the subject such that the daily dose of the biomolecule (e.g., recombinant peptide or recombinant protein) secreted by the cells is at least 1 mg/day (e.g., at least about 1 mg/day, at least about 2 mg/day, at least about 3 mg/day, at least about 4 mg/day, at least about 4.5 mg/day, at least about 5 mg/day, at least about 6 mg/day, at least about 7 mg/day, at least about 8 mg/day, at least about 9 mg/day, or more than 9 mg/day) for a period effective to achieve therapeutic benefits in the subject (e.g., to achieve a therapeutically effective plasma concentration of the biomolecule in the subject). In some embodiments, the cell chamber device is maintained in the subject such that the daily dose of the biomolecule (e.g., recombinant peptide or recombinant protein) secreted by the cells is 0.5 mg/day to 1 mg/day, 1 mg/day to 2 mg/day, 1mg/day to 5 mg/day, 2 mg/day to 5 mg/day, 4 mg/day to 7 mg/day, 5 mg/day to 8 mg/day, 5 mg/day to 10 mg/day, 6 mg/day to 9 mg/day, 7mg/day to 10 mg/day, 10 mg/day to 15 mg/day, 8 mg/day to 11 mg/day, 6mg/day to 12 mg/day, or 9 mg/day to 12 mg/day for a period effective to achieve therapeutic benefits in the subject (e.g., to achieve a therapeutically effective plasma concentration of the biomolecule in the subject).

For example, in some embodiments, the cell chamber device is maintained in the subject such that the plasma concentration of the biomolecule (e.g., recombinant peptide or recombinant protein) is at least 5 µg/mL (e.g., at least about 5 µg/mL, at least about 10 µg/mL, at least about 15 µg/mL, at least about 17 µg/mL, at least about 20 µg/mL, or more than 20 µg/mL) in the subject for a period following implantation, e.g., for a period effective to achieve therapeutic benefits in the subject, e.g., for at least five days, for at least 10 days, at least 20 days, at least 30 days, at least 40 days, at least 50 days, at least 60 days, at least 3 months, at least 4 months, at least 5 months, at least 6 months, at least 12 months, 1 day to 2 days, 1 day to 7 days, 2 days to 4 days, 3 days to 5 days, 4 days to 1 week, 1 week to 2 weeks, 1 week to 4 weeks, 2 weeks to 4 weeks, 2 weeks to 6 weeks, 3 weeks to 5 weeks, 4 weeks to 1 month, 1 month to 2 months, 1 month to 4 months, 1 month to 6 months, 2 months to 4 months, 3 months to 5 months, 4 months to 6 months, 5 months to 7 months, 6 months to 8 months, 6 months to 12 months, 7 months to 9 months, 8 months to 10 months, 9 months to 11 months, 10 months to 12 months, 11 months to 13 months, 12 months to 15 months, 15 months to 18 months, 18 months to 21 months, 21 months to 24 months, 2 years to 2.5 years, 2.5 years to 3 years, 3 years to 3.5 years, or 3.5 years to 4 years following implantation.

In some embodiments, the cell chamber device is maintained in the subject such that the plasma concentration of the biomolecule (e.g., recombinant peptide or recombinant protein) is 4 µg/mL to 8 µg/mL, 5 µg/mL to 9 µg/mL, 5 µg/mL to 12 µg/mL, 6 µg/mL to 10 µg/mL, 7 µg/mL to 11 µg/mL, 7 µg/mL to 15 µg/mL, 8 µg/mL to 12 µg/mL, 9 µg/mL to 13 µg/mL, 10 µg/mL to 15 µg/mL, 15 µg/mL to 20 µg/mL, or 20 µg/mL to 25 µg/mL in the subject for a period following implantation, e.g., for a period effective to achieve therapeutic benefits in the subject, e.g., for 1 day to 2 days, 1 day to 7 days, 2 days to 4 days, 3 days to 5 days, 4 days to 1 week, 1 week to 2 weeks, 1 week to 4 weeks, 2 weeks to 4 weeks, 2 weeks to 6 weeks, 3 weeks to 5 weeks, 4 weeks to 1 month, 1 month to 2 months, 1 month to 4 months, 1 month to 6 months, 2 months to 4 months, 3 months to 5 months, 4 months to 6 months, 5 months to 7 months, 6 months to 8 months, 6 months to 12 months, 7 months to 9 months, 8 months to 10 months, 9 months to 11 months, 10 months to 12 months, 11 months to 13 months, 12 months to 15 months, 15 months to 18 months, 18 months to 21 months, 21 months to 24 months, 2 years to 2.5 years, 2.5 years to 3 years, 3 years to 3.5 years, or 3.5 years to 4 years following implantation.

In some embodiments, the cell chamber device is maintained in the subject such that the plasma concentration of the biomolecule (e.g., recombinant peptide or recombinant protein) is at 1 µg/mL to 5 µg/mL, 1 µg/mL to 10 µg/mL, 5 µg/mL to 10 µg/mL, 5 µg/mL to 15 µg/mL, 10 µg/mL to 15 µg/mL, 10 µg/mL to 20 µg/mL, 15 µg/mL to 20 µg/mL, 15 µg/mL to 30 µg/mL, 20 µg/mL to 25 µg/mL, 20 µg/mL to 40 µg/mL, 25 µg/mL to 30 µg/mL, 25 µg/mL to 50 µg/mL, 30 µg/mL to 35 µg/mL, 30 µg/mL to 60 µg/mL, or 35 µg/mL to 40 µg/mL in the subject for a period following implantation, e.g., for at least five days, for at least 10 days, at least 20 days, at least 30 days, at least 40 days, at least 50 days, at least 60 days, at least 3 months, at least 4 months, at least 5 months, at least 6 months, at least 12 months, 1 day to 2 days,1 day to 7 days, 2 days to 4 days, 3 days to 5 days, 4 days to 1 week, 1 week to 2 weeks, 1 week to 4 weeks, 2 weeks to 4 weeks, 2 weeks to 6 weeks, 3 weeks to 5 weeks, 4 weeks to 1 month, 1 month to 2 months, 1 month to 4 months, 1 month - 6 months, 2 months to 4 months, 3 months to 5 months, 4 months to 6 months, 5 months to 7 months, 6 months to 8 months, 6 months to 12 months, 7 months to 9 months, 8 months to 10 months, 9 months to 11 months, 10 months to 12 months, 11 months to 13 months, 12 months to 15 months, 15 months to 18 months, 18 months to 21 months, 21 months to 24 months, 2 years to 2.5 years, 2.5 years to 3 years, 3 years to 3.5 years, or 3.5 years to 4 years following implantation.

In some embodiments, the cell chamber device is maintained in the subject such that the plasma concentration of the biomolecule (e.g., recombinant peptide or recombinant protein) is at least 17 µg/mL in the subject for a period following implantation, e.g., for at least five days, for at least 10 days, at least 20 days, at least 30 days, at least 40 days, at least 50 days, at least 60 days, at least 3 months, at least 4 months, at least 5 months, at least 6 months, at least 12 months, 1 day to 2 days, 1 day to 7 days, 2 days to 4 days, 3 days to 5 days, 4 days to 7 days, 7 days to 10 days, 10 days to 20 days, 20 days to 30 days, 30 days to 45 days, 45 days to 60 days, 1 week to 2 weeks, 1 week to 4 weeks, 2 weeks to 4 weeks, 2 weeks to 6 weeks, 3 weeks to 5 weeks, 4 weeks to 1 month, 1 month to 2 months, 1 month to 4 months, 1 month to 6 months, 2 months to 4 months, 3 months to 5 months, 4 months to 6 months, 5 months to 7 months, 6 months to 8 months, 6 months - 12 months, 7 months to 9 months, 8 months to 10 months, 9 months to 11 months, 10 months to 12 months, 11 months to 13 months, 12 months to 15 months, 15 months to 18 months, 18 months to 21 months, 21 months to 24 months, 2 years to 2.5 years, 2.5 years to 3 years, 3 years to 3.5 years, or 3.5 years to 4 years following implantation.

In some embodiments, the cell chamber device is maintained in the subject for a period of time effective to deliver a dose of the biomolecule at 0.1 mg/kg body weight to about 10.0 mg/kg body weight of the subject, for example about 2 mg/kg to about 7 mg/kg, about 3 mg/kg to about 6 mg/kg, or about 15 to about 5 mg/kg. In particular embodiments, the dose of the biomolecule delivered over the duration of the implant is about 0.3 mg/kg, about 0.5 mg/kg, about 1 mg/kg, about 2 mg/kg, about 3 mg/kg, about 4 mg/kg, about 5 mg/kg, about 6 mg/kg, about 7 mg/kg, about 8 mg/kg, about 9 mg/kg, or about 10 mg/kg.

In some embodiments, the total dose of the biomolecule delivered over a period of time, e.g., per week, per every two weeks, per every four weeks, per every six weeks, per every eight weeks or per every ten weeks, of the implant may be about 22 mg, about 50 mg, about 72 mg, about 100 mg, about 125 mg, about 150 mg, about 165 mg, about 200 mg, about 300 mg, about 432 mg, about 450 mg or about 600 mg. In some embodiments, the total dose of the biomolecule delivered over a period of time, e.g., per week, per every two weeks, per every four weeks, per every six weeks, per every eight weeks or per every ten weeks, of the implant may be at least 77 mg, at least 125 mg or at least 356 mg. In one embodiment, the total dose of the biomolecule delivered over a period of time, e.g., per week, per every two weeks, per every four weeks, per every six weeks, per every eight weeks or per every ten weeks, of the implant is 165 mg. In another embodiment, the total dose per every two weeks is 108 mg. In another embodiment, the total dose per every two weeks is 216 mg. In another embodiment, the total dose per every two weeks is 150 mg. In another embodiment, the total dose per every two weeks is 200 mg. In another embodiment, the total dose per every four weeks is 300 mg. In another embodiment, the total dose per every eight weeks is 300 mg. For example, to deliver 300 mg of a biomolecule, e.g., antibody, such as an anti-α4β7 antibody (e.g., vedolizumab), or antigen-binding portion thereof per a period of 8 weeks, e.g., every 8 weeks, the implant would be sized and loaded with cells which can deliver about 37.5 mg/week. In another example, to deliver 108 mg of a biomolecule, e.g., antibody, such as an anti-α4β7 antibody (e.g., vedolizumab), or antigen-binding portion thereof per a period of 2 weeks, e.g., every 2 weeks, the implant would be sized and loaded with cells which can deliver about 54 mg/week. The foregoing examples are merely illustrative, and are not intended to be limiting.

In some embodiments, the dose of the biomolecule delivered over the duration of the implant is about 0.5 mg/kg per week to 10 mg/kg per week, 2 mg/kg per week to 6 mg/kg per week, 5 mg/kg per week to 15 mg/kg per week, 10 mg/kg per week to 20 mg/kg per week, 15 mg/kg per week to 30 mg/kg per week, 20 mg/kg per week to 40 mg/kg per week, 30 mg/kg per week to 60 mg/kg week.

In instances where the biomolecule is an antibody, such as an anti-α4β7 antibody (e.g., vedolizumab), or antigen-binding portion thereof, the cell chamber device, in some embodiments, can be maintained in the subject such that the daily dose of the antibody, or antigen binding portion thereof, secreted by the cells is at least about 1 mg/day (e.g., at least about 1 mg/day, at least about 2 mg/day, at least about 3 mg/day, at least about 4 mg/day, at least about 4.5 mg/day, about least about 5 mg/day, at least about 6 mg/day, at least about 7 mg/day, at least about 8 mg/day, at least about 9 mg/day, or more than 9 mg/day) for a period effective to achieve a therapeutic benefit in the subject (e.g., to achieve a therapeutically effective plasma concentration of the antibody, or antigen binding portion thereof, in the subject, or to achieve a clinical benefit in the subject). In some embodiments, the cell chamber device is maintained in the subject such that the serum or plasma concentration of an antibody, or antigen-binding portion thereof, secreted by the cells is at least 5 µg/mL (e.g., at least about 5 µg/mL, at least about 10 µg/mL, at least about 15 µg/mL, at least about 17 µg/mL, at least about 20 µg/mL, or more than 20 µg/mL) in the subject for a period effective to achieve a therapeutic benefit in the subject, e.g., at least 30 days (e.g., at least 30 days, at least 45 days, at least 55 days, at least 60 days, at least 70 days, at least 90 days, at least 120 days, at least 150 days, at least 240 days, at least 365 days, or for a period of 30 days to 90 days, 40 days to 60 days, 50 days to 70 days, 60 days to 70 days, 60 days to 90 days, 60 days to 120 days, 65 days to 75 days, 70 days to 90 days, 90 days to 120 days, 120 days to 240 days, 240 days to 365 days, or more than 365 days) following implantation.

In some embodiments, the cell chamber device is maintained in the subject such that the daily dose of the antibody, or antigen-binding portion thereof, secreted by the cells is 0.5 mg/day to 1 mg/day, 1 mg/day to 2 mg/day, 1mg/day to 5 mg/day, 2 mg/day to 5 mg/day, 2 mg/day to 25 mg/day, 4 mg/day to 7 mg/day, 4 mg/day to 12 mg/day, 5 mg/day to 8 mg/day, 5 mg/day to 10 mg/day, 6 mg/day to 9 mg/day, 7 mg/day to 10 mg/day, 10 mg/day to 15 mg/day, 8 mg/day to 11 mg/day, 6mg/day to 12 mg/day, or 9 mg/day to 12 mg/day in the subject for a period effective to achieve a therapeutic benefit in the subject.

In some embodiments, the cell chamber device is maintained in the subject such that the serum or plasma concentration of the antibody, or antigen-binding portion thereof, secreted by the cells is 2.5 µg/mL to 7.5 µg/mL, 4 µg/mL to 7 µg/mL, 5 µg/mL to 8 µg/mL, 5 µg/mL to 10 µg/mL, 5 µg/mL to 50 µg/mL, 8 µg/mL to 15 µg/mL, 10 µg/mL to 20 µg/mL, 16 µg/mL to 20 µg/mL, 18 µg/mL to 21 µg/mL, 21 µg/mL to 30 µg/mL, 25 µg/mL to 35 µg/mL, or 31 µg/mL to 45 µg/mL in the subject for a period effective to achieve a therapeutic benefit in the subject, e.g., at least 30 days (e.g., at least 30 days, at least 45 days, at least 55 days, at least 60 days, at least 70 days, at least 90 days, at least 120 days, at least 150 days, at least 240 days, at least 365 days, or for a period of 30 days to 90 days, 40 days to 60 days, 50 days to 70 days, 60 days to 70 days, 60 days to 90 days, 60 days to 120 days, 65 days to 75 days, 70 days to 90 days, 90 days to 120 days, 120 days to 240 days, 240 days to 365 days, or more than 365 days following implantation.

In some embodiments, the cell chamber device is maintained in the subject for a period of time effective to deliver a dose of an antibody, such as an anti-α4β7 antibody (e.g., vedolizumab), or antigen-binding portion thereof, at about 0.1 mg/kg body weight to about 10.0 mg/kg body weight, for example about 2 mg/kg to about 7 mg/kg, about 3 mg/kg to about 6 mg/kg, or about 15 to about 5 mg/kg, or about 5 mg/kg body weight to about 25 mg/kg body weight, or about 10 mg/kg body weight to about 20 mg/kg body weight,. In particular embodiments, the total dose of the antibody (e.g., vedolizumab), or antigen-binding fragment thereof, delivered over the duration of the implant is about 0.3 mg/kg, about 0.5 mg/kg, about 1 mg/kg, about 2 mg/kg, about 3 mg/kg, about 4 mg/kg, about 5 mg/kg, about 6 mg/kg, about 7 mg/kg, about 8 mg/kg, about 9 mg/kg, or about 10 mg/kg.

In some embodiments, the total dose of the antibody, or antigen-binding fragment thereof, delivered over the duration, e.g., about two weeks, about four weeks, about six weeks, about eight weeks, about 10 weeks, about 12 weeks, about 14 weeks, about 16 weeks, about 20 weeks, about 24 weeks, about 28 weeks, about 32 weeks, about 36 weeks or more, of the implant may be about 22 mg, about 50 mg, about 72 mg, about 125 mg, about 165 mg, or about 432 mg, about 450 mg, about 300 mg, about 600 mg, about 650 mg, about 900 mg, about 1200 mg, about 1300 mg, about 1500 mg, about 1800 mg, about 2100 mg or more. In some embodiments, the total dose of the antibody, or antigen-binding fragment thereof, delivered over the duration of the implant may be at least 77 mg, at least 125 mg or at least 356 mg. In one embodiment, the total dose of the antibody, or antigen-binding fragment thereof, delivered over the duration of the implant is 165 mg. In another embodiment, the total dose of the antibody, or antigen-binding fragment thereof, is 108 mg. In another embodiment, the total dose of the antibody, or antigen-binding fragment thereof, is 216 mg. In another embodiment, the total dose of the antibody, or antigen-binding fragment thereof, is 300 mg. For example, an implant which steadily delivers three total doses of 300 mg biomolecule, typically administered every eight weeks by intravenous administration, so it is implanted for 24 weeks and the device would deliver 900 mg total (and would deliver about 5.36 mg per day). In another example, an implant which steadily delivers six total doses of 108 mg biomolecule, typically administered every two weeks by subcutaneous administration, so it is implanted for 12 weeks and the device would deliver about 650 mg (and would deliver about 7.7 mg/day).

In some embodiments, the dose of the antibody, or antigen-binding portion thereof, delivered over the duration of the implant is about 0.5 mg/kg per week to 10 mg/kg per week, 2 mg/kg per week to 6 mg/kg per week, 5 mg/kg per week to 15 mg/kg per week, 10 mg/kg per week to 20 mg/kg per week, 15 mg/kg per week to 30 mg/kg per week, 20 mg/kg per week to 40 mg/kg per week, or 30 mg/kg per week to 60 mg/kg week. These dosage amounts are illustrative, and are not intended to be limiting.

In a further aspect, provided is a method of treating a subject having a disease by administering to the subject a device disclosed herein, wherein the cell chamber of the device comprises cells secreting a biomolecule in an amount and for a duration suitable to treat the disease or ameliorate one or more symptoms of the disease. Cells secreting a desired biomolecule can be selected or engineered based on the disease to be treated.

Examples of diseases that can be treated in accordance with the methods herein include a gastrointestinal disorder, a cancer, a respiratory disorder, a cardiovascular disease, a neurological condition, an autoimmune disorder, an endocrine and/or metabolism disorder, a hematological disorder, or an ocular disorder.

In some embodiments, the cell chamber device can be used to treat a gastrointestinal disorder, such as inflammatory bowel disease or short bowel syndrome. Examples of inflammatory bowel diseases (IBD) that can be treated in accordance with the methods herein include ulcerative colitis, Crohn's disease, primary sclerosing cholangitis, eosinophilic esophagitis, autoimmune hepatitis, ileitis, Celiac disease, nontropical Sprue, enteropathy associated with seronegative arthropathies, microscopic or collagenous colitis, eosinophilic gastroenteritis, or pouchitis resulting after proctocolectomy, and ileoanal anastomosis. In some embodiments, the inflammatory bowel disease is Crohn's disease or ulcerative colitis. In one embodiment, provided herein is a method of treating a subject having a gastrointestinal disorder comprising administering to the subject a device disclosed herein. In certain such embodiments, the subject having the gastrointestinal disorder is administered a device comprising cells that secrete a biomolecule that treats the gastrointestinal disorder.

In some embodiments, the cell chamber device is implanted into the peritoneum, mesentery, or peritoneal spaces of a subject having inflammatory bowel disease or short bowel syndrome. In some embodiments, the cell chamber device is implanted into the omentum, into or onto the lesser sac (i.e., the omental bursa or bursalis omentum), the greater omentum, lesser omentum, in or near the stomach, in or near small intestine, in or near the large intestine, or small bowel mesentery of the subject having inflammatory bowel disease or short bowel syndrome.

A cell chamber device provided herein can be used to deliver to a subject any biomolecule secreted by cells enclosed therein. For example, as described above, the cell chamber device can be used to deliver protein or peptide therapeutics, and therapeutic antibodies, or antigen-binding portions thereof. By way of example, the following embodiments illustrate exemplary methods of treatment that can be achieved using the cell chamber device described herein. These exemplary embodiments should not be construed to limit the invention in any way, as the methods can be readily be adapted for the treatment of other diseases or disorders through delivery of an appropriate biomolecule, e.g., therapeutic protein, peptide, antibody, etc., using a cell chamber device provided herein.

In one embodiment, provided herein is a method of treating a subject having Crohn's Disease or Ulcerative Colitis, comprising administering to the subject a device disclosed herein. In certain such embodiments, the subject having Crohn's Disease or Ulcerative Colitis is administered a device comprising cells that secrete an antibody, such as an anti-α4β7 antibody (e.g., vedolizumab or abrilumab), or an antigen-binding portion thereof. In some embodiments, the cell chamber device is maintained in the subject having Crohn's Disease or Ulcerative Colitis such that the serum or plasma concentration of the antibody, or antigen-binding portion thereof, is at least 5 µg/mL (e.g., at least about 5 µg/mL, at least about 10 µg/mL, at least about 15 µg/mL, at least about 17 µg/mL, at least about 20 µg/mL, or more than 20 µg/mL) in the subject for a period effective to achieve a therapeutic benefit in the subject, e.g., a period of at least 30 days (e.g., at least 30 days, at least 45 days, at least 55 days, at least 60 days, at least 70 days, at least 90 days, 30 days to 90 days, 40 days to 60 days, 50 days to 70 days, 60 days to 70 days, 60 days to 90 days, 60 days to 120 days, 65 days to 75 days, 70 days to 90 days, 90 days to 120 days, 120 days to 240 days, 240 days to 365 days, or more than 365 days) following implantation. In some embodiments, the cell chamber device is maintained in the subject having Crohn's Disease or Ulcerative Colitis such that the device delivers the antibody, or antigen-binding portion thereof at a rate of 10 mg/week to 50 mg/week, 20 mg/week to 60 mg/week, 30 mg/week to 75 mg/week, or 40 mg/week to 90 mg/week in the subject for a period effective to achieve a therapeutic benefit in the subject, e.g., for a period of at least 60 days (e.g., 60 days to 70 days, 65 days to 75 days, 70 days to 90 days, 90 days to 120 days, 120 days to 240 days, 240 days to 365 days, or more than 365 days) following implantation. In some embodiments, the cell chamber device is maintained in the subject having Crohn's Disease or Ulcerative Colitis such that the serum or plasma concentration of the antibody, or antigen-binding portion thereof, is 2.5 µg/mL to 7.5 µg/mL, 4 µg/mL to 7 µg/mL, 5 µg/mL to 8 µg/mL, 5 µg/mL to 10 µg/mL, 5 µg/mL to 50 µg/mL, 8 µg/mL to 15 µg/mL, 10 µg/mL to 20 µg/mL, 16 µg/mL to 20 µg/mL, 18 µg/mL to 21 µg/mL, 21 µg/mL to 30 µg/mL, 25 µg/mL to 35 µg/mL, or 31 µg/mL to 45 µg/mL in the subject for a period effective to achieve a therapeutic benefit in the subject, e.g., for a period of at least 60 days (e.g., 60 days to 70 days, 65 days to 75 days, 70 days to 90 days, 90 days to 120 days, 120 days to 240 days, 240 days to 365 days, or more than 365 days) following implantation. In some embodiments, Crohn's disease subjects can have moderately to severely active Crohn's disease (e.g., Crohn's Disease Activity Index (CDAI) score 220 to 450). Treatment may achieve clinical response or achieve clinical remission in patients suffering from moderate to severely active Crohn's disease. For example, treatment may result in mucosal healing. Treatment may also result in a reduction, elimination, or reduction and elimination of corticosteroid use by the patient (e.g., corticosteroid-free remission). In some embodiments, ulcerative colitis subjects may have moderate to severely active ulcerative colitis (e.g., having a Mayo score of six to 12 with endoscopy subscore of two or three). Treatment may result in induction and maintenance of clinical response, induction and maintenance of clinical remission, or mucosal healing in patients suffering from moderate to severely active ulcerative colitis. Treatment may also result in a reduction, elimination, or reduction and elimination of corticosteroid use by the patient (e.g., corticosteroid-free remission).

In another aspect, provided herein is a method of treating a subject having inflammatory bowel disease (IBD), comprising administering to the subject a cell chamber device disclosed herein, wherein the cells in the cell chamber secrete an anti-α4β7 antibody (e.g., vedolizumab or abrilumab), or an antigen-binding portion thereof. Examples of inflammatory bowel diseases (IBD) that can be treated in accordance with the methods herein include ulcerative colitis, Crohn's disease, ileitis, Celiac disease, nontropical Sprue, enteropathy associated with seronegative arthropathies, microscopic or collagenous colitis, eosinophilic gastroenteritis, or pouchitis resulting after proctocolectomy, and ileoanal anastomosis. In some embodiments, the cell chamber device is maintained in the subject having IBD such that the serum or plasma concentration of the antibody, or antigen-binding portion thereof, is at least 5 µg/mL (e.g., at least about 5 µg/mL, at least about 10 µg/mL, at least about 15 µg/mL, at least about 17 µg/mL, at least about 20 µg/mL, or more than 20 µg/mL) in the subject for a period effective to achieve a therapeutic benefit in the subject, e.g., a period of at least 30 days (e.g., at least 30 days, at least 45 days, at least 55 days, at least 60 days, at least 70 days, at least 90 days, 30 days to 90 days, 40 days to 60 days, 50 days to 70 days, 60 days to 70 days, 60 days to 90 days, 60 days to 120 days, 65 days to 75 days, 70 days to 90 days, 90 days to 120 days, 120 days to 240 days, 240 days to 365 days, or more than 365 days) following implantation. In some embodiments, the cell chamber device is maintained in the subject having IBD such that the serum or plasma concentration of the antibody, or antigen-binding portion thereof, is 2.5 µg/mL to 7.5 µg/mL, 4 µg/mL to 7 µg/mL, 5 µg/mL to 8 µg/mL, 5 µg/mL to 10 µg/mL, 5 µg/mL to 50 µg/mL, 8 µg/mL to 15 µg/mL, 10 µg/mL to 20 µg/mL, 16 µg/mL to 20 µg/mL, 18 µg/mL to 21 µg/mL, 21 µg/mL to 30 µg/mL, 25 µg/mL to 35 µg/mL, or 31 µg/mL to 45 µg/mL in the subject for a period effective to achieve a therapeutic benefit in the subject, e.g., for a period of at least 60 days (e.g., 60 days to 70 days, 65 days to 75 days, 70 days to 90 days, 90 days to 120 days, 120 days to 240 days, 240 days to 365 days, or more than 365 days) following implantation.

In some embodiments, the cell chamber device is implanted into the peritoneum, mesentery, or peritoneal spaces of a subject having Crohn's Disease, ulcerative Colitis, or other forms of IBD. In some embodiments, the cell chamber device is implanted into the omentum, into or onto the lesser sac (i.e., the omental bursa or bursalis omentum), the greater omentum, lesser omentum, in or near the stomach, in or near small intestine, or in or near the large intestine of the subject having Crohn's Disease, ulcerative Colitis, or other forms of IBD.

In one embodiment, provided herein is a method of treating a subject having primary sclerosing cholangitis, comprising administering to the subject a device disclosed herein. In certain such embodiments, the subject having primary sclerosing cholangitis is administered a device comprising cells that secrete an antibody, such as an anti-α4β7 antibody (e.g., vedolizumab or abrilumab), or an antigen-binding portion thereof. In some embodiments, the cell chamber device is maintained in the subject having primary sclerosing cholangitis such that the serum or plasma concentration of the antibody, or antigen-binding portion thereof, is at least 5 µg/mL (e.g., at least about 5 µg/mL, at least about 10 µg/mL, at least about 15 µg/mL, at least about 17 µg/mL, at least about 20 µg/mL, or more than 20 µg/mL) in the subject for a period effective to achieve a therapeutic benefit in the subject, e.g., a period of at least 30 days (e.g., at least 30 days, at least 45 days, at least 55 days, at least 60 days, at least 70 days, at least 90 days, 30 days to 90 days, 40 days to 60 days, 50 days to 70 days, 60 days to 70 days, 60 days to 90 days, 60 days to 120 days, 65 days to 75 days, 70 days to 90 days, 90 days to 120 days, 120 days to 240 days, 240 days to 365 days, or more than 365 days) following implantation. In some embodiments, the cell chamber device is maintained in the subject having primary sclerosing cholangitis such that the serum or plasma concentration of the antibody, or antigen-binding portion thereof, is 2.5 µg/mL to 7.5 µg/mL, 4 µg/mL to 7 µg/mL, 5 µg/mL to 8 µg/mL, 5 µg/mL to 10 µg/mL, 5 µg/mL to 50 µg/mL, 8 µg/mL to 15 µg/mL, 10 µg/mL to 20 µg/mL, 16 µg/mL to 20 µg/mL, 18 µg/mL to 21 µg/mL, 21 µg/mL to 30 µg/mL, 25 µg/mL to 35 µg/mL, or 31 µg/mL to 45 µg/mL in the subject for a period effective to achieve a therapeutic benefit in the subject, e.g., a period of at least 30 days (e.g., at least 30 days, at least 45 days, at least 55 days, at least 60 days, at least 70 days, at least 90 days, 30 days to 90 days, 40 days to 60 days, 50 days to 70 days, 60 days to 70 days, 60 days to 90 days, 60 days to 120 days, 65 days to 75 days, 70 days to 90 days, 90 days to 120 days, 120 days to 240 days, 240 days to 365 days, or more than 365 days) following implantation. In some embodiments, the cell chamber device is maintained in the subject having primary sclerosing cholangitis such that the device delivers the antibody, or antigen-binding portion thereof at a rate of 10 mg/week to 50 mg/week, 20 mg/week to 60 mg/week, 30 mg/week to 75 mg/week, or 40 mg/week to 90 mg/week in the subject for a period effective to achieve a therapeutic benefit in the subject, e.g., for a period of at least 60 days (e.g., 60 days to 70 days, 65 days to 75 days, 70 days to 90 days, 90 days to 120 days, 120 days to 240 days, 240 days to 365 days, or more than 365 days) following implantation.

In some embodiments, the cell chamber device is implanted into the peritoneum, mesentery, or peritoneal spaces of a subject having primary sclerosing cholangitis. In some embodiments, the cell chamber device is implanted into the omentum, into or onto the lesser sac (i.e., the omental bursa or bursalis omentum), the greater omentum, lesser omentum, in or near the liver, or in or near the gallbladder of the subject having primary sclerosing cholangitis.

In one embodiment, provided herein is a method of treating a subject having lymphocytic esophagitis or eosinophilic esophagitis, comprising administering to the subject a device disclosed herein. In certain such embodiments, the subject having eosinophilic esophagitis is administered a device comprising cells that secrete an antibody, such as an anti-α4β7 antibody (e.g., vedolizumab or abrilumab), or an antigen-binding portion thereof. In some embodiments, the cell chamber device is maintained in the subject having eosinophilic esophagitis such that the serum or plasma concentration of the antibody, or antigen-binding portion thereof, is at least 5 µg/mL (e.g., at least about 5 µg/mL, at least about 10 µg/mL, at least about 15 µg/mL, at least about 17 µg/mL, at least about 20 µg/mL, or more than 20 µg/mL) in the subject for a period effective to achieve a therapeutic benefit in the subject, e.g., a period of at least 30 days (e.g., at least 30 days, at least 45 days, at least 55 days, at least 60 days, at least 70 days, at least 90 days, 30 days to 90 days, 40 days to 60 days, 50 days to 70 days, 60 days to 70 days, 60 days to 90 days, 60 days to 120 days, 65 days to 75 days, 70 days to 90 days, 90 days to 120 days, 120 days to 240 days, 240 days to 365 days, or more than 365 days) following implantation (e.g., above the diaphragm). In some embodiments, the cell chamber device is maintained in the subject having eosinophilic esophagitis such that the serum or plasma concentration of the antibody, or antigen-binding portion thereof , is 2.5 µg/mL to 7.5 µg/mL, 4 µg/mL to 7 µg/mL, 5 µg/mL to 8 µg/mL, 5 µg/mL to 10 µg/mL, 5 µg/mL to 50 µg/mL, 8 µg/mL to 15 µg/mL, 10 µg/mL to 20 µg/mL, 16 µg/mL to 20 µg/mL, 18 µg/mL to 21 µg/mL, 21 µg/mL to 30 µg/mL, 25 µg/mL to 35 µg/mL, or 31 µg/mL to 45 µg/mL in the subject for a period effective to achieve a therapeutic benefit in the subject, e.g., at least 30 days (e.g., at least 30 days, at least 45 days, at least 55 days, at least 60 days, at least 70 days, at least 90 days, 30 days to 90 days, 40 days to 60 days, 50 days to 70 days, 60 days to 70 days, 60 days to 90 days, 60 days to 120 days, 65 days to 75 days, 70 days to 90 days, 90 days to 120 days, 120 days to 240 days, 240 days to 365 days, or more than 365 days) following implantation. In some embodiments, the cell chamber device is maintained in the subject having lymphocytic esophagitis or eosinophilic esophagitis such that the device delivers the antibody, or antigen-binding portion thereof at a rate of 10 mg/week to 50 mg/week, 20 mg/week to 60 mg/week, 30 mg/week to 75 mg/week, or 40 mg/week to 90 mg/week in the subject for a period effective to achieve a therapeutic benefit in the subject, e.g., for a period of at least 60 days (e.g., 60 days to 70 days, 65 days to 75 days, 70 days to 90 days, 90 days to 120 days, 120 days to 240 days, 240 days to 365 days, or more than 365 days) following implantation.

In some embodiments, the cell chamber device is implanted into the peritoneum, mesentery, or peritoneal spaces of a subject having eosinophilic esophagitis. In some embodiments, the cell chamber device is implanted into the omentum, into or onto the lesser sac (i.e., the omental bursa or bursalis omentum), the greater omentum, lesser omentum, in or near the esophagus, or above the diaphragm of the subject having lymphocytic esophagitis or eosinophilic esophagitis.

In one embodiment, provided herein is a method of treating a subject having autoimmune hepatitis, comprising administering to the subject a device disclosed herein. In certain such embodiments, the subject having autoimmune hepatitis is administered a device comprising cells that secrete an antibody, such as an anti-α4β7 antibody (e.g., vedolizumab or abrilumab), or an antigen-binding portion thereof. In some embodiments, the cell chamber device is maintained in the subject having autoimmune hepatitis such that the serum or plasma concentration of the antibody, or antigen-binding portion thereof, is at least 5 µg/mL (e.g., at least about 5 µg/mL, at least about 10 µg/mL, at least about 15 µg/mL, at least about 17 µg/mL, at least about 20 µg/mL, or more than 20 µg/mL) in the subject for a period effective to achieve a therapeutic benefit in the subject, e.g., at least 30 days (e.g., at least 30 days, at least 45 days, at least 55 days, at least 60 days, at least 70 days, at least 90 days, 30 days to 90 days, 40 days to 60 days, 50 days to 70 days, 60 days to 70 days, 60 days to 90 days, 60 days to 120 days, 65 days to 75 days, 70 days to 90 days, 90 days to 120 days, 120 days to 240 days, 240 days to 365 days, or more than 365 days)following implantation. In some embodiments, the cell chamber device is maintained in the subject having autoimmune hepatitis such that the serum or plasma concentration of the antibody, or antigen-binding portion thereof, is 2.5 µg/mL to 7.5 µg/mL, 4 µg/mL to 7 µg/mL, 5 µg/mL to 8 µg/mL, 5 µg/mL to 10 µg/mL, 5 µg/mL to 50 µg/mL, 8 µg/mL to 15 µg/mL, 10 µg/mL to 20 µg/mL, 16 µg/mL to 20 µg/mL, 18 µg/mL to 21 µg/mL, 21 µg/mL to 30 µg/mL, 25 µg/mL to 35 µg/mL, or 31 µg/mL to 45 µg/mL in the subject for a period effective to achieve a therapeutic benefit in the subject, e.g., at least 30 days (e.g., at least 30 days, at least 45 days, at least 55 days, at least 60 days, at least 70 days, at least 90 days, 30 days to 90 days, 40 days to 60 days, 50 days to 70 days, 60 days to 70 days, 60 days to 90 days, 60 days to 120 days, 65 days to 75 days, 70 days to 90 days, 90 days to 120 days, 120 days to 240 days, 240 days to 365 days, or more than 365 days) following implantation. In some embodiments, the cell chamber device is maintained in the subject having autoimmune hepatitis such that the device delivers the antibody, or antigen-binding portion thereof at a rate of 10 mg/week to 50 mg/week, 20 mg/week to 60 mg/week, 30 mg/week to 75 mg/week, or 40 mg/week to 90 mg/week in the subject for a period effective to achieve a therapeutic benefit in the subject, e.g., for a period of at least 60 days (e.g., 60 days to 70 days, 65 days to 75 days, 70 days to 90 days, 90 days to 120 days, 120 days to 240 days, 240 days to 365 days, or more than 365 days) following implantation.

In some embodiments, the cell chamber device is implanted into the peritoneum, mesentery, or peritoneal spaces of a subject having autoimmune hepatitis. In some embodiments, the cell chamber device is implanted into the omentum, into or onto the lesser sac (i.e., the omental bursa or bursalis omentum), the greater omentum, lesser omentum, or in or near the liver of the subject having autoimmune hepatitis.

Diseases or pathogens whose etiologies exploit the interaction of MAdCAM (e.g., MAdCAM-1) with α4β7 can also be treated with an anti- α4β7 antibody (e.g., vedolizumab or abrilumab) or antigen-binding portion thereof delivered by a cell chamber device described herein. Examples of such diseases include immunodeficiency disorders, such as caused by human immunodeficiency virus (See, e.g., WO2008140602).

Pancreatitis and insulin-dependent diabetes mellitus are other diseases which can be treated with an anti- α4β7 antibody (e.g., vedolizumab or abrilumab) or antigen-binding portion thereof delivered using the cell chamber device disclosed herein. It has been reported that MAdCAM (e.g., MAdCAM-1) is expressed by some vessels in the exocrine pancreas from NOD (non-obese diabetic) mice, as well as from BALB/c and SJL mice. Expression of MAdCAM (e.g., MAdCAM-1) was reportedly induced on endothelium in inflamed islets of the pancreas of the NOD mouse, and MAdCAM (e.g., MAdCAM-1) was the predominant addressin expressed by NOD islet endothelium at early stages of insulitis (Hanninen, A., et al., J. Clin. Invest., 92: 2509-2515 (1993)). Treatment of NOD mice with either anti-MAdCAM or anti-beta 7 antibodies prevented the development of diabetes (Yang et al., Diabetes, 46:1542-1547 (1997)). Further, accumulation of lymphocytes expressing α4β7 within islets was observed, and MAdCAM-1 was implicated in the binding of lymphoma cells via a α4β7 to vessels from inflamed islets (Hanninen, A., et al., J. Clin. Invest., 92: 2509-2515 (1993)) or to the gastrointestinal tract in mantle cell lymphoma (Geissmann et al., Am. J. Pathol., 153:1701-1705 (1998)).

Other inflammatory diseases associated with mucosal tissues which can be treated with an anti- α4β7 antibody (e.g., vedolizumab or abrilumab) or antigen-binding portion thereof delivered using a cell chamber device herein include cholecystitis, cholangitis (Adams and Eksteen Nature Reviews 6:244-251 (2006) Grant et al., Hepatology 33:1065-1072 (2001)), e.g., Behcet's disease, e.g., of the intestine, or pericholangitis (bile duct and surrounding tissue of the liver), and graft versus host disease (e.g., in the gastrointestinal tract (e.g., after a bone marrow transplant) (Petrovic et al. Blood 103:1542-1547 (2004)). As seen in Crohn's disease, inflammation often extends beyond the mucosal surface, accordingly chronic inflammatory diseases, such as sarcoidosis, chronic gastritis, e.g., autoimmune gastritis (Katakai et al., Int. Immunol., 14:167-175 (2002)) and other idiopathic conditions can be amenable to treatment.

Also provided herein is a method of inhibiting leukocyte infiltration of mucosal tissue with an anti- α4β7 antibody (e.g., vedolizumab or abrilumab) or antigen-binding portion thereof delivered using a cell chamber device herein. Further provided herein is a method for treating cancer (e.g., an α4β7 positive tumor, such as a lymphoma). Other examples of inflammatory diseases associated with mucosal tissues which can be treated with an anti- α4β7 antibody (e.g., vedolizumab or abrilumab) or antigen-binding portion thereof delivered using a cell chamber device herein include mastitis (mammary gland) and irritable bowel syndrome.

The cell chamber device can be loaded with a number of cells and implanted for a duration sufficient to deliver an effective amount of an anti-α4β7 antibody or antigen-binding portion thereof which inhibits binding of α4β7 integrin to a ligand thereof. For therapy, an effective amount will be sufficient to achieve the desired therapeutic (including prophylactic) effect (such as an amount sufficient to reduce or prevent α4β7 integrin-mediated binding and/or signaling, thereby inhibiting leukocyte adhesion and infiltration and/or associated cellular responses). An effective amount of an anti-α4β7 antibody, e.g., an effective titer sufficient to maintain saturation, e.g., neutralization, of α4β7 integrin, can induce clinical response or remission of an α4β7-associated condition described herein, e.g., Crohn's disease, ulcerative colitis, inflammatory bowel disease, primary sclerosing cholangitis, eosinophilic esophagitis, autoimmune hepatitis, pancreatitis, cholecystitis, cholangitis, lymphoma, etc. The size of the cell chamber device and the number of anti-α4β7 antibody-producing cells in the device can be adjusted in accordance with the present methods to produce an effective amount of the anti-α4β7 antibody in the subject. For example, an anti-α4β7 antibody can be delivered via a cell chamber device to a subject in an amount and for a duration disclosed herein

The dosage of the anti-α4β7 antibody delivered by the cell chamber device can be optimized to induce a clinical response and clinical remission in the inflammatory bowel disease of the patient. In some embodiments, the dosing regimen does not alter the ratio of CD4 to CD8 in cerebrospinal fluid of patients receiving treatment. CD4:CD8 ratios can be measured in blood, lymph node aspirate, and cerebro-spinal fluid (CSF). The CSF CD4+:CD8+ lymphocyte ratios in healthy individuals are typically greater than or equal to about 1. (Svenningsson et al., J. Neuroimmunol. 1995; 63:39-46; Svenningsson et al., Ann Neurol. 1993; 34:155-161). An immunomodulator can alter the CD4:CD8 ratio to less than 1.

In one embodiment, provided herein is a method of treating a subject having rheumatoid arthritis, psoriatic arthritis, ankylosing spondylitis, Crohn's disease, ulcerative colitis, psoriasis, hidradenitis suppurativa, uveitis, or juvenile idiopathic arthritis, comprising administering to the subject a device disclosed herein. In certain such embodiments, the subject having rheumatoid arthritis, psoriatic arthritis, ankylosing spondylitis, Crohn's disease, ulcerative colitis, psoriasis, hidradenitis suppurativa, uveitis, or juvenile idiopathic arthritis is administered a device comprising cells that secrete an antibody, such as an anti-TNFα antibody (e.g., adalimumab, certolizumab, golimumab, or infliximab), or an antigen-binding portion thereof. In some embodiments, the cell chamber device is maintained in the subject having rheumatoid arthritis, psoriatic arthritis, ankylosing spondylitis, Crohn's disease, ulcerative colitis, psoriasis, hidradenitis suppurativa, uveitis, or juvenile idiopathic arthritis such that the anti-TNFα antibody (e.g., adalimumab, certolizumab, golimumab, or infliximab) or an antigen-binding portion thereof, is secreted at a rate to sufficient to provide a weekly, biweekly, monthly, or bimonthly dose of at least 20 mg (e.g., at least 20 mg, at least about 30 mg, at least about 40 mg, at least about 50 mg, at least about 60 mg, at least about 70 mg, at least about 80 mg, at least 100 mg, at least 150 mg, at least 200 mg, at least 300 mg, or at least 400 mg) in the subject for a period effective to achieve a therapeutic benefit in the subject. In some embodiments, the cell chamber device is maintained in the subject having rheumatoid arthritis, psoriatic arthritis, ankylosing spondylitis, Crohn's disease, ulcerative colitis, psoriasis, hidradenitis suppurativa, uveitis, or juvenile idiopathic arthritis such that plasma or serum concentration of the anti-TNFα antibody (e.g., adalimumab, certolizumab, golimumab, or infliximab) or antigen-binding portion thereof, is 2.5 µg/mL to 7.5 µg/mL, 4 µg/mL to 7 µg/mL, 5 µg/mL to 8 µg/mL, 5 µg/mL to 10 µg/mL, 5 µg/mL to 50 µg/mL, 8 µg/mL to 15 µg/mL, 10 µg/mL to 20 µg/mL, 16 µg/mL to 20 µg/mL, 18 µg/mL to 21 µg/mL, 21 µg/mL to 30 µg/mL, 25 µg/mL to 35 µg/mL, or 31 µg/mL to 45 µg/mL in the subject for a period effective to achieve a therapeutic benefit in the subject, e.g., at least 30 days (e.g., at least 30 days, at least 45 days, at least 55 days, at least 60 days, at least 70 days, at least 90 days, 30 days to 90 days, 40 days to 60 days, 50 days to 70 days, 60 days to 70 days, 60 days to 90 days, 60 days to 120 days, 65 days to 75 days, 70 days to 90 days, 90 days to 120 days, 120 days to 240 days, 240 days to 365 days, or more than 365 days) following implantation.

In another aspect, provided herein is a method of treating a subject having inflammatory bowel disease (IBD), comprising administering to the subject a cell chamber device disclosed herein, wherein the cells in the cell chamber secrete an anti-TNFα antibody (e.g., adalimumab, certolizumab, golimumab, or infliximab). Examples of inflammatory bowel diseases (IBD) that can be treated in accordance with the methods herein include ulcerative colitis, Crohn's disease, ileitis, Celiac disease, nontropical Sprue, enteropathy associated with seronegative arthropathies, microscopic or collagenous colitis, eosinophilic gastroenteritis, or pouchitis resulting after proctocolectomy, and ileoanal anastomosis. In some embodiments, the inflammatory bowel disease is Crohn's disease or ulcerative colitis.

In another embodiment, provided herein is a method of treating a subject having ulcerative colitis or Crohn's disease, comprising administering to the subject a device disclosed herein. In certain such embodiments, the subject having ulcerative colitis or Crohn's disease is administered a device comprising cells that secrete an antibody, such as an anti-integrin β₇ antibody (e.g., etrolizumab). In some embodiments, the cell chamber device is maintained in the subject having ulcerative colitis or Crohn's disease such that the anti-integrin β₇ antibody (e.g., etrolizumab) or antigen-binding portion thereof, is secreted at a rate to sufficient to provide a weekly, biweekly, monthly, or bimonthly dose of at least 50 mg (e.g., at least about 50 mg, at least about 25 mg, at least about 75 mg, at least about 100 mg, at least about 125 mg, at least about 150 mg, at least about 175 mg, at least about 200 mg, at least about 225 mg, or at least about 250 mg) in the subject for a period effective to achieve a therapeutic benefit in the subject. In some embodiments, the cell chamber device is maintained in the subject having ulcerative colitis or Crohn's disease such that the plasma or serum concentration of the anti-integrin β₇ antibody (e.g., etrolizumab) or antigen-binding portion thereof, is 2.5 µg/mL to 7.5 µg/mL, 4 µg/mL to 7 µg/mL, 5 µg/mL to 8 µg/mL, 5 µg/mL to 10 µg/mL, 5 µg/mL to 50 µg/mL, 8 µg/mL to 15 µg/mL, 10 µg/mL to 20 µg/mL, 16 µg/mL to 20 µg/mL, 18 µg/mL to 21 µg/mL, 21 µg/mL to 30 µg/mL, 25 µg/mL to 35 µg/mL, or 31 µg/mL to 45 µg/mL in the subject for a period effective to achieve a therapeutic benefit in the subject, e.g., at least 30 days (e.g., at least 30 days, at least 45 days, at least 55 days, at least 60 days, at least 70 days, at least 90 days, 30 days to 90 days, 40 days to 60 days, 50 days to 70 days, 60 days to 70 days, 60 days to 90 days, 60 days to 120 days, 65 days to 75 days, 70 days to 90 days, 90 days to 120 days, 120 days to 240 days, 240 days to 365 days, or more than 365 days) following implantation.

In some embodiments, the subject having ulcerative colitis or Crohn's disease is administered a device comprising cells that secrete an antibody, such as an anti-IL-12/IL-23 antibody (e.g., ustekinumab). In some embodiments, the cell chamber device is maintained in the subject having ulcerative colitis or Crohn's disease such that the anti-IL-12/IL-23 antibody (e.g., ustekinumab) or antigen-binding portion thereof, is secreted at a rate to sufficient to provide a weekly, biweekly, monthly, or bimonthly dose of at least 30 mg (e.g., at least about 30 mg, at least about 40 mg, at least about 75 mg, at least about 100 mg, at least about 125 mg, at least about 150 mg, at least about 175 mg, at least about 200 mg, at least about 225 mg, at least about 250 mg, at least about 300 mg, at least about 350 mg, at least about 400 mg, at least about 450 mg, at least about 500 mg, or at least about 550 mg) in the subject for a period effective to achieve a therapeutic benefit in the subject. In some embodiments, the cell chamber device is maintained in the subject having ulcerative colitis or Crohn's disease such that the plasma or serum concentration of the anti-IL-12/IL-23 antibody (e.g., ustekinumab) or antigen-binding portion thereof, is 2.5 µg/mL to 7.5 µg/mL, 4 µg/mL to 7 µg/mL, 5 µg/mL to 8 µg/mL, 5 µg/mL to 10 µg/mL, 5 µg/mL to 50 µg/mL, 8 µg/mL to 15 µg/mL, 10 µg/mL to 20 µg/mL, 16 µg/mL to 20 µg/mL, 18 µg/mL to 21 µg/mL, 21 µg/mL to 30 µg/mL, 25 µg/mL to 35 µg/mL, or 31 µg/mL to 45 µg/mL in the subject for a period effective to achieve a therapeutic benefit in the subject, e.g., at least 30 days (e.g., at least 30 days, at least 45 days, at least 55 days, at least 60 days, at least 70 days, at least 90 days, 30 days to 90 days, 40 days to 60 days, 50 days to 70 days, 60 days to 70 days, 60 days to 90 days, 60 days to 120 days, 65 days to 75 days, 70 days to 90 days, 90 days to 120 days, 120 days to 240 days, 240 days to 365 days, or more than 365 days) following implantation.

In one embodiment, provided herein is a method of treating a subject having an autoimmune disease or a cancer, comprising administering to the subject a device disclosed herein. In certain such embodiments, the subject having an autoimmune disease (e.g., rheumatoid arthritis) or a cancer (e.g., Non-Hodgkin's lymphoma, chronic lymphocytic leukemia) is administered a device comprising cells that secrete an antibody, such as an anti-CD20 antibody (e.g., rituximab). In some embodiments, the cell chamber device is maintained in the subject having autoimmune disease or a cancer such that the anti-CD20 antibody (e.g., rituximab) concentration, or concentration of an antigen-binding portion thereof, is at least 200 mg/m² (e.g., at least about 200 mg/m², at least about 225 mg/m², at least about 250 mg/m², at least about 300 mg/m², at least about 350 mg/m², at least about 400 mg/m², at least about 450 mg/m², at least about 500 mg/m², or at least about 550 mg/m²) in the subject for a period effective to achieve a therapeutic benefit in the subject. In some embodiments, the cell chamber device is maintained in the subject having autoimmune disease or a cancer such that the anti-CD20 antibody (e.g., rituximab) or antigen-binding portion thereof, is secreted at a rate to sufficient to provide a weekly, biweekly, monthly, or bimonthly dose of at least 200-250 mg/m², 225-275 mg/m², 250-325 mg/m², 300-350 mg/m², 325-375 mg/m², 350-400 mg/m², 375-425 mg/m², 400-450 mg/m², 425-475 mg/m², 450-500 mg/m², or 475-525 mg/m² in the subject for a period effective to achieve a therapeutic benefit in the subject. In some embodiments, the cell chamber device is maintained in the subject having autoimmune disease or cancer such that the anti-CD20 antibody (e.g., rituximab) or antigen-binding portion thereof, is secreted at a rate to sufficient to provide a weekly, biweekly, monthly, or bimonthly dose of at least 150-200 mg, 175-225 mg, 200-250 mg, 225-275 mg, 250-325 mg, 300-350 mg, 325-375mg, 350-400 mg, 375-425 mg, 400-450mg, 425-475 mg, 450-500 mg, 475-525 mg, 500-550 mg, 525-575 mg, 550-600 mg, 575-625 mg, 600-650 mg, 625-675 mg, 650-700 mg, 675-725 mg, 700-750 mg, 725-775 mg, 750-800 mg, 775-825 mg, 800-850mg, 825-875 mg, 850-900 mg, 875-925 mg, 900-950 mg, 925-975 mg, 950-1000 mg, 900-1100 mg, or 1000 - 1200 mg in the subject for a period effective to achieve a therapeutic benefit in the subject. In some embodiments, the cell chamber device is maintained in the subject having autoimmune disease or cancer such that the plasma or serum concentration of the anti-CD20 antibody (e.g., rituximab) or antigen-binding portion thereof, is 2.5 µg/mL to 7.5 µg/mL, 4 µg/mL to 7 µg/mL, 5 µg/mL to 8 µg/mL, 5 µg/mL to 10 µg/mL, 5 µg/mL to 50 µg/mL, 8 µg/mL to 15 µg/mL, 10 µg/mL to 20 µg/mL, 16 µg/mL to 20 µg/mL, 18 µg/mL to 21 µg/mL, 21 µg/mL to 30 µg/mL, 25 µg/mL to 35 µg/mL, or 31 µg/mL to 45 µg/mL in the subject for a period effective to achieve a therapeutic benefit in the subject, e.g., at least 30 days (e.g., at least 30 days, at least 45 days, at least 55 days, at least 60 days, at least 70 days, at least 90 days, 30 days to 90 days, 40 days to 60 days, 50 days to 70 days, 60 days to 70 days, 60 days to 90 days, 60 days to 120 days, 65 days to 75 days, 70 days to 90 days, 90 days to 120 days, 120 days to 240 days, 240 days to 365 days, or more than 365 days) following implantation.

In some embodiments, the subject having multiple sclerosis or Crohn's disease is administered a device comprising cells that secrete an antibody, such as an anti-alpha-4 integrin antibody (e.g., natalizumab). In some embodiments, the cell chamber device is maintained in the subject having multiple sclerosis or Crohn's disease such that the anti-alpha-4 integrin antibody (e.g., natalizumab) or antigen-binding portion thereof, is secreted at a rate to sufficient to provide a weekly, biweekly, monthly, or bimonthly dose of at least 30 mg (e.g., at least about 30 mg, at least about 04 mg, at least about 75 mg, at least about 100 mg, at least about 125 mg, at least about 150 mg, at least about 175 mg, at least about 200 mg, at least about 225 mg, at least about 250 mg, at least about 300 mg, or at least about 350 mg) in the subject for a period effective to achieve a therapeutic benefit in the subject. In some embodiments, the cell chamber device is maintained in the subject having multiple sclerosis or Crohn's disease such that the plasma or serum concentration of the anti-alpha-4 integrin antibody (e.g., natalizumab) or antigen-binding portion thereof, is 2.5 µg/mL to 7.5 µg/mL, 4 µg/mL to 7 µg/mL, 5 µg/mL to 8 µg/mL, 5 µg/mL to 10 µg/mL, 5 µg/mL to 50 µg/mL, 8 µg/mL to 15 µg/mL, 10 µg/mL to 20 µg/mL, 16 µg/mL to 20 µg/mL, 18 µg/mL to 21 µg/mL, 21 µg/mL to 30 µg/mL, 25 µg/mL to 35 µg/mL, or 31 µg/mL to 45 µg/mL in the subject for a period effective to achieve a therapeutic benefit in the subject, e.g., at least 30 days (e.g., at least 30 days, at least 45 days, at least 55 days, at least 60 days, at least 70 days, at least 90 days, 30 days to 90 days, 40 days to 60 days, 50 days to 70 days, 60 days to 70 days, 60 days to 90 days, 60 days to 120 days, 65 days to 75 days, 70 days to 90 days, 90 days to 120 days, 120 days to 240 days, 240 days to 365 days, or more than 365 days) following implantation.

In another embodiment, provided herein is a method of treating a subject having short bowel syndrome, comprising administering to the subject a device disclosed herein. In certain such embodiments, the subject having short bowel syndrome is administered a device comprising cells that secrete a peptide therapeutic for gastrointestinal use.

In some embodiments, the cell chamber device can be used to treat a subject having cancer, for example, a melanoma, a non-small cell lung cancer, a small-cell lung cancer, a lung cancer, a leukemia, a hepatocarcinoma, a retinoblastoma, an astrocytoma, a glioblastoma, a gum cancer, a tongue cancer, a neuroblastoma, a head cancer, a neck cancer, a breast cancer, a pancreatic cancer, a prostate cancer, a renal cancer, a bone cancer, a testicular cancer, an ovarian cancer, a mesothelioma, a cervical cancer, a gastrointestinal cancer, a lymphoma, a myeloma, a brain cancer, a colon cancer, a sarcoma or a bladder cancer. Accordingly, in one embodiment, provided herein is a method of treating a subject having a cancer comprising administering to the subject a cell chamber device disclosed herein. In certain such embodiments, the subject having the cancer is administered a cell chamber device comprising cells that secrete a biomolecule (e.g., an antibody) that treats the cancer. For example, in some embodiments, the cells in the cell chamber device secrete an antibody, or antigen-binding portion thereof, that specifically binds CD20 (e.g., rituximab), VEGF (e.g., bevacizumab), HER2/neu (e.g., trastuzumab), PD-L1 (e.g., atezolizumab, avelumab, durvalumab), PD-1 (e.g., pembrolizumab, nivolumab, cemiplimab), or EGFR (e.g., cetuximab, panitumumab). In some embodiments, the cell chamber device is maintained in the subject having cancer such that the plasma or serum concentration of the antibody, or antigen-binding portion thereof, is 2.5 µg/mL to 7.5 µg/mL, 4 µg/mL to 7 µg/mL, 5 µg/mL to 8 µg/mL, 5 µg/mL to 10 µg/mL, 5 µg/mL to 50 µg/mL, 8 µg/mL to 15 µg/mL, 10 µg/mL to 20 µg/mL, 16 µg/mL to 20 µg/mL, 18 µg/mL to 21 µg/mL, 21 µg/mL to 30 µg/mL, 25 µg/mL to 35 µg/mL, or 31 µg/mL to 45 µg/mL in the subject for a period effective to achieve a therapeutic benefit in the subject, e.g., at least 30 days (e.g., at least 30 days, at least 45 days, at least 55 days, at least 60 days, at least 70 days, at least 90 days, 30 days to 90 days, 40 days to 60 days, 50 days to 70 days, 60 days to 70 days, 60 days to 90 days, 60 days to 120 days, 65 days to 75 days, 70 days to 90 days, 90 days to 120 days, 120 days to 240 days, 240 days to 365 days, or more than 365 days) following implantation. In some embodiments, the implant can be placed subcutaneously near the site of the cancer in the subject, e.g., near the organ comprising a tumor.

In some embodiments, the cell chamber device can be used to treat a subject having an autoimmune disorder, for example, graft versus host disease (GVHD), organ transplant rejection, autoimmune hepatitis, primary biliary cirrhosis, autoimmune cholangitis, primary sclerosing cholangitis, irritable bowel syndrome (IBS), multiple sclerosis (MS), chronic granulomatous disease, ankylosing spondylitis, scleroderma, polymyositis, (dermato)myositis, systemic vasculitis, systemic lupus erythematosus (SLE), Crohn's disease, insulin-dependent diabetes (type 1) or ulcerative colitis. Accordingly, in one embodiment, provided herein is a method of treating a subject having an autoimmune disorder comprising administering to the subject a cell chamber device disclosed herein. In certain such embodiments, the subject having the autoimmune disorder is administered a cell chamber device comprising cells that secrete a biomolecule that treats the autoimmune disorder. For example, in some embodiments, the cells in the cell chamber device secrete an antibody, or antigen-binding portion thereof, that specifically binds α4β7 (e.g., vedolizumab, abrilumab), CD20 (e.g., rituximab), IL-12/IL-23 (e.g., ustekinumab), integrin α4 (e.g., natalizumab), TNF-α (e.g., adalimumab, certolizumab, golimumab, infliximab), integrin β₇ (e.g., etrolizumab, CD25 (e.g., basiliximab), IL-2Rα (e.g., daclizumab), or IgE (e.g., omalizumab). In some embodiments, the cell chamber device is maintained in the subject having an autoimmune disorder such that the plasma or serum concentration of the antibody, or antigen-binding portion thereof, is 2.5 µg/mL to 7.5 µg/mL, 4 µg/mL to 7 µg/mL, 5 µg/mL to 8 µg/mL, 5 µg/mL to 10 µg/mL, 5 µg/mL to 50 µg/mL, 8 µg/mL to 15 µg/mL, 10 µg/mL to 20 µg/mL, 16 µg/mL to 20 µg/mL, 18 µg/mL to 21 µg/mL, 21 µg/mL to 30 µg/mL, 25 µg/mL to 35 µg/mL, or 31 µg/mL to 45 µg/mL in the subject for a period effective to achieve a therapeutic benefit in the subject, e.g., at least 30 days (e.g., at least 30 days, at least 45 days, at least 55 days, at least 60 days, at least 70 days, at least 90 days, 30 days to 90 days, 40 days to 60 days, 50 days to 70 days, 60 days to 70 days, 60 days to 90 days, 60 days to 120 days, 65 days to 75 days, 70 days to 90 days, 90 days to 120 days, 120 days to 240 days, 240 days to 365 days, or more than 365 days) following implantation. In some embodiments, the cell chamber device is implanted near lymph tissue or mesentery.

In some embodiments, the cell chamber device can be used to treat a subject in need of enzyme replacement therapy (ERT). In such embodiments, the cell chamber can be seeded with cells that express one or more enzymes that are deficient (e.g., absent, non-functional, partially functional, or expressed at sub-optional levels) in the subject. Accordingly, in some embodiments, provided herein is a method of treating a subject in need of enzyme replacement therapy, comprising administering to the subject a cell chamber device disclosed herein. In certain such embodiments, the device can comprise cells that secrete an enzyme that is deficient in the subject.

For example, in some embodiments, provided herein is a method of treating a subject having mucopolysaccharidosis type I (MPS I), comprising administering to the subject a device disclosed herein. In certain such embodiments, the subject having mucopolysaccharidosis type I (MPS I) is administered a device comprising cells that secrete an enzyme, such as laronidase (e.g., SEQ ID NO: 50). In certain embodiments, the device comprises cells that secrete an enzyme comprising the amino acid sequence of SEQ ID NO :50, or an enzyme having at least 90%, 92%, 94%, 95%, 96%, 98%, or 99% to SEQ ID NO 50.

In other embodiments, provided herein is a method of treating a subject having mucopolysaccharidosis type II (MPS II), comprising administering to the subject a device disclosed herein. In certain such embodiments, the subject having mucopolysaccharidosis type I (MPS I) is administered a device comprising cells that secrete an enzyme, such as idursulfase (e.g., SEQ ID NO: 51). In certain embodiments, the device comprises cells that secrete an enzyme comprising the amino acid sequence of SEQ ID NO :51, or an enzyme having at least 90%, 92%, 94%, 95%, 96%, 98%, or 99% to SEQ ID NO :51.

In other embodiments, provided herein is a method of treating a subject having metachromatic leukodystrophy (MLD), comprising administering to the subject a device disclosed herein. In certain such embodiments, the subject having mucopolysaccharidosis type I (MPS I) is administered a device comprising cells that secrete an enzyme, such as arylsulfatase A (e.g., SEQ ID NO: 52). In certain embodiments, the device comprises cells that secrete an enzyme comprising the amino acid sequence of SEQ ID NO :52, or an enzyme having at least 90%, 92%, 94%, 95%, 96%, 98%, or 99% to SEQ ID NO :52.

In some embodiments, the cell chamber device can be used to treat a subject having a lysosomal storage disease, for example, Pompe disease, adult-onset glycogen storage disease II (GSD II), Gaucher disease, Fabry disease, mucopolysaccharidosis type I, mucopolysaccharidosis type II, Niemann-Pick disease (including Type A, Type B, and Type C), Morquio disease (including Type A and Type B), Batten disease, Maroteaux-Lamy disease, metachromatic leukodystrophy disease, Tay-Sachs disease, sphingolipidoses, Hurler disease, or Hunter syndrome. Optionally, the lysosomal storage disease to be treated is characterized by reduced or deficient activity of a lysosomal enzyme in the brain of the patient. Accordingly, in one embodiment, provided herein is a method of treating a subject having a lysosomal storage disease comprising administering to the subject a cell chamber device disclosed herein. In certain such embodiments, the subject having the lysosomal storage disease is administered a cell chamber device comprising cells that secrete a biomolecule that treats the lysosomal storage disease. For example, in some embodiments, the cells in the cell chamber device secrete an enzyme that is deficient or absent in the subject having the lysosomal storage disease, including but not limited to hexosaminidase A, alpha-galactosidase A, glucocerebrosidase, arylsulfatase A, galactocerebrosidase, and sphingomyelinase. In some embodiments, the cells in the cell chamber device secrete an enzyme therapeutic selected from agalsidase beta, agalsidase alfa, imiglucerase, taliglucease alfa, velaglucerase alfa, alglucerase, sebelipase alpha, laronidase, idursulfase, elosulfase alpha, galsulfase, and alglucosidase alpha. The cell chamber device, and related methods, disclosed herein is also useful for treatment of lysosomal storage disorders that feature severe brain involvement. In some embodiments, the cell chamber device delivers a biomolecule across the blood brain barrier. In other embodiments, the cell chamber device is implanted in the brain of the subject.

In some embodiments, the cell chamber device can be used to treat an endocrine and/or metabolism disorder, such as diabetes mellitus, thyroid disorder, or osteoporosis. In one embodiment, provided herein is a method of treating a subject having an endocrine and/or metabolism disorder (e.g., diabetes) comprising administering to the subject a cell chamber device disclosed herein. In certain such embodiments, the subject having the endocrine and/or metabolism disorder (e.g., diabetes) is administered a cell chamber device comprising cells that secrete a biomolecule (e.g., insulin) that treats the endocrine and/or metabolism disorder. For example, in some embodiments, a cell chamber device for treating diabetes comprises cells that secrete insulin. In other embodiments, a cell chamber device for treating thyroid disorders comprises cells that secrete a thyroid hormone, e.g., levothyroxine. In other embodiments, a cell chamber device for treating osteoporosis comprises cells that secrete an antibody that specifically binds RANKL (e.g., denosumab), or an antigen-binding portion thereof.

In some embodiments, the cell chamber device can be used to treat a hematological disorder, such as leukemia, lymphoma, myeloma, anemia, sickle cell anemia or cachexia. In one embodiment, provided herein is a method of treating a subject having a hematological disorder comprising administering to the subject a cell chamber device disclosed herein. In certain such embodiments, the subject having the hematological disorder is administered a cell chamber device comprising cells that secrete a biomolecule that treats the hematological disorder. For example, in some embodiments, the cells in the cell chamber device secrete an antibody, or antigen binding portion thereof, that specifically binds CD20 (e.g., rituximab, obinutuzumab, ofatumumab), CD52 (e.g., alemtuzumab), CD19 (e.g., blinatumomab), CD22 (e.g., inotuzumab), CD38 (e.g., daratumumab), CD33 (e.g., gemtuzumab), or SLAMF7 (e.g., elotuzumab).

In some embodiments, the cell chamber device can be used to treat anemia, for example, anemia associated with chronic kidney disease or end-stage renal disease. In certain such embodiments, the subject having anemia is administered a cell chamber device comprising cells that secrete recombinant erythropoietin (e.g., epoetin alfa, darbpoetin alfa).

In some embodiments, the cell chamber device can be used to treat a cardiovascular disease, such as congestive heart failure, hypertension, cardiomyopathy, myocarditis, atherosclerosis, chronic venous disease, or heart arrhythmia. In one embodiment, provided herein is a method of treating a subject having a cardiovascular disease comprising administering to the subject a cell chamber device disclosed herein. In certain such embodiments, the subject having the cardiovascular disease is administered a cell chamber device comprising cells that secrete a biomolecule known that treats the cardiovascular disease.

In some embodiments, the cell chamber device can be used to treat a respiratory disorder, such as atopic asthma, non-atopic asthma, emphysema, bronchitis, chronic obstructive pulmonary disease, sinusitis, allergic rhinitis, fibrotic lung disease, ARDS, pulmonary vascular disease/pulmonary hypertension, Cor Pulmonale, or cystic fibrosis. In one embodiment, provided herein is a method of treating a subject having a respiratory disorder comprising administering to the subject a cell chamber device disclosed herein. In certain such embodiments, the subject having the respiratory disorder is administered a cell chamber device comprising cells that secrete a biomolecule that treats the respiratory disorder. For example, in some embodiments, the cells in the cell chamber device secrete an antibody, or antigen-binding portion thereof, that specifically binds IL-4α (e.g., dupilumab), IL-5 (e.g., mepolizumab), IgE (e.g., omalizumab), IL-5 (e.g., reslizumab), or IL-5Rα (e.g., benralizumab).

In some aspects, a durable clinical remission, for example, a clinical remission which is sustained through at least two, at least three, at least four visits with a caretaking physician within a six-month or one-year period after beginning treatment, may be achieved following treatment with the cell chamber device herein. In some aspects, a durable clinical response, for example, a clinical response which is sustained for at least 6 months, at least 9 months, at least a year, after the start of treatment, may be achieved following treatment with the cell chamber device herein.

The disclosure is further illustrated by the following examples. The examples provided are for illustrative purposes only, and should not be construed as limiting the scope or content of the disclosure in any way.

### EXAMPLES

### Example 1: Fabrication of an Electrospun Cell Chamber

The following method was used to construct a cell chamber device using electrospun polymers. This exemplary cell chamber device contains three nanofibrous electrospun polymer layers, and a central polyester membrane. The following methods can be adapted to fabricate a device that contains additional layers of electrospun polymer, fewer layers of electrospun polymer, or layers of different electrospun polymer(s). The method can also be adapted to fabricate a device that lacks a central polyester membrane, if desired.

A first borosilicate vial was prepared containing polyethylene terephthalate (PET) and polybutylene terephthalate (PBT) chips, and hexafluoroisoproposal (HFIP). A second borosilicate vial was prepared containing polyurethane (PU) chips (either polycarbonate, polyether, or polyester-based) and HFIP. Vials were placed on a rotator set at 45 RPM or higher until the chips were dissolved.

The PET-PBT solution was loaded into a 10 mL syringe, that was connected to the pump on an espin unit. Polyester film containing 0.2 µm to 2 µm pores was secured to the mandrel. The size of the mandrel and/or the film can be adjusted based on the desired size of the scaffold. A layer of nanofibrous, electrospun PET-PBT was applied to the film using an e-spinning distance of 10-20 cm, an e-spinning voltage of 20-23 kV, and a rotation speed of 15-150 RPM.

The film was removed from the mandrel and turned over, such that the uncoated side was facing outward. A layer of nanofibrous, electrospun PET-PBT was applied to the uncoated side of the film. A syringe loaded with the PU solution was connected to the espin unit, and a layer of nanofibrous electrospun PU was applied on top of the nPET-PBT layer, on one side of the film. In this manner, a trilayer scaffold containing a central film/membrane was created (nPET-PBT//polyester membrane//nPET-PBT//PU). The mandrel was rinsed by immersion in ethanol followed by sonication. The scaffold was then rinsed with distilled water, allowed to dry, and removed from the mandrel.

The scaffold was cut to a 50 mm x 40 mm sheet. The size of the scaffold can be increased or decreased depending on the desired size of the cell chamber. The scaffold was folded in half, with the nPU layer facing in, to form the inner surface of the cell chamber. Three edges were ultrasonically welded using a Sonobond SeamMaster unit. The remaining edge was trimmed to a point by cutting the scaffold on a diagonal from the central point of the unsealed edge to a position part way down each sealed edge. The resulting edges were sealed by ultrasonic welding, such that the cell chamber had five sealed sides. The central point was clipped to create a small opening in the chamber that can serve as a cell loading port, which may be re-sealed after loading. Scaffolds may be treated with ethyldiamine to render positive charge to polymers, or treated with sodium hydroxide to render negative charges to polymers. To reduce inflammation due to implantation trauma, the cell scaffold device can also optionally be loaded with tacrolimus, as described in Example 8.

### Example 2: Development of Vedolizumab / Luc-ARPE-19 Cells

ARPE-19 cells were selected as an exemplary cell type for loading into the cell chamber device in the following Examples. In this Example, ARPE-19 cells were engineered to secrete vedolizumab (e.g., for delivery into a subject when loaded into the cell chamber device). The cells were also engineered to express luciferase to enable monitoring of cells *in vitro* and *in vivo.*

Two expression constructs were introduced into the ARPE-19 cells. One expression construct encoded the vedolizumab heavy chain, while the second expression construct encoded luciferase and the vedolizumab light chain. Both the vedolizumab light chain and heavy chain were under the control of a human elongation factor-1 alpha (EF-1a) constitutive promoter. Clones were also engineered that just expressed vedolizumab (without luciferase). Cells were then grown and tested for the level of vedolizumab expression (see Table 1).

**Table 1: Vedolizumab Expression**

| | **Vedolizumab-ARPE-19 Clone** | **Vedolizumab / Luciferase - ARPE19 Clone 1** | **Vedolizumab / Luciferase - ARPE19 Clone 2** |
|---|---|---|---|
| **Vedolizumab ELISA** | 41.5 picogram/cell/day (PCD) | 12.6 PCD | 28.5 PCD |
| **Fc ELISA** | 45.2 PCD | 11.3 PCD | 27.8 PCD |

Vedolizumab-ARPE19 clones were isolated that express ~40 picograms/cell/day. Additionally, AREP19 cells that express both vedolizumab and luciferase were isolated (vedolizumab/Luc-ARPE19 clones) for use in Examples described further herein.

Based on the amount of vedolizumab secreted per cell per day and the estimated number of cells per cm² of scaffold, a simulated model was prepared to estimate the plasma concentration levels of vedolizumab that could be attained over time. It was estimated that 625,000 cells/cm² were grown on the scaffold. 625,000 cells/cm² secreting 45 picograms of vedolizumab per day would secrete approximately 28 µg vedolizumab/cm² daily. An 8 x 10 cm device would accordingly secrete about 4.5 mg vedolizumab/day. As shown in the simulation in Fig. 2, such a device would be predicted to reach a therapeutic plasma concentration of vedolizumab (17 µg/mL) within 60 days of implantation.

### Example 3: In vitro cell seeding assay with Vedolizumab / Luc-ARPE-19 cells

ARPE-19 cells that express vedolizumab and luciferase (vedolizumab/Luc-ARPE19 cells) were seeded a 16 mm disc composed of an outer layer of polyethylene terephthalate - polybutylene terephthalate (nPET-PBT), and an inner layer of polyurethane (PU).

To assess whether cells could grow on the inner PU layer of the scaffold, dolizumab/Luc-ARPE19 cells were stained with CellTrackerOrange (5uM) and 250,000 prestained cells were seeded onto the PU side of nPET-PBT/PU (16mm disc). The discs were then observed by fluorescence imaging 24 hours post-seeding. As shown in Fig. 3A, cells grew on the PU side and have a monolayer appearance. No cells were detected on the outer nPET-PBT side of the scaffold. Further, no cell migration was observed from the PU side to the PBT-PET side.

The number of cells loaded into the nPET-PBT/PU disc was compared to the number of cells that could be loaded onto a tissue culture (TC) plate. Images of cells on the tissue culture plate and the nPET-PBT/PU disc are shown in Fig. 3B, with the top row showing CellTracker Orange fluorescence in the cytosol in cells in each condition, and the bottom row showing Hoechst 33342 stained nuclei. The number of cells in the nPET-PBT/PU disc as compared to the tissue culture plate was determined by quantifying the amount of DNA isolated from cells in each condition. As shown in Fig. 3C, the amount of DNA isolated from cells grown on nPET-PBT/PU was 5 times higher than that isolated from cells on the TC plate, indicating that at least 5 times more cells can be cultured on nPET-PBT/PU than a tissue culture plate. Further, comparison of the amounts of vedolizumab secreted in each condition showed that the cells on the nPET-PBT/PU membrane secreted five times more vedolizumab than cells on a tissue culture plate (Fig. 3D).

Vedolizumab/Luc-ARPE19 cell seeding and vedolizumab secretion by said cells was then assessed on nPET-PBT with charged surface modifications. Discs comprising nPET-PBT/PU were treated with liquid ethylenediamine (nPET-PBT (EDA)) to generate a positively charged surface or liquid sodium hydroxide to generate a negatively charged surface (nPET-PBT (HYD)). Vedolizumab/Luciferase expressing ARPE19 clone were seeded onto discs comprising nPET-PBT/PU, nPET-PBT, nPET-PBT(EDA), or nPET-PBT(HYD) at the density of 1 million cells/16 mm discs. The cells were maintained in DMEM/F-12 medium supplemented with 10% FBS under the conditions of 5% CO2 in 37°C incubator, and the medium was changed with fresh one every three to four days. Three weeks post seeding, Vedolizumab secretion (Fig. 4A) and luminescence intensity (Fig. 4B) were evaluated by Vedolizumab ELISA and IVIS imaging, respectively. As shown in Figs. 4A and 4B, Vedolizumab / Luc-ARPE-19 cells could be seeded onto surface-modified materials and the cells maintained vedolizumab secretion.

Subsequently, the cytokine secretion profile of vedolizumab/Luc-ARPE19 cells grown on charge modified or unmodified nPET-PBT was assessed. Vedolizumab/Luc-ARPE19 cells were seeded on discs comprising nPET-PBT/PU, nPET-PBT, nPET-PBT(EDA), or nPET-PBT(HYD), as described above. Three weeks post-seeding, cytokine secretion by the vedolizumab/Luc-ARPE19 cells was assessed. The data was normalized by the secretion data from the cells seeded on nPET-PBT/PU membranes. As shown in Table 2, there was only a 0.5 to 1.2-fold change in cytokine secretion from the ARPE-19 cells, indicating that the charged surface modifications did not have effects on cell function.

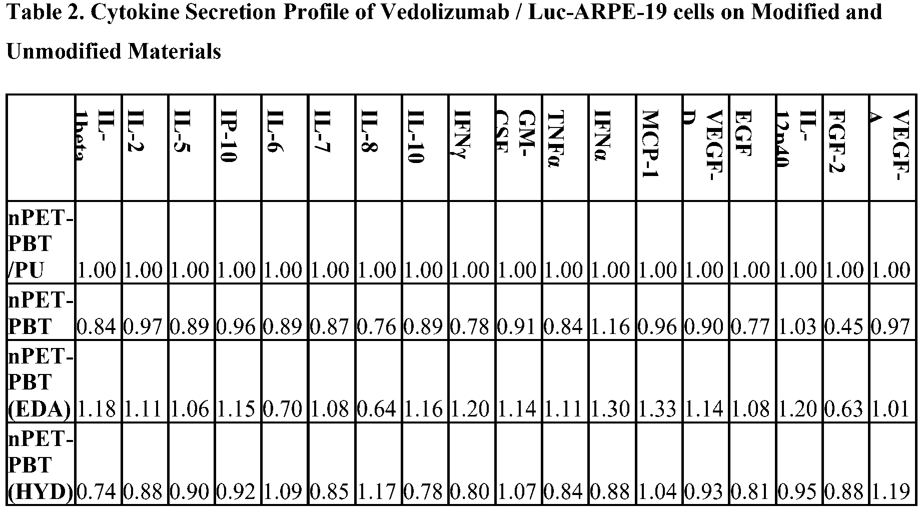

### Example 4: In vitro and in vivo cell distribution of Vedolizumab / Luc-ARPE-19 cells in a nanofibrous polymer cell chamber device

Two cell chamber devices were tested in this study. The first device contained a scaffold comprising an outer layer of nPET-PBT and an inner layer of PU surrounding a cell chamber. This device is depicted in Fig. 1C. The other device contained the same nPET-PBT/PU scaffold, and in addition, a second scaffold positioned inside the cell chamber, comprising nanofibrous polybutylene (nPBT) modified with holes. This device is depicted in Fig. 1D. To assess the distribution of cells in each device *in vitro,* the cell chambers were loaded with vedolizumab/Luc-ARPE19 cells. Cell distribution was assessed by luminescence imaging for 4 days after cell seeding. As shown in Fig. 5A, the cells were distributed over nPET-PBT/PU cell chambers in both devices, i.e., with and without an inner nPBT scaffold.

To assess the viability and distribution of cells on nPET-PBT/PU chambers with and without an inner nPBT scaffold following *in vivo* transplantation, both chambers were implanted in BALB/c nude mice (subcutaneous, near shoulders). Mice were assessed by luminescence imaging and blood collection twice per week over the course of 62 days. Luminescence imaging was performed by injecting D-Luciferin/PBS solution (15 mg/mL, 100 µL/10g) intraperitoneally followed by imaging the mice 10 minutes after injection. As shown in Figs. 5B and 5C, which depicts the luminescence intensity from the implanted chambers as a function of days after implant, cells were alive inside cell chamber devices with and without an inner nPBT scaffold for over 65 days or 90 days, respectively. Further, as shown in Fig. 5D, vedolizumab was detected by western blot (primary antibody: Goat anti-human antibody, Fc region specific; Secondary antibody: rabbit anti-goat antibody, HRP conjugated) in mice transplanted with either chamber device, 90 days after implantation.

To assess whether cells had leaked from the chambers, D-luciferin was intraperitoneally injected into mice transplanted with the cell chambers, and IVIS imaging was performed 8 minutes after the injection. The mice were then euthanized and the chambers were removed, after which IVIS imaging was performed again to determine whether cells were located on the back of the mice. As shown in Fig. 5E, there was no detectable luminescence signal on the backs of the mice following removal of the chambers, indicating that vedolizumab/Luc-ARPE19 cells had not leaked from the chambers. Explanted chambers removed 42 days after transplant were assessed. Explanted chambers continued to secrete vedolizumab, as shown in Table 3. In addition, as shown in Figs. 5F and 5G, no significant fibrotic response was observed visually.

**Table 3. Vedolizumab secretion from explanted chambers**

| | **Vedolizumab Secretion** |
|---|---|
| nPET-PBT/PU chamber with inner nPBT scaffold (Cage 1, #2) | 4.56 µg/day |
| nPET-PBT/PU chamber with inner nPBT scaffold (Cage 1, #3) | 8.34 µg/day |
| nPET-PBT/PU chamber with inner nPBT scaffold (Cage 3, #2) | 2.94 µg/day |
| nPET-PBT/PU chamber without inner nPBT scaffold (Cage 3, #2) | 1.02 µg/day |

To assess the inflammatory response to various scaffold materials over an extended time period, mice were implanted with either (i) a scaffold comprising a solid (non-nanofibrous) PET sheet having 0.4 µm pores, coated on one side with electrospun nPET-PBT, and on the other side with electrospun nPU, or (ii) a scaffold comprising a solid PET sheet having 0.4 µm pores, that was not coated with nanofibrous polymers. The scaffolds were maintained in mice for 41 days following implantation, at which time the histology of the area surrounding the implant was assessed by H&E staining. Fig. 5H (left panel) shows the area surrounding the first scaffold, containing a solid PET sheet coated with nPET-PBT and nPU. Fig. 5H (right panel) shows the area surrounding the second scaffold, containing a solid PET sheet without nanofibrous coating. As shown in Fig. 5H, the inflammatory response to the nanofibrous scaffold material was significantly reduced, relative to the scaffold that lacks a nanofibrous polymer coating.

### Example 5: In vitro cell attachment assay with Vedolizumab / Luc-ARPE-19 cells

To assess the attachment of cells on surface-modified materials, ARPE-19 cells engineered to express luciferase (Luc-ARPE-19 cells) were assessed for cell attachment to disks composed of nPET-PBT, nPET-PBT(EDA), or nPET-PBT(HYD). Cell attachment to a normal tissue culture (TC) plate was assessed as a control. 400,000 cells were seeded per disc of material, and the percentage of attached cells was assessed at multiple time points after seeding.

As shown in Fig. 6, ARPE-19 cells rapidly attached to all three electrospun surfaces in a time-dependent fashion. Charged materials showed comparable attachment to tissue culture plastic, with all of the materials loading 50-60% of cells within 60 minutes. However, charged materials (nPET-PBT(EDA) or nPET-PBT(HYD)) had greater cell loading (90% attached cells) than unmodified nPET-PBT after 180 minutes of incubation.

These results indicate that cells rapidly attached to the electrospun materials in a time-dependent fashion. In addition, charged surface modifications improved the amount of cell loading over time. The short time requirement for cell loading and attachment allows the cell chamber to be loaded with cells on the day of implantation.

### Example 6: In vivo implant assay with Vedolizumab / Luc-ARPE-19 cells

To test the retention of the cells on the electrospun scaffold materials *in vivo,* 16 mm membrane discs composed of nPET-PBT, nPET-PBT(EDA), or nPET-PBT(HYD) were seeded with cells that stably express vedolizumab and luciferase (vedolizumab/Luc-ARPE19 cells; 400,000 cells per 16 mm disc) and implanted into a female Nude mouse by subcutaneous implantation. The discs used in this experiment do not comprise an enclosed chamber. Administration of 100 µl vedolizumab/Luc-ARPE19 cells by subcutaneous injection was assessed as a control. Cells were loaded into each disc on the day of implantation. Twenty-four or four hours after loading, the scaffolds were administered to mice by subcutaneous implantation (n= 4 mice). Luminescence was monitored by live cell imaging every 3-4 days over 70 days to determine the degree of cell retention. As shown in Figs. 7A-7E, ARPE-19 cells on the electrospun materials had a longer retention time at the implantation site than subcutaneously injected cells.

### Example 7: In vitro cell seeding assay with Vedolizumab / Luc-ARPE-19 cells on tacrolimus-treated nPET-PBT

To reduce inflammation due to implantation trauma, the cell scaffold device can be loaded with tacrolimus (FK506). To assess cell seeding and biomolecule secretion on tacrolimus-treated materials, electrospun nPET-PBT was loaded with tacrolimus by dissolving 0% tacrolimus, 2% tacrolimus, or 4% tacrolimus to the polymer solution prior to electrospinning.

Incorporation of tacrolimus into nPET-PBT was assessed by solvent extracting the tacrolimus from the electrospun materials. The solvent extracted solution was then analyzed by high performance liquid chromatography (HPLC). As shown in Fig. 8A (tacrolimus alone) and Fig. 8B (tacrolimus following extraction), HPLC analysis indicated that there was no drug degradation following loading of tacrolimus onto electrospun nPET-PBT.

To assess the bioactivity of tacrolimus-loaded nPET-PBT, an *in vitro* T cell activation assay was performed, in which T cells were incubated with medium that had been exposed to tacrolimus-loaded nPET-PBT. Electrospun nPET-PBT was soaked in 400 µL of RPMI1640 supplemented with 10% Fetal bovine serum (FBS) and incubated for 24 hours (Initial samples). The nPET-PBT was then transferred into pre-warmed fresh medium and incubated for 24 hours (second sample). The nPET-PBT was transferred again into pre-warmed fresh medium and incubated for 24 hours (third sample). Each medium sample was collected for drug concentration evaluation by ELISA/HPLC. Human PBMCs were incubated with the collected medium samples for 30 minutes. Subsequently, the human PBMCs were activated with CD3/CD28 activation Dynabeads for 48 hours. The supernatant from the T-cell culture medium was then isolated and tested for IL-1β production. As shown in Fig. 8C, medium exposed to tacrolimus-loaded nPET-PBT inhibited T-cell activation, as measured by IL-1β levels, indicating that tacrolimus maintained efficacy after loading on electrospun materials and that tacrolimus was released gradually over three days (Fig. 8C).

Next, an *in vitro* assay was performed to evaluate if tacrolimus-loaded, electrospun nPET-PBT impacted cell growth and vedolizumab secretion by vedolizumab/Luc-ARPE19 cells. nPET-PBT with or without tacrolimus was seeded with vedolizumab/Luc-ARPE19 cells (200,000 cells / condition). Cells were grown on tacrolimus-loaded nPET-PBT discs over three days, after which the surfaces of the nPET-PBT material were visually inspected by fluorescence imaging. As shown in Fig. 8D, tacrolimus had no detectable effect on cell proliferation or growth. Cells were further assessed three weeks post-seeding. As shown in Figs. 8E and 8F, Vedolizumab / Luc-ARPE-19 cells could be seeded onto tacrolimus-loaded nPET-PBT and the cells maintained vedolizumab secretion. These results indicate that tacrolimus had no off-target effects on the vedolizumab/Luc-ARPE19 cells.

Subsequently, the cytokine secretion profile of vedolizumab/Luc-ARPE19 cells grown on nPET-PBT with or without tacrolimus was assessed. Cells were grown on tacrolimus-treated nPET-PBT over three days, as described above. Three weeks post-seeding, cytokine secretion was evaluated for cells seeded on each material. The data was normalized by the secretion data from the cells seeded on nPET-PBT membranes without tacrolimus treatment. As shown in Table 4, there was only a 0.5 to 2-fold change in cytokine secretion from the ARPE-19 cells, indicating that tacrolimus did not have effects on cell function.

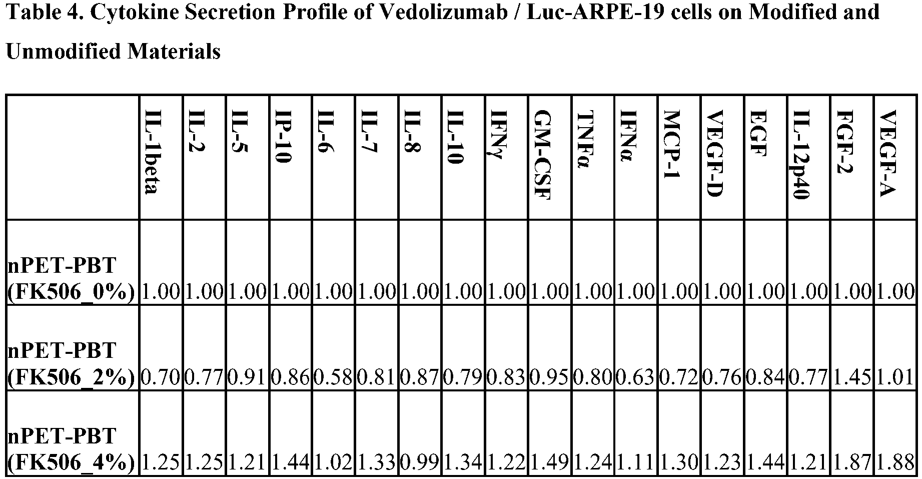

### Example 8: In vitro cell seeding assay with Adalimumab / ARPE-19 cells and Ustekinumab / ARPE-19 cells

ARPE-19 cells were generated to express and secrete adalimumab (adalimumab/ ARPE19 cells) or ustekinumab (ustekinumab/ARPE19 cells), in the same manner described above for the production of ARPE-19 cells secreting vedolizumab.

Adalimumab/ARPE19 cells or ustekinumab/ARPE19 cells were seeded onto 16 mm discs comprising an outer layer of polyethylene terephthalate - polybutylene terephthalate (nPET-PBT), and an inner layer of polyurethane (PU).

Biomolecule (i.e., adalimumab or ustekinumab) secretion by cells loaded onto nPET-PBT/PU was compared to the number of cells that could be loaded onto a tissue culture (TC) plate. As shown in Figs. 9A and 9B, cells on the nPET-PBT/PU membrane secreted more adalimumab (Fig. 9A) or ustekinumab (Fig. 9B) than cells on a tissue culture plate.

### Example 9: Functional activity of antibodies secreted using a cell chamber device

The functional activity of antibodies secreted by ARPE-19 cells was assessed by an in vitro assay. ARPE-19 cell secreting adalimumab, ustekinumab, or vedolizumab (Adalimumab-ARPE-19, Ustekinumab-ARPE-19, and Vedolizumab-ARPE-19) were seeded into cell culture flasks at the density of 50,000 cells/cm^2 in SFM4MegaVir media and cultured for 15 days. The amount of each antibody secreted by cells was determined based on Adalimumab ELISA, Ustekinumab ELISA, and Vedolizumab ELISA assays (see Table 1 and Table 5). The cell culture medium comprising antibodies secreted by the cells was assayed for antibody functional activity, as outlined below.

Adalimumab is an anti-TNFα antibody. To assay for adalimumab activity in the cell culture medium of adalimumab/ARPE-19 cells, L-929 cells were seeded into a 96 well cell culture plate at the density of 5,000 cells/well in DMEM supplemented with 10% FBS. After 20-hour incubation, the cells were sensitized with Actinomycin D (2 µg/mL) for two hours. Then the cells were incubated with culture medium comprising antibodies secreted by the ARPE-19 cells in the presence of human TNFα (1 ng/mL). After another 20-hour incubation the cells were evaluated for cell viability by a CCK-8 assay to test for neutralization of human TNFα activity. As shown in Fig. 10A, these results show that adalimumab secreted by ARPE-19 cells retained TNFα neutralizing activity.

Vedolizumab is an anti-α4β7 integrin antibody. To assay for vedolizumab activity in the cell culture medium of 3 vedolizumab-expressing ARPE-19 clones and a vedolizumab/Luciferase-expressing ARPE-19 clone, an α4β7 integrin binding assay with HuT-78 cells that specifically express α4β7 integrin was performed using flow cytometry. The cells were cultured for two weeks in SFM4MegaVir media and these medium samples were used for a binding assay after the determination of vedolizumab concentration by vedolizumab ELISA. The binding activities of the conditioned medium were evaluated by competitive assay with fluorescently labeled-purified vedolizumab (R-PE labeled MLN002) secreted from CHO cells. As shown in FIG. 10B, Vedolizumab secreted by ARPE-19 cells inhibited the binding of R-PE labeled MLN002. These results show that vedolizumab secreted by ARPE-19 cells were functional and bound α4β7 integrin as well as vedolizumab derived from CHO cells.

**Table 5. Ustekinumab and Adalimumab Expression in ARPE-19 Cells**

| **·** | **FcELISA** | **Ustekinumab ELISA** | **Adalimumab ELISA** |
|---|---|---|---|
| **Parental** | - | - | - |
| **Ustekinumab Clone1** | + (30.8 Picogram/Cell/Day (PCD)) | + (30.7 PCD) | - |
| **Adalimumab Clone 1** | + (10.0 PCD) | - | + (19.0 PCD) |
| **Adalimumab Clone 2** | + (57.0 PCD) | - | + (70.3 PCD) |

### Example 10: In vitro expression assay with Vedolizumab / Luc-ARPE-19 cells on a nPET-PBT/PET/nPU cell chamber device

In this Example, *in vitro* production of vedolizumab was assessed in a cell chamber device seeded with different densities of ARPE-19 cell stably expressing vedolizumab and luciferase. The cell chamber device contained a scaffold comprising an outer layer of nPET-PBT and an inner layer of nPU with a porous, non-nanofibrous PET membrane (0.4 µm pore size) between the inner and outer layers (nPET-PBT/PET/nPU cell chamber device, as shown in Figs. 1E-1G and as further described below). As a comparator, a Theracyte immunoisolation device was assessed, which does not include nanofibrous materials and instead includes a woven polyester outer layer, a vascularizing membrane (with 5 µm PTFE pores), a non-woven polyester insert, and an immunoisolating membrane with 0.45 µm PTFE pores. The Theracyte device is further described, for example, in Rafeal, et. al., (1999). European surgical research, 31(3), 249-258.

To generate the nPET-PBT/PET/nPU cell chamber device, nanofibrous polymer membranes were engineered by electrospinning (see, e.g., Huang, et al. Composites Science and Technology (2003). 63:2223-2253) and assembled by ultrasonic welding. The membrane had a trilaminate scaffold including an outer layer of nanofibrous polyethylene terephthalate (PET) and polybutylene terephthalate (PBT) (nPET-PBT), commercially available non-fibrous PET membrane with 0.4 um pores in the middle, and an inner internal-facing layer comprising electrospun nanofibrous polyurethane (nPU) polymers (nPET-PBT/PET/PU). The flat sheet of nPET-PBT/PET/nPU was cut out and sealed using an ultrasonic cutting/sealing process. After assembly, an air-leak test was performed as a quality control. To facilitate the loading of the cells, blunt needles (23G) were inserted at a loading port, and the device was sterilized by E-beam in individual sterilizing packets. The structure of the nPET-PBT/PET/PU device is illustrated in Fig. 1E. SEM images of each membrane in the device are shown in Fig. 1F, and images of the chamber 44 days after cell loading as visualized by H & E staining are shown in Fig. 1G.

ARPE-19 cells stably expressing vedolizumab and luciferase were loaded into the nPET-PBT/PET/nPU chambers at three different densities: 2.5 million, 5.0 million, and 10 million cells/chamber. A day after cell loading, the chambers were incubated in fresh MegavirSFM for two hours followed by optical Imaging with an IVIS Spectrum Imaging platform (PerkinElmer) by placing the chambers into culture medium containing 150 µg/ml D-luciferin. Luminescent images of the cell chambers in each condition are shown in Fig. 11A. The data were analyzed with Living Image Software (PerkinElmer) by delimiting a constant region of interest (ROI) around the device and quantifying total radiance in photon/sec to assess the linearity of luminescence intensity vs cell numbers (Fig. 11B). Further, the vedolizumab concentration in the medium was assessed by a vedolizumab ELISA assay to assess the linearity of vedolizumab secretion vs cell numbers (Fig. 11C). This data demonstrates that ARPE-19 cells express detectable levels of luminescence when cultured within the cell chamber, and actively secrete vedolizumab from the chamber into the culture medium.

### Example 11: In vivo production of vedolizumab in a cell chamber device

In this Example, *in vivo* production of vedolizumab was assessed in a cell chamber device implanted in immune compromised mice. The cell chamber device contained a scaffold comprising an outer layer of nPET-PBT and an inner layer of nPU with a porous, non-nanofibrous PET membrane (0.4 µm pore size) between the inner and outer layers (nPET-PBT/PET/nPU cell chamber device,), as further described in Figs. 1E-1G and in Example 10. As a comparator, a Theracyte immunoisolation device was also assessed, as described in Example 10.

To assess cell viability and production of vedolizumab and/or luciferase from each cell chamber device *in vivo,* the nPET-PBT/PET/nPU cell chamber device and the Theracyte cell chamber device were loaded with vedolizumab/Luciferase-expressing ARPE19 cells or vedolizumab-expressing ARPE19 cells and subcutaneously implanted in immune compromised mice lacking T cells (i.e., nude mice). Mice were assessed by luminescence imaging to assess the cell viability inside the cell chamber devices. *In vivo* imaging was performed using the IVIS Spectrum Imaging platform (PerkinElmer) to detect luminescence. Filtered 150mg/kg D-luciferin in sterile PBS was intraperitoneally injected into the mice. Ten minutes after injection, mice were sequentially scanned in the imaging chamber under isoflurane anesthesia for 30 minutes with one minute intervals. Fig. 12A shows luminescent images taken at peak signal for nude mice implanted with nPET-PBT/PET/nPU cell chambers (top row) or Theracyte devices (bottom panel). As shown in Fig. 12A, cells were viable and remain localized to the implantation site of the cell chamber device.

The level of luminescence in each mouse was analyzed with Living Image Software (PerkinElmer) by delimiting a constant region of interest (ROI) around the cell chamber device and quantifying total radiance in photon/sec. As shown in Fig. 12B, which depicts the luminescence intensity from the implanted chambers as a function of days after implant, high steady state luminescence signals indicated that the cells survived and produced luciferase inside both the nPET-PBT/PET/nPU cell chamber device and the Theracyte device for over 100 days. ARPE-19 cells were also visible following H&E staining or Masson's Trichrome Staining of explanted nPET-PBT/PET/nPU cell chamber devices 30 days and 64 days following implantation in mice (Fig. 12E).

To assess the ability to deliver biologics into the blood flow, vedolizumab concentration in plasma of mice implanted with the nPET-PBT/PET/nPU cell chamber device or the Theracyte device was tested by vedolizumab-ELISA assay. Each cell chamber device was seeded with vedolizumab/Luciferase-ARPE19 cells (Fig. 12B) or vedolizumab-ARPE19 cells (Fig. 12C). Whole blood was collected from the tail of mice in each condition once or twice a week following implantation. As shown in Fig. 12B, the average vedolizumab concentration in plasma from mice implanted with the nPET-PBT/PET/nPU cell chamber device reached a target plasma concentration of 17 ug/mL vedolizumab by Day 14 and maintained a concentration at or above 17 ug/mL for 30 days. In contrast, vedolizumab was not detected (less than 1 ug/mL) in the plasma of mice implanted with the Theracyte device including vedolizumab/Luciferase-ARPE19 cells (Fig. 12C). Mice implanted with cell chamber devices seeded with ARPE-19 clones that produce higher levels of vedolizumab reached a target plasma concentration of 17 ug/mL vedolizumab by Day 3 and maintained a concentration at or above 17 ug/mL for 50 days (Fig. 12D).

To assess the inflammatory response to various scaffold materials, integration into the host tissue, migration of the host tissue into the chambers, and the presence of ARPE-19 cells inside the chambers over an extended time period, the histology of the area surrounding each cell chamber device was assessed by H&E staining or Masson's Trichrome staining cell 30 days and 64 days following implantation. As shown in Fig. 12E, the inflammatory response to the nanofibrous scaffold material was minimal to none, and the host tissue integration into the outer layer, nanofibrous electrospun PET-PBT, with neovascularization (yellow allow) was observed. No cells were seen in the solid (non-fibrous) PET sheet, which indicate that there was no host cell migration into the chambers. The cell layer between the nanofibrous PU layers indicated the presence of ARPE-19 cells.

To assess the barrier function of the scaffold against host cell migration into the chambers, Nude mice were implanted with the nPET-PBT/PET/nPU or Theracyte cell chamber devices without cells inside the cell chamber. The scaffolds were maintained in mice for 60 days following implantation, at which time the histology of the area surrounding the implant was assessed by H&E staining. As shown in Fig. 12F, no cells (e.g., host cells) were observed inside the cell chamber device 60 days post-implantation. This data indicates the scaffold blocked the migration of the host cells migration into the cell chamber.

To visually assess the neo-vascularization around the scaffold and fibrotic reaction against the scaffold, the area surrounding the nPET-PBT/PET/nPU or Theracyte cell chamber devices seeded with vedolizumab/Luciferase-expressing ARPE19 cells was photographed 30 days and 64 days post-implantation. As shown in Fig. 12G (top row and 1^{st} and 4^{th} image in second row), blood vessel structures were seen surrounding the both the nPET-PBT/PET/nPU and Theracyte cell chamber devices. The nPET-PBT/PET/nPU cell chamber device could be removed easily, suggesting that there was no significant fibrotic response. In contrast, the Theracyte cell chamber device adhered to the surrounding tissue and displayed evidence of tissue site reactions surrounding the implant. These results indicate that the nPET-PBT/PET/nPU cell chamber device exhibited greater biocompatibility and reduced fibrotic response *in vivo* as compared to the Theracyte device.

To assess cell leakage from the device, *in vivo* imaging was performed pre and post removal of the nPET-PBT/PET/nPU or Theracyte cell chamber devices with the IVIS Spectrum imaging platform (PerkinElmer). Filtered 150mg/kg D-luciferin in sterile PBS was intraperitoneally injected into the mice. 30 minutes after injection, the mice were scanned in the imaging chamber under isoflurane anesthesia. Mice were then euthanized and the chambers were removed, after which IVIS imaging was performed again to determine whether cells were located on the back of the mice. The images were analyzed with Living Image Software (PerkinElmer) by delimiting a constant region of interest (ROI) around the device and quantifying total radiance in photon/sec. There was no detectable luminescence signal on the backs of the mice following removal of the chambers, indicating that vedolizumab/Luc-ARPE19 cells had not leaked from the chambers (nPET-PBT/PET/nPU Replicate #1:0.02%, nPET-PBT/PET/nPU; Replicate #2: 0.16%, Theracyte Replicate #1: 0.01%, Theracyte Replicate #2: 0.01%) indicating there was no active cell migration/leakage from chambers/devices to outside (Fig. 12H).

### Example 12: In vivo production of adalimumab or ustekinumab in a cell chamber device

To assess the ability to deliver biologics into the blood flow and the applicability of the devices to other biologics, secretion of adalimumab or ustekinumab from the nPET-PBT/PET/nPU cell chamber device described in Example 10 was assessed *in vivo.* The nPET-PBT/PET/nPU cell chamber device was seeded with ARPE19 cells stably expressing adalimumab or ustekinumab (see also Example 8) and subcutaneously implanted in Nude mice. Mice were assessed by blood collection over the course of >100 days to measure the plasma concentration of each antibody over time. Human IgG concentration in plasma was tested by an ELISA assay which detected the human IgG Fc region. As shown in Fig. 13A, the average adalimumab plasma concentration reached a plateau of approximately 50-100 µg/ml around Day 25 and maintained a steady-state over 80 days post-implantation. (Day1-Day60: N=5, Day61-Day81: N=4, Day82-92: N=3, Day93-Day108: N=2). Similarly, the average ustekinumab plasma concentration reached a plateau of approximately 60-110 µg/mL around Day 20 and maintained a steady-state over 80 days (N=5).

### Example 13: Three dimensional cell culture using a cell chamber device

The following experiments were performed to assess the ability of the cell chamber device to preserve the superstructure of tissues or three-dimensional cell cultures.

Intestine from donor rats was harvested, flushed, and cut into 1 cm length sections. Intestine segments were inserted into 1.5 cm tubular chambers comprising a single layer of electrospun nPET-PBT. Sealed chambers were implanted into subcutaneous pockets in the rat dorsum (n = 4 pockets/rat). After 7 and 28 days, rats were sacrificed (n = 2-4 segments per test group), and gross assessments and histology assessments of the chambers was performed. This workflow is illustrated in Fig. 14. In addition, a parallel experiment was performed in which intestine segments were implanted in like manner, without the use of a cell chamber device.

In rats implanted with the intestine implant, absent enclosure in the cell chamber device, complete loss of the intestine structure was observed, and intense collagen deposition indicative of a fibrotic response was evident in the area surrounding the implant.

In rats implanted with the intestine segments encapsulated by a non-drug loaded cell chamber device, intestinal cells were present within the electrospun material 7 and 28 days following administration. Some loss of intestine structure and tissue architecture was observed within the chamber. Minimal fibrotic response to the chamber material was observed. Representative images are provided in Fig. 15.

The foregoing experiment was repeated, using identical cell chambers in which the scaffold of the cell chamber had been loaded with Pirfenidone, Roflumilast, or Tacrolimus. The three-dimensional superstructure of intestine was preserved within Roflumilast-loaded, Pirfenidone-loaded, and Tacrolimus-loaded cell chambers, at both 7 and 28 days post-implantation. Notably, villi within intestine segments remained visible after 28 days, and the intestinal wall was also present. In addition, there was evidence of fibrosis being curtailed in and around the implanted materials. Representative images are provided in Fig. 16.

This data indicates that the electrospun cell chamber device shields intestinal implants from a host response. Addition of an anti-inflammatory agent to the electrospun polymer can additionally prevent fibrosis and resorption of the implanted tissue, and promote the maintenance of the three dimensional organization and superstructural features of the tissue.

### EQUIVALENTS

Those skilled in the art will recognize, or be able to ascertain using no more than routine experimentation, many equivalents to the specific embodiments of the invention described herein. Such equivalents are intended to be encompassed by the following claims. The contents of all references, patents and published patent applications cited throughout this application are incorporated herein by reference.

### THE FOLLOWING ITEMS FORM PART OF THE PRESENT DISCLOSURE

1. A device comprising a multilayer scaffold surrounding a cell chamber, wherein the multilayer scaffold comprises an outer layer and an inner layer in contact with the cell chamber, and wherein the outer layer and the inner layer each comprise a nanofibrous polymer.
2. The device of item 1, wherein the outer layer comprises nanofibrous polyethylene terephthalate and polybutylene terephthalate.
3. The device of item 1, wherein the outer layer comprises electrospun polyethylene terephthalate and polybutylene terephthalate.
4. The device of any one of items 1-3, wherein the inner layer comprises nanofibrous polyurethane.
5. The device of item 4, wherein the inner layer comprises electrospun polyurethane.
6. The device of any one of items 1-3, wherein the inner layer comprises nanofibrous polyethylene terephthalate and polybutylene terephthalate.
7. The device of any one of items 1-3, wherein the inner layer comprises electrospun polyethylene terephthalate and polybutylene terephthalate.
8. The device of any one of the preceding items, wherein the outer layer and/or the inner layer comprises one or more charged surface modifications.
9. The device of item 8, wherein the outer layer and/or the inner layer have a net positive charge.
10. The device of item 9, wherein the scaffold has been treated with ethylenediamine.
11. The device of item 8, wherein the outer layer and/or the inner layer have a net negative charge.
12. The device of item 11, wherein the scaffold has been treated with sodium hydroxide.
13. The device of any one of the proceeding items, wherein the outer layer and/or the inner layer comprises an anti-inflammatory agent.
14. The device of item 13, wherein the anti-inflammatory agent is a calcineurin inhibitor.
15. The device of item 14, wherein the anti-inflammatory agent is tacrolimus.
16. The device of item 13, wherein the anti-inflammatory agent is a pyridone.
17. The device of item 16, wherein the anti-inflammatory agent is pirfenidone.
18. The device of item 13, wherein the anti-inflammatory agent is a phosphodiesterase inhibitor.
19. The device of item 18, wherein the anti-inflammatory agent is roflumilast.
20. The device of any one of the preceding items, wherein the outer layer and/or the inner layer comprise pores.
21. The device of item 20, wherein the pores are sized to permit the passage of biomolecules.
22. The device of item 21, wherein the biomolecules are 250 kDa or less.
23. The device of item 20, wherein the pores are sized to permit the passage of an antibody, or antigen-binding portion thereof.
24. The device of any one of items 20-23, wherein the pores are sized to prevent the passage of cells.
25. The device of any one of items 20-24, wherein the pores are sized to prevent cells on one side of the multilayer scaffold from contacting cells on the other side of the multilayer scaffold.
26. The device of any one of items 20-25, wherein the pores have a diameter of 1 µm or less.
27. The device of any one of items 20-26, wherein the pores have a diameter of 0.5 µm or less.
28. The device of any one of the preceding items, wherein the multilayer scaffold further comprises a porous membrane positioned between the inner layer and the outer layer.
29. The device of item 28, wherein the porous membrane comprises polyethylene terephthalate.
30. The device of item 29, wherein the porous membrane comprises nanofibrous polyethylene terephthalate.
31. The device of item 29, wherein the nanoporous membrane comprises non-nanofibrous polyethylene terephthalate.
32. The device of any one of items 28-31, wherein the porous membrane comprises membrane pores sized to permit the passage of biomolecules.
33. The device of item 32, wherein the biomolecules are 250 kDa or less.
34. The device of any one of items 28-33, wherein the membrane pores are sized to permit the passage of an antibody, or antigen-binding portion thereof.
35. The device of any one of items 28-34, wherein the membrane pores are sized to prevent the passage of cells.
36. The device of any one of items 28-35, wherein the membrane pores are sized to prevent cells on one side of the multilayer scaffold from contacting cells on the other side of the multilayer scaffold.
37. A device comprising a multilayer scaffold surrounding a cell chamber, wherein the multilayer scaffold comprises
   (i) an outer layer comprising nanofibrous polyethylene terephthalate and polybutylene terephthalate;
   (ii) a non-nanofibrous membrane positioned between the inner layer and the outer layer, wherein the membrane comprises nanopores sized to prevent the passage of cells across the membrane; and
   (iii) an inner layer comprising nanofibrous polyurethane.
38. A device comprising a multilayer scaffold surrounding a cell chamber, wherein the multilayer scaffold comprises
   (i) an outer layer comprising nanofibrous polyethylene terephthalate and polybutylene terephthalate;
   (ii) a non-nanofibrous membrane positioned between the inner layer and the outer layer, wherein the membrane comprises nanopores sized to prevent the passage of cells across the membrane; and
   (iii) an inner layer comprising nanofibrous polyethylene terephthalate and polybutylene terephthalate.
39. The device of any one of items 28-38, wherein the membrane pores have a diameter of 1 µm or less.
40. The device of any one of items 28-39, wherein the membrane pores have a diameter of 0.5 µm or less.
41. The device of any one of items 28-39, wherein the membrane pores have a diameter of about 0.2-0.6 µm.
42. The device of any one of items 28-39, wherein the membrane pores have a diameter of about 0.4 µm.
43. The device of any one of the preceding items, further comprising a loading port to permit the loading of cells into the cell chamber.
44. The device of any one of the preceding items, wherein the device comprises a total thickness of 250 µm or less.
45. The device of any one of the preceding items, wherein the device comprises a total thickness of 150 µm or less.
46. The device of any one of the preceding items, wherein the cell chamber is sized to accommodate up to 1x10⁹ cells.
47. The device of any one of the preceding items, wherein the cell chamber is sized to accommodate up to 1x10⁷ cells.
48. A device comprising a multilayer scaffold surrounding a cell chamber, wherein the multilayer scaffold comprises an outer layer comprising nanofibrous polyethylene terephthalate and polybutylene terephthalate, and an inner layer comprising nanofibrous polyurethane, wherein the outer layer and the inner layer comprise nanopores having a diameter of 1 µm or less.
49. The device of any one of the preceding items, wherein the cell chamber comprises cells.
50. The device of item 49, wherein the cells adhere to the inner layer of the scaffold.
51. The device of item 49 or 50, wherein the cells comprise retinal pigment epithelial cells.
52. The device of item 51, wherein the cells comprise ARPE-19 cells.
53. The device of any one of items 49-52, wherein the device comprises about 1x10⁶ cells to about 1x10⁸ cells.
54. The device of any one of items 49-53, wherein the device comprises about 1x10⁶ cells to about 1x10⁷ cells.
55. The device of any one of items 49-54, wherein the cells secrete a recombinant peptide or protein.
56. The device of item 55, wherein the cells secrete a protein selected from the group consisting of an antibody or antigen-binding portion thereof, a growth factor, a hormone, a prostaglandin, an enzyme, a cytokine, a peptide therapeutic, or a combination thereof.
57. The device of item 55 or 56, wherein the cells secrete a peptide therapeutic.
58. The device of item 55 or 56, wherein the cells secrete an enzyme.
59. The device of item 58, wherein the enzyme is laronidase.
60. The device of item 58, wherein the enzyme is idursulfase.
61. The device of item 58, wherein the enzyme is arylsulfatase A.
62. The device of item 55 or 56, wherein the cells secrete an antibody, or antigen-binding portion thereof.
63. The device of item 62, wherein the cells secrete a chimeric antibody, or antigen-binding portion thereof.
64. The device of item 62, wherein the cells secrete a humanized antibody, or antigen-binding portion thereof.
65. The device of item 62, wherein the cells secrete a human antibody, or antigen-binding portion thereof.
66. The device of any one of items 62-65, wherein the cells secrete a monoclonal antibody.
67. The device of any one of items 62-65, wherein the cells secrete an antibody fragment selected from the group consisting of a Fab, a F(ab')₂, an scFv, a tandem scFv, a diabody, a minibody, and a single domain antibody.
68. The device of any one of items 62-67, wherein the cells secrete an antibody, or antigen-binding portion thereof, that specifically binds an antigen selected from the group consisting of α4β7, integrin β7, TNFα, IL-12, IL-23, or CD20.
69. The device of item 68, wherein the cells secrete an antibody selected from vedolizumab, abrilumab, adalimumab, etrolizumab, certolizumab, golimumab, ustekinumab, infliximab, rituximab, and natalizumab.
70. The device of any one of items 62-67, wherein the cells secrete an antibody, or antigen-binding portion thereof, that specifically binds α4β7.
71. The device of item 70, wherein the cells secrete vedolizumab, or an antigen-binding portion thereof.
72. The device of item 49 or 50, wherein the cell chamber comprises cells having a three-dimensional architecture.
73. The device of item 72, wherein the cells having a three-dimensional architecture comprise a tissue explant.
74. The device of item 73, wherein the tissue is liver tissue or pancreatic tissue.
75. The device of item 72, wherein the cells having a three-dimensional architecture comprise organoids or spheroids.
76. The device of item 75, wherein the organoids comprise hepatocytes, liver cells, or pancreatic cells.
77. The device of item 75 or 76, wherein the organoids are organized around a sinusoid or a duct.
78. A method of delivering a biomolecule to a subject, comprising administering to the subject a device of any one of the preceding items, wherein the cell chamber of the device comprises cells secreting the biomolecule.
79. A method of delivering a recombinant peptide or a recombinant protein to a subject, comprising administering to the subject a device of any one of items 55-61.
80. A method of delivering an antibody, or antigen-binding portion thereof, to a subject, comprising administering to the subject a device of any one of items 62-71.
81. The method of any one of items 78-80, wherein the device is administered to the subject by implantation at a site selected from under the skin (subcutaneous implantation), on the omentum, in the liver, in the brain, or in the spinal canal.
82. The method of any one of items 78-81, wherein the device is implanted in the subject for at least 30 days.
83. The method of any one of items 78-81, wherein the device is implanted in the subject for at least 90 days.
84. The method of any one of items 78-81, wherein the device is implanted in the subject for at least 120 days.
85. The method of any one of items 78-81, wherein the device is implanted in the subject for at least 1 year.
86. The method of any one of items 78, 79, and 81-85, wherein the plasma concentration of the biomolecule, recombinant peptide, or recombinant protein is at least 5 µg/mL in the subject for a period of at least 60 days following implantation.
87. The method of any one of items 78-85, wherein the plasma concentration of the antibody, or antigen-binding portion thereof, is at least 5 µg/mL in the subject for a period of at least 60 days following implantation.
88. A method of treating a subject having Crohn's Disease or Ulcerative Colitis, comprising administering to the subject a device of item 70 or 71.
89. A method of treating a subject having primary sclerosing cholangitis, comprising administering to the subject a device of item 70 or 71.
90. A method of treating a subject having eosinophilic esophagitis, comprising administering to the subject a device of item 70 or 71.
91. A method of treating a subject having autoimmune hepatitis, comprising administering to the subject a device of item 70 or 71.
92. A method of treating a subject having short bowel syndrome, comprising administering to the subject a device of item 57.
93. A method of treating a subject having mucopolysaccharidosis type I (MPS I), comprising administering to the subject a device of item 59.
94. A method of treating a subject having mucopolysaccharidoses type II (MPS II), comprising administering to the subject a device of item 60.
95. A method of treating a subject having metachromatic leukodystrophy (MLD), comprising administering to the subject a device of item 61.
96. A device comprising a single-layer polymer scaffold surrounding a cell chamber, wherein the scaffold comprises a nanofibrous polymer.
97. The device of item 96, wherein the scaffold comprises nanofibrous polyethylene terephthalate and polybutylene terephthalate.
98. The device of item 96, wherein the scaffold comprises nanofibrous polyurethane.
99. The device of any one of items 96-98, wherein the scaffold further comprises an anti-inflammatory agent.
100. The device of item 99, wherein the anti-inflammatory agent is tacrolimus, pirfenidone, or roflumilast.
101. The device of any one of items 96-100, wherein the device further comprises a population of cells.
102. The device of item 101, wherein the population of cells is organized with a three-dimensional architecture.
103. The device of item 102, wherein the population of cells comprises a tissue explant.
104. The device of item 103, wherein the tissue explant is derived from liver tissue or pancreatic tissue.
105. The device of item 102, wherein the population of cells comprises an organoid.
106. The device of item 105, wherein the organoid comprise hepatocytes, liver cells, or pancreatic cells.
107. The device of item 105 or 106, wherein the organoid is organized around a sinusoid or a duct.

### SEQUENCE TABLE

| **SEQ ID NO:** | **DESCRIPTION** | **SEQUENCE** |
|---|---|---|
| 1 | Heavy chain (HC) variable region (amino acid) | |
| 2 | HC CDR1 (amino acid) | SYWMH |
| 3 | HC CDR2 (amino acid) | EIDPSESNTNYNQKFKG |
| 4 | HC CDR3 (amino acid) | GGYDGWDYAIDY |
| 5 | Light chain (LC) variable region (amino acid) | |
| 6 | LC CDR1 (amino acid) | RSSQSLAKSYGNTYLS |
| 7 | LC CDR2 (amino acid) | GISNRFS |
| 8 | LC CDR3 (amino acid) | LQGTHQPYT |
| 9 | Heavy chain amino acid sequence | QVQLVQSGAEVKKPGASVKVSCKGSGYTFTSYW MHWVRQAPGQRLEWIGEIDPSESNTNYNQKFKGR VTLTVDISASTAYMELSSLRSEDTAVYYCARGGY DGWDYAIDYWGQGTLVTVSSASTKGPSVFPLAPS SKSTSGGTAALGCLVKDYFPEPVTVSWNSGALTS GVHTFPAVLQSSGLYSLSSVVTVPSSSLGTQTYICN VNHKPSNTKVDKKVEPKSCDKTHTCPPCPAPELA GAPSVFLFPPKPKDTLMISRTPEVTCVVVDVSHED PEVKFNWYVDGVEVHNAKTKPREEQYNSTYRVV SVLTVLHQDWLNGKEYKCKVSNKALPAPIEKTIS KAKGQPREPQVYTLPPSRDELTKNQVSLTCLVKG FYPSDIAVEWESNGQPENNYKTTPPVLDSDGSFFL YSKLTVDKSRWQQGNVFSCSVMHEALHNHYTQK SLSLSPGK |
| 10 | Light chain amino acid sequence | DVVMTQSPLSLPVTPGEPASISCRSSQSLAKSYGNT YLSWYLQKPGQSPQLLIYGISNRFSGVPDRFSGSGS GTDFTLKISRVEAEDVGVYYCLQGTHQPYTFGQG TKVEIKRTVAAPSVFIFPPSDEQLKSGTASVVCLLN NFYPREAKVQWKVDNALQSGNSQESVTEQDSKD STYSLSSTLTLSKADYEKHKVYACEVTHQGLSSPV TKSFNRGEC |
| 11 | Light chain variable region (nucleic acid) | GATGTAGTGATGACTCAAAGTCCACTCTCCCTG CCTGTCACCCCTGGAGAACCAGCTTCTATCTCTT GCAGGTCTAGTCAGAGTCTTGCAAAGAGTTATG GGAACACCTATTTGTCTTGGTACCTGCAGAAGC CTGGCCAGTCTCCACAGCTCCTCATCTATGGGAT TTCCAACAGATTTTCTGGGGTGCCAGACAGGTT CAGTGGCAGTGGTTCAGGGACAGATTTCACACT CAAGATCTCGCGAGTAGAGGCTGAGGACGTGG GAGTGTATTACTGCTTACAAGGTACACATCAGC CGTACACGTTCGGACAGGGGACCAAGGTGGAG ATCAAG |
| 12 | Heavy chain variable region (nucleic acid) | CAGGTGCAATTGGTGCAGTCTGGGGCTGAGGTT AAGAAGCCTGGGGCTTCAGTGAAGGTGTCCTGC AAGGGTTCTGGCTACACCTTCACCAGCTACTGG ATGCATTGGGTGAGGCAGGCGCCTGGCCAACGT CTAGAGTGGATCGGAGAGATTGATCCTTCTGAG AGTAATACTAACTACAATCAAAAATTCAAGGGA CGCGTCACATTGACTGTAGACATTTCCGCTAGC ACAGCCTACATGGAGCTCTCCAGCCTGAGATCT GAGGACACTGCGGTCTACTATTGTGCAAGAGGG GGTTACGACGGATGGGACTATGCTATTGACTAC TGGGGTCAAGGCACCCTGGTCACCGTCAGCTCA |
| 13 | Light chain (nucleic acid) | GATGTAGTGATGACTCAAAGTCCACTCTCCCTG CCTGTCACCCCTGGAGAACCAGCTTCTATCTCTT GCAGGTCTAGTCAGAGTCTTGCAAAGAGTTATG GGAACACCTATTTGTCTTGGTACCTGCAGAAGC CTGGCCAGTCTCCACAGCTCCTCATCTATGGGAT TTCCAACAGATTTTCTGGGGTGCCAGACAGGTT CAGTGGCAGTGGTTCAGGGACAGATTTCACACT CAAGATCTCGCGAGTAGAGGCTGAGGACGTGG GAGTGTATTACTGCTTACAAGGTACACATCAGC CGTACACGTTCGGACAGGGGACCAAGGTGGAG ATCAAGCGTACGGTGGCTGCACCATCTGTCTTC ATCTTCCCGCCATCTGATGAGCAGTTGAAATCT GGAACTGCCTCTGTTGTGTGCCTGCTGAATAACT TCTATCCCAGAGAGGCCAAAGTACAGTGGAAGG TGGATAACGCCCTCCAATCGGGTAACTCCCAGG AGAGTGTCACAGAGCAGGACAGCAAGGACAGC ACCTACAGCCTCAGCAGCACCCTGACCCTGAGC AAAGCAGACTACGAGAAACACAAAGTCTACGC CTGCGAAGTCACCCATCAGGGCCTGAGCTCGCC CGTCACAAAGAGCTTCAACAGGGGAGAGTGT |
| 14 | Heavy chain (nucleic acid) | CAGGTGCAATTGGTGCAGTCTGGGGCTGAGGTT AAGAAGCCTGGGGCTTCAGTGAAGGTGTCCTGC AAGGGTTCTGGCTACACCTTCACCAGCTACTGG |
| | | ATGCATTGGGTGAGGCAGGCGCCTGGCCAACGT CTAGAGTGGATCGGAGAGATTGATCCTTCTGAG AGTAATACTAACTACAATCAAAAATTCAAGGGA CGCGTCACATTGACTGTAGACATTTCCGCTAGC ACAGCCTACATGGAGCTCTCCAGCCTGAGATCT GAGGACACTGCGGTCTACTATTGTGCAAGAGGG GGTTACGACGGATGGGACTATGCTATTGACTAC TGGGGTCAAGGCACCCTGGTCACCGTCAGCTCA GCCTCCACCAAGGGCCCATCGGTCTTCCCCCTG GCACCCTCCTCCAAGAGCACCTCTGGGGGCACA GCGGCCCTGGGCTGCCTGGTCAAGGACTACTTC CCCGAACCGGTGACGGTGTCGTGGAACTCAGGC GCCCTGACCAGCGGCGTGCACACCTTCCCGGCT GTCCTACAGTCCTCAGGACTCTACTCCCTCAGCA GCGTGGTGACCGTGCCCTCCAGCAGCTTGGGCA CCCAGACCTACATCTGCAACGTGAATCACAAGC CCAGCAACACCAAGGTGGACAAGAAAGTTGAG CCCAAATCTTGTGACAAAACTCACACATGCCCA CCGTGCCCAGCACCTGAACTCGCGGGGGCACCG TCAGTCTTCCTCTTCCCCCCAAAACCCAAGGAC ACCCTCATGATCTCCCGGACCCCTGAGGTCACA TGCGTGGTGGTGGACGTGAGCCACGAAGACCCT GAGGTCAAGTTCAACTGGTACGTGGACGGCGTG GAGGTGCATAATGCCAAGACAAAGCCGCGGGA GGAGCAGTACAACAGCACGTACCGTGTGGTCAG CGTCCTCACCGTCCTGCACCAGGACTGGCTGAA TGGCAAGGAGTACAAGTGCAAGGTCTCCAACAA AGCCCTCCCAGCCCCCATCGAGAAAACCATCTC CAAAGCCAAAGGGCAGCCCCGAGAACCACAGG TGTACACCCTGCCCCCATCCCGGGATGAGCTGA CCAAGAACCAGGTCAGCCTGACCTGCCTGGTCA AAGGCTTCTATCCCAGCGACATCGCCGTGGAGT GGGAGAGCAATGGGCAGCCGGAGAACAACTAC AAGACCACGCCTCCCGTGCTGGACTCCGACGGC TCCTTCTTCCTCTACAGCAAGCTCACCGTGGACA AGAGCAGGTGGCAGCAGGGGAACGTCTTCTCAT GCTCCGTGATGCATGAGGCTCTGCACAACCACT ACACGCAGAAGAGCCTCTCCCTGTCTCCGGGTA AA |
| 15 | Heavy chain (nucleic acid) | GGTACCAGAATTCCTCACCATGGGCTGGTCCTG CATCATCCTGTTTCTGGTGGCCACAGCCACAGG GGTGCACAGCCAGGTGCAGCTGGTGCAGTCTGG GGCTGAGGTGAAGAAGCCAGGGGCCTCTGTGA AGGTGTCCTGCAAGGGCTCTGGCTACACCTTCA CCAGCTACTGGATGCACTGGGTGCGCCAGGCCC CAGGCCAGAGGCTGGAGTGGATCGGGGAGATC |
| | | GACCCCTCTGAGAGCAACACCAACTACAACCAG AAGTTCAAGGGCAGGGTGACCCTGACAGTGGAC ATCTCTGCCAGCACAGCCTACATGGAGCTGTCC AGCCTGAGATCTGAGGACACAGCTGTGTACTAC TGTGCCAGGGGGGGATATGATGGCTGGGACTAT GCCATCGACTACTGGGGCCAGGGCACCCTGGTG ACAGTCAGCTCAGCCAGCACCAAGGGCCCCTCT GTGTTCCCCCTGGCCCCCAGCAGCAAGAGCACC TCTGGGGGCACAGCTGCCCTGGGCTGCCTGGTG AAGGACTACTTCCCAGAGCCAGTGACAGTGTCC TGGAACTCTGGGGCCCTGACATCTGGGGTGCAC ACCTTCCCTGCTGTGCTGCAGTCCTCTGGCCTGT ATAGCCTGTCCTCTGTGGTGACAGTGCCCAGCA GCAGCCTGGGCACCCAGACCTACATCTGCAATG TGAACCACAAGCCCAGCAACACAAAGGTGGAC AAGAAGGTGGAGCCCAAGAGCTGTGACAAGAC CCACACCTGCCCCCCCTGCCCAGCCCCAGAGCT GGCTGGGGCTCCATCTGTGTTTCTGTTCCCCCC AAGCCCAAGGACACCCTGATGATCAGCAGGACC CCAGAGGTGACCTGTGTGGTGGTGGATGTGAGC CATGAGGATCCAGAGGTGAAGTTCAATTGGTAT GTGGATGGGGTGGAGGTGCACAATGCCAAGAC CAAGCCCAGAGAGGAGCAGTACAACAGCACCT ACAGGGTGGTGTCTGTGCTGACAGTGCTGCACC AGGACTGGCTGAATGGCAAGGAGTACAAGTGC AAGGTCTCCAATAAGGCCCTGCCAGCCCCCATC GAAAAGACCATCAGCAAGGCCAAGGGCCAGCC AAGGGAGCCCCAGGTGTACACCCTGCCCCCCTC CAGAGATGAGCTGACCAAGAACCAGGTGTCCCT GACCTGTCTGGTGAAGGGCTTCTACCCCTCTGA CATCGCTGTGGAGTGGGAGAGCAATGGCCAGCC AGAGAACAACTACAAGACCACCCCCCCAGTGCT GGATTCTGATGGCAGCTTCTTCCTGTACAGCAA GCTGACAGTGGATAAGTCCAGGTGGCAGCAGG GCAATGTGTTCAGCTGCTCTGTGATGCATGAGG CCCTGCACAACCACTACACCCAGAAGAGCCTGT CCCTGAGCCCAGGCAAGTGATAGTCTAGAGGAG CTC |
| 16 | Light chain (nucleic acid) | GGTACCAGCGGCCGCCTCACCATGGGCTGGTCC TGCATCATCCTGTTTCTGGTGGCCACAGCCACA GGGGTGCACTCTGATGTGGTGATGACCCAGAGC CCCCTGAGCCTGCCAGTGACCCCAGGGGAGCCA GCCAGCATCAGCTGCAGGTCCAGCCAGAGCCTG GCCAAGAGCTATGGCAACACCTACCTGAGCTGG TATCTGCAGAAGCCAGGCCAGTCCCCCCAGCTG CTGATCTATGGCATCAGCAACAGGTTCTCTGGG |
| | | GTGCCAGACAGATTCTCTGGCTCTGGGTCTGGC ACAGACTTCACCCTGAAGATCAGCAGGGTGGAG GCTGAGGATGTGGGGGTGTACTACTGCCTGCAG GGCACACACCAGCCCTACACCTTTGGCCAGGGC ACCAAGGTGGAGATCAAGCGTACGGTGGCTGCC CCCTCTGTGTTCATCTTCCCCCCCTCTGATGAGC AGCTGAAGTCTGGGACAGCCTCTGTGGTGTGCC TGCTGAACAACTTCTACCCCAGGGAGGCCAAGG TGCAGTGGAAGGTGGACAATGCCCTGCAGTCTG GCAACAGCCAGGAGTCTGTGACAGAGCAGGAC AGCAAGGACTCCACCTACAGCCTGAGCAGCACC CTGACCCTGAGCAAGGCTGACTATGAGAAGCAC AAGGTGTACGCCTGTGAGGTGACCCACCAGGGC CTGTCCAGCCCAGTGACCAAGAGCTTCAACAGG GGGGAGTGCTGATAGTCTAGAGGAGCTC |
| 17 | Heavy chain (nucleic acid) | GGTACCGAATTCCTCACCATGGGCTGGTCCTGC ATCATCCTGTTTCTGGTGGCCACCGCCACCGGC GTGCACAGCCAGGTGCAGCTGGTGCAGAGCGG AGCCGAGGTGAAGAAGCCAGGCGCCAGCGTCA AGGTGTCCTGCAAGGGCAGCGGCTACACCTTCA CCAGCTACTGGATGCACTGGGTGCGGCAGGCCC CAGGCCAGAGGCTGGAGTGGATCGGCGAGATC GACCCCAGCGAGAGCAACACCAACTACAACCA GAAGTTCAAGGGCAGGGTCACCCTGACCGTGGA CATCAGCGCCAGCACCGCCTACATGGAGCTGTC CAGCCTGAGAAGCGAGGACACCGCCGTGTACTA CTGCGCCAGGGGCGGCTACGACGGCTGGGACTA CGCCATCGACTACTGGGGCCAGGGCACCCTGGT GACCGTCAGCTCAGCCAGCACCAAGGGCCCCAG CGTGTTCCCCCTGGCCCCCAGCAGCAAGAGCAC CAGCGGCGGCACAGCCGCCCTGGGCTGCCTGGT GAAGGACTACTTCCCCGAGCCCGTGACCGTGTC CTGGAACAGCGGAGCCCTGACCAGCGGGGTGC ACACCTTCCCCGCCGTGCTGCAGAGCAGCGGCC TGTACAGCCTGTCCAGCGTGGTGACAGTGCCCA GCAGCAGCCTGGGCACCCAGACCTACATCTGCA ACGTGAACCACAAGCCCTCCAACACCAAGGTGG ACAAGAAGGTGGAGCCCAAGAGCTGCGACAAG ACCCACACCTGCCCCCCCTGCCCAGCCCCCGAG CTGGCCGGAGCCCCCTCCGTGTTCCTGTTCCCCC CCAAGCCCAAGGACACCCTGATGATCAGCAGGA CCCCCGAGGTGACCTGCGTGGTGGTGGACGTGA GCCACGAGGACCCAGAGGTGAAGTTCAACTGGT ACGTGGACGGCGTGGAGGTGCACAACGCCAAG ACCAAGCCCAGAGAGGAGCAGTACAACAGCAC CTACAGGGTGGTGTCCGTGCTGACCGTGCTGCA |
| | | CCAGGACTGGCTGAACGGCAAGGAATACAAGT GCAAGGTCTCCAACAAGGCCCTGCCTGCCCCCA TCGAAAAGACCATCAGCAAGGCCAAGGGCCAG CCACGGGAGCCCCAGGTGTACACCCTGCCCCCC TCCCGGGACGAGCTGACCAAGAACCAGGTGTCC CTGACCTGTCTGGTGAAGGGCTTCTACCCCAGC GACATCGCCGTGGAGTGGGAGAGCAACGGCCA GCCCGAGAACAACTACAAGACCACCCCCCCAGT GCTGGACAGCGACGGCAGCTTCTTCCTGTACAG CAAGCTGACAGTGGACAAGAGCAGGTGGCAGC AGGGCAACGTGTTCAGCTGCAGCGTGATGCACG AGGCCCTGCACAACCACTACACCCAGAAGAGCC TGAGCCTGTCCCCCGGCAAGTGATAGTCTAGAG GAGCTC |
| 18 | Light chain (nucleic acid) | GGTACCGCGGCCGCCTCACCATGGGCTGGTCCT GCATCATCCTGTTTCTGGTGGCCACCGCCACCG GCGTGCACAGCGACGTGGTGATGACCCAGAGCC CCCTGAGCCTGCCCGTGACCCCAGGCGAGCCCG CCAGCATCAGCTGCAGGTCCAGCCAGAGCCTGG CCAAGAGCTACGGCAACACCTACCTGAGCTGGT ATCTGCAGAAGCCCGGCCAGTCCCCCCAGCTGC TGATCTACGGCATCAGCAACAGGTTCAGCGGCG TGCCCGACAGGTTCTCCGGCAGCGGCTCCGGCA CCGACTTCACCCTGAAAATCAGCAGGGTGGAGG CCGAGGACGTGGGCGTGTACTACTGCCTGCAGG GCACACACCAGCCCTACACCTTCGGCCAGGGCA CCAAGGTGGAAATCAAGCGTACGGTGGCCGCTC CCAGCGTGTTCATCTTCCCCCCCAGCGACGAGC AGCTGAAGAGCGGCACCGCCAGCGTGGTGTGCC TGCTGAACAACTTCTACCCCCGGGAGGCCAAGG TGCAGTGGAAGGTGGACAACGCCCTGCAGAGC GGCAACAGCCAGGAGAGCGTCACCGAGCAGGA CAGCAAGGACTCCACCTACAGCCTGAGCAGCAC CCTGACCCTGAGCAAGGCCGACTACGAGAAGCA CAAGGTGTACGCCTGCGAGGTGACCCACCAGGG CCTGTCCAGCCCCGTGACCAAGAGCTTCAACAG GGGCGAGTGCTGATAGTCTAGAGGAGCTC |
| 19 | Light chain (nucleic acid) | ATGGGCTGGTCCTGCATCATCCTGTTTCTGGTGG CCACMGCCACMGGSGTGCACWSYGAYGTGGTG ATGACCCAGAGCCCCCTGAGCCTGCCMGTGACC CCAGGSGAGCCMGCCAGCATCAGCTGCAGGTCC AGGCAGAGCCTGGCCAAGAGCTAYGGCAACAC CTACCTGAGCCGGTATCTGCAGAAGCCMGGCCA GTCGCCCCAGCTGCTGATCTAYGGCATCAGCAA CAGGTTCWSYGGSGTGCCMGACAGRTTCTCYGG CWSYGGSTCYGGCACMGACTTCACCCTGAARAT |
| | | CAGCAGGGTGGAGGCYGAGGAYGTGGGSGTGT ACTACTGCCTGCAGGGCACACACCAGCCCTACA CCTTYGGCCAGGGCACCAAGGTGGARATCAAGC GTACGGTGGCYGCYCCCWSYGTGTTCATCTTCC CCCCCWSYGAYGAGCAGCTGAAGWSYGGSACM GCCWSYGTGGTGTGCCTGCTGAACAACTTCTAC CCCMGGGAGGCCAAGGTGCAGTGGAAGGTGGA CAAYGCCCTGCAGWSYGGCAACAGCCAGGAGW SYGTSACMGAGCAGGACAGCAAGGACTCCACCT ACAGCCTGAGCAGCACCCTGACCCTGAGCAAGG CYGACTAYGAGAAGCACAAGGTGTACGCCTGY GAGGTGACCCACCAGGGCCTGTCCAGCCCMGTG ACCAAGAGCTTCAACAGGGGSGAGTGCTGATAG TCTAGAGGAGCTC |
| 20 | Heavy chain (nucleic acid) | ATGGGCTGGTCCTGCATCATCCTGTTTCTGGTGG CCACMGCCACMGGSGTGCACAGCCAGGTGCAG CTGGTGCAGWSYGGRGCYGAGGTGAAGAAGCC AGGSGCCWSYGTSAAGGTGTCCTGCAAGGGCWS YGGCTACACCTTCACCAGCTACTGGATGCACTG GGTGCGSCAGGCCCCAGGCCAGAGGCTGGAGTG GATCGGSGAGATCGACCCCWSYGAGAGCAACA CCAACTACAACCAGAAGTTCAAGGGCAGGGTSA CCCTGACMGTGGACATCWSYGCCAGCACMGCC TACATGGAGCTGTCCAGCCTGAGAWSYGAGGA CACMGCYGTGTACTACTGYGCCAGGGGSGGMT AYGAYGGCTGGGACTAYGCCATCGACTACTGGG GCCAGGGCACCCTGGTGACMGTCAGCTCAGCCA GCACCAAGGGCCCCWSYGTGTTCCCCCTGGCCC CCAGCAGCAAGAGCACCWSYGGSGGCACAGCY GCCCTGGGCTGCCTGGTGAAGGACTACTTCCCM GAGCCMGTGACMGTGTCCTGGAACWSYGGRGC CCTGACMWSYGGGGTGCACACCTTCCCYGCYGT GCTGCAGWSCWSYGGCCTGTAYAGCCTGTCCW SYGTGGTGACAGTGCCCAGCAGCAGCCTGGGCA CCCAGACCTACATCTGCAAYGTGAACCACAAGC CCWSCAACACMAAGGTGGACAAGAAGGTGGAG CCCAAGAGCTGYGACAAGACCCACACCTGCCCC CCCTGCCCAGCCCCMGAGCTGGCYGGRGCYCC MTCYGTGTTYCTGTTCCCCCCCAAGCCCAAGGA CACCCTGATGATCAGCAGGACCCCMGAGGTGA CCTGYGTGGTGGTGGAYGTGAGCCAYGAGGAY CCAGAGGTGAAGTTCAAYTGGTAYGTGGAYGGS GTGGAGGTGCACAAYGCCAAGACCAAGCCCAG AGAGGAGCAGTACAACAGCACCTACAGGGTGG TGTCYGTGCTGACMGTGCTGCACCAGGACTGGC TGAAYGGCAAGGARTACAAGTGCAAGGTCTCCA |
| | | AYAAGGCCCTGCCWGCCCCCATCGAAAAGACC ATCAGCAAGGCCAAGGGCCAGCCAMGGGAGCC CCAGGTGTACACCCTGCCCCCCTCCMGRGAYGA GCTGACCAAGAACCAGGTGTCCCTGACCTGTCT GGTGAAGGGCTTCTACCCCWSYGACATCGCYGT GGAGTGGGAGAGCAAYGGCCAGCCMGAGAACA ACTACAAGACCACCCCCCCAGTGCTGGAYWSYG AYGGCAGCTTCTTCCTGTACAGCAAGCTGACAG TGGAYAAGWSCAGGTGGCAGCAGGGCAAYGTG TTCAGCTGCWSYGTGATGCAYGAGGCCCTGCAC AACCACTACACCCAGAAGAGCCTGWSCCTGWS CCCMGGCAAGTGATAGTCTAGAGGAGCTC |
| 21 | Peptide therapeutic | HGDGSFSDEMNTILDNLAARDFINWLIQTKITD |
| 22 | Heavy chain (HC) variable region (amino acid) | |
| 23 | Light chain (LC) variable region (amino acid) | |
| 24 | HC CDR1 (amino acid) | GFTFDDY |
| 25 | HC CDR2 (amino acid) | TWNSGH |
| 26 | HC CDR3 (amino acid) | VSYLSTASSLDY |
| 27 | LC CDR1 (amino acid) | QGIRNYLA |
| 28 | LC CDR2 (amino acid) | AASTLQS |
| 29 | LC CDR3 (amino acid) | QRYNRAPYT |
| 30 | Heavy chain amino acid sequence | EVQLVESGGGLVQPGRSLRLSCAASGFTFDDYAM HWVRQAPGKGLEWVSAITWNSGHIDYADSVEGR FTISRDNAKNSLYLQMNSLRAEDTAVYYCAKVSY LSTASSLDYWGQGTLVTVSSASTKGPSVFPLAPSS KSTSGGTAALGCLVKDYFPEPVTVSWNSGALTSG VHTFPAVLQSSGLYSLSSVVTVPSSSLGTQTYICNV NHKPSNTKVDKKVEPKSCDKTHTCPPCPAPELLG GPSVFLFPPKPKDTLMISRTPEVTCVVVDVSHEDP EVKFNWYVDGVEVHNAKTKPREEQYNSTYRVVS VLTVLHQDWLNGKEYKCKVSNKALPAPIEKTISK AKGQPREPQVYTLPPSRDELTKNQVSLTCLVKGF YPSDIAVEWESNGQPENNYKTTPPVLDSDGSFFLY |
| | | SKLTVDKSRWQQGNVFSCSVMHEALHNHYTQKS LSLSPGK |
| 31 | Light chain amino acid sequence | DIQMTQSPSSLSASVGDRVTITCRASQGIRNYLAW YQQKPGKAPKLLIYAASTLQSGVPSRFSGSGSGTD FTLTISSLQPEDVATYYCQRYNRAPYTFGQGTKVE IKRTVAAPSVFIFPPSDEQLKSGTASVVCLLNNFYP REAKVQWKVDNALQSGNSQESVTEQDSKDSTYS LSSTLTLSKADYEKHKVYACEVTHQGLSSPVTKSF NRGEC |
| 32 | Heavy chain (HC) variable region (amino acid) | EVQLVQSGAEVKKPGESLKISCKGSGYSFTTYWL GWVRQMPGKGLDWIGIMSPVDSDIRYSPSFQGQV TMSVDKSITTAYLQWNSLKASDTAMYYCARRRP GQGYFDFWGQGTLVTVSS |
| 33 | Light chain (LC) variable region (amino acid) | DIQMTQSPSSLSASVGDRVTITCRASQGISSWLAW YQQKPEKAPKSLIYAASSLQSGVPSRFSGSGSGTD FTLTISSLQPEDFATYYCQQYNIYPYTFGQGTKLEI K |
| 34 | HC CDR1 (amino acid) | GYSFTTY |
| 35 | HC CDR2 (amino acid) | SPVDSD |
| 36 | HC CDR3 (amino acid) | RRPGQGYFDF |
| 37 | LC CDR1 (amino acid) | QGISSWLA |
| 38 | LC CDR2 (amino acid) | AASSLQS |
| 39 | LC CDR3 (amino acid) | QQYNIYPYT |
| 40 | Heavy chain amino acid sequence | EVQLVQSGAEVKKPGESLKISCKGSGYSFTTYWL GWVRQMPGKGLDWIGIMSPVDSDIRYSPSFQGQV TMSVDKSITTAYLQWNSLKASDTAMYYCARRRP GQGYFDFWGQGTLVTVSSSSTKGPSVFPLAPSSKS TSGGTAALGCLVKDYFPEPVTVSWNSGALTSGVH TFPAVLQSSGLYSLSSVVTVPSSSLGTQTYICNVNH KPSNTKVDKRVEPKSCDKTHTCPPCPAPELLGGPS VFLFPPKPKDTLMISRTPEVTCVVVDVSHEDPEVK FNWYVDGVEVHNAKTKPREEQYNSTYRVVSVLT VLHQDWLNGKEYKCKVSNKALPAPIEKTISKAKG QPREPQVYTLPPSRDELTKNQVSLTCLVKGFYPSD IAVEWESNGQPENNYKTTPPVLDSDGSFFLYSKLT VDKSRWQQGNVFSCSVMHEALHNHYTQKSLSLS PGK |
| 41 | Light chain amino acid sequence | DIQMTQSPSSLSASVGDRVTITCRASQGISSWLAW YQQKPEKAPKSLIYAASSLQSGVPSRFSGSGSGTD FTLTISSLQPEDFATYYCQQYNIYPYTFGQGTKLEI |
| | | KRTVAAPSVFIFPPSDEQLKSGTASVVCLLNNFYP REAKVQWKVDNALQSGNSQESVTEQDSKDSTYS LSSTLTLSKADYEKHKVYACEVTHQGLSSPVTKSF NRGEC |
| 42 | Heavy chain variable region (nucleic acid) | GAGGTGCAGCTGGTGGAGAGCGGCGGCGGCCT GGTGCAGCCCGGCCGGAGCCTGCGGCTGAGCTG CGCCGCCAGCGGCTTCACCTTCGACGACTACGC CATGCACTGGGTGCGGCAGGCCCCCGGCAAGGG CCTGGAGTGGGTGAGCGCCATCACCTGGAACAG CGGCCACATCGACTACGCCGACAGCGTGGAGGG CCGGTTCACCATCAGCCGGGACAACGCCAAGAA CAGCCTGTACCTGCAGATGAACAGCCTGCGGGC CGAGGACACCGCCGTGTACTACTGCGCCAAGGT GAGCTACCTGAGCACCGCCAGCAGCCTGGACTA CTGGGGCCAGGGCACCCTGGTGACCGTGAGCAG C |
| 43 | Light chain variable region (nucleic acid) | GACATCCAGATGACCCAGAGCCCCAGCAGCCTG AGCGCCAGCGTGGGCGACCGGGTGACCATCACC TGCCGGGCCAGCCAGGGCATCCGGAACTACCTG GCCTGGTACCAGCAGAAGCCCGGCAAGGCCCCC AAGCTGCTGATCTACGCCGCCAGCACCCTGCAG AGCGGCGTGCCCAGCCGGTTCAGCGGCAGCGGC AGCGGCACCGACTTCACCCTGACCATCAGCAGC CTGCAGCCCGAGGACGTGGCCACCTACTACTGC CAGCGGTACAACCGGGCCCCCTACACCTTCGGC CAGGGCACCAAGGTGGAGATCAAG |
| 44 | Heavy chain (nucleic acid) | GAGGTGCAGCTGGTGGAGAGCGGCGGCGGCCT GGTGCAGCCCGGCCGGAGCCTGCGGCTGAGCTG CGCCGCCAGCGGCTTCACCTTCGACGACTACGC CATGCACTGGGTGCGGCAGGCCCCCGGCAAGGG CCTGGAGTGGGTGAGCGCCATCACCTGGAACAG CGGCCACATCGACTACGCCGACAGCGTGGAGGG CCGGTTCACCATCAGCCGGGACAACGCCAAGAA CAGCCTGTACCTGCAGATGAACAGCCTGCGGGC CGAGGACACCGCCGTGTACTACTGCGCCAAGGT GAGCTACCTGAGCACCGCCAGCAGCCTGGACTA CTGGGGCCAGGGCACCCTGGTGACCGTGAGCAG CGCCAGCACCAAGGGCCCCAGCGTGTTCCCCCT GGCCCCCAGCAGCAAGAGCACCAGCGGCGGCA CCGCCGCCCTGGGCTGCCTGGTGAAGGACTACT TCCCCGAGCCCGTGACCGTGAGCTGGAACAGCG GCGCCCTGACCAGCGGCGTGCACACCTTCCCCG CCGTGCTGCAGAGCAGCGGCCTGTACAGCCTGA GCAGCGTGGTGACCGTGCCCAGCAGCAGCCTGG GCACCCAGACCTACATCTGCAACGTGAACCACA |
| | | AGCCCAGCAACACCAAGGTGGACAAGAAGGTG GAGCCCAAGAGCTGCGACAAGACCCACACCTGC CCCCCCTGCCCCGCCCCCGAGCTGCTGGGCGGC CCCAGCGTGTTCCTGTTCCCCCCCAAGCCCAAG GACACCCTGATGATCAGCCGGACCCCCGAGGTG ACCTGCGTGGTGGTGGACGTGAGCCACGAGGAC CCCGAGGTGAAGTTCAACTGGTACGTGGACGGC GTGGAGGTGCACAACGCCAAGACCAAGCCCCG GGAGGAGCAGTACAACAGCACCTACCGGGTGG TGAGCGTGCTGACCGTGCTGCACCAGGACTGGC TGAACGGCAAGGAGTACAAGTGCAAGGTGAGC AACAAGGCCCTGCCCGCCCCCATCGAGAAGACC ATCAGCAAGGCCAAGGGCCAGCCCCGGGAGCC CCAGGTGTACACCCTGCCCCCCAGCCGGGACGA GCTGACCAAGAACCAGGTGAGCCTGACCTGCCT GGTGAAGGGCTTCTACCCCAGCGACATCGCCGT GGAGTGGGAGAGCAACGGCCAGCCCGAGAACA ACTACAAGACCACCCCCCCCGTGCTGGACAGCG ACGGCAGCTTCTTCCTGTACAGCAAGCTGACCG TGGACAAGAGCCGGTGGCAGCAGGGCAACGTG TTCAGCTGCAGCGTGATGCACGAGGCCCTGCAC AACCACTACACCCAGAAGAGCCTGAGCCTGAGC CCCGGCAAG |
| 45 | Light chain (nucleic acid) | GACATCCAGATGACCCAGAGCCCCAGCAGCCTG AGCGCCAGCGTGGGCGACCGGGTGACCATCACC TGCCGGGCCAGCCAGGGCATCCGGAACTACCTG GCCTGGTACCAGCAGAAGCCCGGCAAGGCCCCC AAGCTGCTGATCTACGCCGCCAGCACCCTGCAG AGCGGCGTGCCCAGCCGGTTCAGCGGCAGCGGC AGCGGCACCGACTTCACCCTGACCATCAGCAGC CTGCAGCCCGAGGACGTGGCCACCTACTACTGC CAGCGGTACAACCGGGCCCCCTACACCTTCGGC CAGGGCACCAAGGTGGAGATCAAGCGGACCGT GGCCGCCCCCAGCGTGTTCATCTTCCCCCCCAGC GACGAGCAGCTGAAGAGCGGCACCGCCAGCGT GGTGTGCCTGCTGAACAACTTCTACCCCCGGGA GGCCAAGGTGCAGTGGAAGGTGGACAACGCCC TGCAGAGCGGCAACAGCCAGGAGAGCGTGACC GAGCAGGACAGCAAGGACAGCACCTACAGCCT GAGCAGCACCCTGACCCTGAGCAAGGCCGACTA CGAGAAGCACAAGGTGTACGCCTGCGAGGTGA CCCACCAGGGCCTGAGCAGCCCCGTGACCAAGA GCTTCAACCGGGGCGAGTGC |
| 46 | Heavy chain variable region (nucleic acid) | GAGGTGCAGCTGGTGCAGAGCGGCGCCGAGGT GAAGAAGCCCGGCGAGAGCCTGAAGATCAGCT GCAAGGGCAGCGGCTACAGCTTCACCACCTACT |
| | | GGCTGGGCTGGGTGCGGCAGATGCCCGGCAAG GGCCTGGACTGGATCGGCATCATGAGCCCCGTG GACAGCGACATCCGGTACAGCCCCAGCTTCCAG GGCCAGGTGACCATGAGCGTGGACAAGAGCAT CACCACCGCCTACCTGCAGTGGAACAGCCTGAA GGCCAGCGACACCGCCATGTACTACTGCGCCCG GCGGCGGCCCGGCCAGGGCTACTTCGACTTCTG GGGCCAGGGCACCCTGGTGACCGTGAGCAGC |
| 47 | Light chain variable region (nucleic acid) | GACATCCAGATGACCCAGAGCCCCAGCAGCCTG AGCGCCAGCGTGGGCGACCGGGTGACCATCACC TGCCGGGCCAGCCAGGGCATCAGCAGCTGGCTG GCCTGGTACCAGCAGAAGCCCGAGAAGGCCCCC AAGAGCCTGATCTACGCCGCCAGCAGCCTGCAG AGCGGCGTGCCCAGCCGGTTCAGCGGCAGCGGC AGCGGCACCGACTTCACCCTGACCATCAGCAGC CTGCAGCCCGAGGACTTCGCCACCTACTACTGC CAGCAGTACAACATCTACCCCTACACCTTCGGC CAGGGCACCAAGCTGGAGATCAAG |
| 48 | Heavy chain (nucleic acid) | GAGGTGCAGCTGGTGCAGAGCGGCGCCGAGGT GAAGAAGCCCGGCGAGAGCCTGAAGATCAGCT GCAAGGGCAGCGGCTACAGCTTCACCACCTACT GGCTGGGCTGGGTGCGGCAGATGCCCGGCAAG GGCCTGGACTGGATCGGCATCATGAGCCCCGTG GACAGCGACATCCGGTACAGCCCCAGCTTCCAG GGCCAGGTGACCATGAGCGTGGACAAGAGCAT CACCACCGCCTACCTGCAGTGGAACAGCCTGAA GGCCAGCGACACCGCCATGTACTACTGCGCCCG GCGGCGGCCCGGCCAGGGCTACTTCGACTTCTG GGGCCAGGGCACCCTGGTGACCGTGAGCAGCA GCAGCACCAAGGGCCCCAGCGTGTTCCCCCTGG CCCCCAGCAGCAAGAGCACCAGCGGCGGCACC GCCGCCCTGGGCTGCCTGGTGAAGGACTACTTC CCCGAGCCCGTGACCGTGAGCTGGAACAGCGGC GCCCTGACCAGCGGCGTGCACACCTTCCCCGCC GTGCTGCAGAGCAGCGGCCTGTACAGCCTGAGC AGCGTGGTGACCGTGCCCAGCAGCAGCCTGGGC ACCCAGACCTACATCTGCAACGTGAACCACAAG CCCAGCAACACCAAGGTGGACAAGCGGGTGGA GCCCAAGAGCTGCGACAAGACCCACACCTGCCC CCCCTGCCCCGCCCCCGAGCTGCTGGGCGGCCC CAGCGTGTTCCTGTTCCCCCCCAAGCCCAAGGA CACCCTGATGATCAGCCGGACCCCCGAGGTGAC CTGCGTGGTGGTGGACGTGAGCCACGAGGACCC CGAGGTGAAGTTCAACTGGTACGTGGACGGCGT GGAGGTGCACAACGCCAAGACCAAGCCCCGGG AGGAGCAGTACAACAGCACCTACCGGGTGGTG |
| | | AGCGTGCTGACCGTGCTGCACCAGGACTGGCTG AACGGCAAGGAGTACAAGTGCAAGGTGAGCAA CAAGGCCCTGCCCGCCCCCATCGAGAAGACCAT CAGCAAGGCCAAGGGCCAGCCCCGGGAGCCCC AGGTGTACACCCTGCCCCCCAGCCGGGACGAGC TGACCAAGAACCAGGTGAGCCTGACCTGCCTGG TGAAGGGCTTCTACCCCAGCGACATCGCCGTGG AGTGGGAGAGCAACGGCCAGCCCGAGAACAAC TACAAGACCACCCCCCCCGTGCTGGACAGCGAC GGCAGCTTCTTCCTGTACAGCAAGCTGACCGTG GACAAGAGCCGGTGGCAGCAGGGCAACGTGTT CAGCTGCAGCGTGATGCACGAGGCCCTGCACAA CCACTACACCCAGAAGAGCCTGAGCCTGAGCCC CGGCAAG |
| 49 | Light chain (nucleic acid) | GACATCCAGATGACCCAGAGCCCCAGCAGCCTG AGCGCCAGCGTGGGCGACCGGGTGACCATCACC TGCCGGGCCAGCCAGGGCATCAGCAGCTGGCTG GCCTGGTACCAGCAGAAGCCCGAGAAGGCCCCC AAGAGCCTGATCTACGCCGCCAGCAGCCTGCAG AGCGGCGTGCCCAGCCGGTTCAGCGGCAGCGGC AGCGGCACCGACTTCACCCTGACCATCAGCAGC CTGCAGCCCGAGGACTTCGCCACCTACTACTGC CAGCAGTACAACATCTACCCCTACACCTTCGGC CAGGGCACCAAGCTGGAGATCAAGCGGACCGT GGCCGCCCCCAGCGTGTTCATCTTCCCCCCCAGC GACGAGCAGCTGAAGAGCGGCACCGCCAGCGT GGTGTGCCTGCTGAACAACTTCTACCCCCGGGA GGCCAAGGTGCAGTGGAAGGTGGACAACGCCC TGCAGAGCGGCAACAGCCAGGAGAGCGTGACC GAGCAGGACAGCAAGGACAGCACCTACAGCCT GAGCAGCACCCTGACCCTGAGCAAGGCCGACTA CGAGAAGCACAAGGTGTACGCCTGCGAGGTGA CCCACCAGGGCCTGAGCAGCCCCGTGACCAAGA GCTTCAACCGGGGCGAGTGC |
| 50 | Laronidase | AEAPHLVHVDAARALWPLRRFWRSTGFCPPLPHS QADQYVLSWDQQLNLAYVGAVPHRGIKQVRTH WLLELVTTRGSTGRGLSYNFTHLDGYLDLLRENQ LLPGFELMGSASGHFTDFEDKQQVFEWKDLVSSL ARRYIGRYGLAHVSKWNFETWNEPDHHDFDNVS MTMQGFLNYYDACSEGLRAASPALRLGGPGDSFH TPPRSPLSWGLLRHCHDGTNFFTGEAGVRLDYISL HRKGARSSISILEQEKVVAQQIRQLFPKFADTPIYN DEADPLVGWSLPQPWRADVTYAAMVVKVIAQHQ NLLLANTTSAFPYALLSNDNAFLSYHPHPFAQRTL TARFQVNNTRPPHVQLLRKPVLTAMGLLALLDEE QLWAEVSQAGTVLDSNHTVGVLASAHRPQGPAD |
| | | AWRAAVLIYASDDTRAHPNRSVAVTLRLRGVPPG PGLVYVTRYLDNGLCSPDGEWRRLGRPVFPTAEQ FRRMRAAEDPVAAAPRPLPAGGRLTLRPALRLPSL LLVHVCARPEKPPGQVTRLRALPLTQGQLVLVWS DEHVGSKCLWTYEIQFSQDGKAYTPVSRKPSTFNL FVFSPDTGAVSGSYRVRALDYWARPGPFSDPVPY LEVPVPRGPPSPGNP |
| 51 | Idursulfase | SETQANSTTDALNVLLIIVDDLRPSLGCYGDKLVR SPNIDQLASHSLLFQNAFAQQAVCAPSRVSFLTGR RPDTTRLYDFNSYWRVHAGNFSTIPQYFKENGYV TMSVGKVFHPGISSNHTDDSPYSWSFPPYHPSSEK YENTKTCRGPDGELHANLLCPVDVLDVPEGTLPD KQSTEQAIQLLEKMKTSASPFFLAVGYHKPHIPFR YPKEFQKLYPLENITLAPDPEVPDGLPPVAYNPWM DIRQREDVQALNISVPYGPIPVDFQRKIRQSYFASV SYLDTQVGRLLSALDDLQLANSTIIAFTSDHGWAL GEHGEWAKYSNFDVATHVPLIFYVPGRTASLPEA GEKLFPYLDPFDSASQLMEPGRQSMDLVELVSLFP TLAGLAGLQVPPRCPVPSFHVELCREGKNLLKHFR FRDLEEDPYLPGNPRELIAYSQYPRPSDIPQWNSD KPSLKDIKIMGYSIRTIDYRYTVWVGFNPDEFLAN FSDIHAGELYFVDSDPLQDHNMYNDSQGGDLFQL LMP |
| 52 | Arylsulfatase A | |

## Claims

1. A device comprising a multilayer scaffold surrounding a cell chamber, wherein the multilayer scaffold comprises an outer layer and an inner layer in contact with the cell chamber, wherein the outer layer and the inner layer each comprise a nanofibrous polymer, wherein the outer layer comprises electrospun polyethylene terephthalate and polybutylene terephthalate, and wherein the inner layer comprises nanofibrous polyurethane or nanofibrous polyethylene terephthalate and polybutylene terephthalate.

2. The device of claim 1, wherein the outer layer and/or the inner layer comprises nanopores.

3. The device of claim 2, wherein the nanopores are sized to permit the passage of biomolecules.

4. The device of claim 2 or 3, wherein the nanopores are sized to prevent cells on one side of the multilayer scaffold from contacting cells on the other side of the multilayer scaffold.

5. The device of any one of claims 2 to 4, wherein the nanopores have a diameter of about 1 µm or less.

6. The device of any one of claims 1 to 5, wherein the device further comprises a polymeric film with a pore size less than or equal to 1 µm positioned between the outer layer and the inner layer of the scaffold.

7. The device of any one of claims 1 to 6, wherein the outer layer and/or the inner layer comprises one or more charged surface modifications.

8. The device of any one of the preceding claims, wherein the cell chamber of the device comprises cells that secrete a recombinant peptide or protein.

9. The device of any one of claims 1 to 7, wherein the cell chamber of the device comprises cells that secrete a monoclonal antibody.

10. The device of claim 9, wherein the cells secrete vedolizumab.

11. The device of any one of claims 8 to 10, wherein the device comprises about 1x10⁴ cells/cm² to about 1x10⁶ cells/cm².

12. The device of any one of the preceding claims, for use in a method of treating a gastrointestinal disorder.

13. The device of claim 12, wherein the gastrointestinal disorder is inflammatory bowel disease.
